# EUROPÄISCHE PATENTANMELDUNG

(11) **EP 2 239 260 A1**
(43) Veröffentlichungstag der Anmeldung: **13.10.2010**
(21) Anmeldenummer: 10003710.0
(22) Anmeldetag: 09.10.2003
(51) Int. Cl.: C07D 407/14, C07D 401/06, C07D 413/14, C07D 417/14, C07D 407/12, C07D 417/12, C07D 239/72, C07D 471/04, C07D 401/12, C07D 413/12, C07D 491/04, C07D 215/20, C07D 239/88, C07D 451/04

(54) **Neue Verbindungen mit antibakterieller Aktivität**

(30) Priorität: 10.10.2002 DE 10247233; 02.12.2002 DE 10256405
(62) Teilanmeldung aus: 03808720.1
(71) Anmelder: Morphochem Aktiengesellschaft Für Kombinatorische Chemie, 81379 München (DE)
(72) Erfinder: Surivet, Jean-Philippe, 68300 Saint-Louis (FR); Zumbrunn, Cornelia, 4058 Basel (CH); Hubschwerlen, Christian, 68480 Durmenach (FR); Perez Frutos Höner, Annabelle, 4056 Basel (CH)
(74) Vertreter: Forstmeyer, Dietmar

(57) **Zusammenfassung**

Die vorliegende Anmeldung beschreibt neuartige antibakterielle Verbindungen der Formel (I).

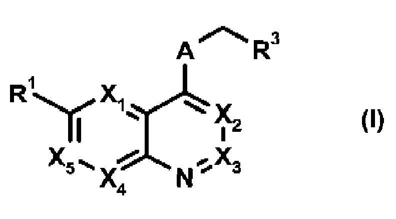

## Beschreibung

In vielen Ländern der Welt hat die Resistenz gegenüber den derzeit gebräuchlichen Antibiotika in den letzten Jahren beträchtlich zugenommen und zum Teil bedrohliche Ausmasse angenommen. Das Hauptproblem dabei ist, dass diese Erreger nicht nur eine, sondern in der Regel mehrfache Resistenzen tragen. Dies gilt insbesondere für einige Gram-positive Erregergruppen, wie Staphylokokken, Pneumokokken und Enterokokken (S. Ewig et al.; Antibiotika-Resistenz bei Erregern ambulant erworbener Atemwegsinfektionen; Chemother. J. 2002, 11, 12-26; F. Tenover; Development and spread of bacterial resistance to antimicrobial agents: an overview; Clin. Infect. Dis. 2001 Sep 15, 33 Suppl. 3, 108-115)

Eine lange befürchtete Entwicklung ist kürzlich eingetreten: In den USA wurde der erste Stamm von *Staphylococcus* aureus beschrieben, welcher nicht nur Methicillin-resistent, sondern auch gegen Vancomycin hochresistent ist (Centers for Disease Control and Prevention; Staphylococcus aureus resistant to vancomycin - United States, 2002; MMWR 2002, 51, 565-567).

Neben hygienischen Massnahmen in Krankenhäusern sind daher auch verstärkt Anstrengungen erforderlich, neue Antibiotika zu finden, die möglichst eine neue Struktur und einen neuen Wirkungsmechanismus besitzen, um gegen diese Problemkeime wirksam zu sein.

Die vorliegende Anmeldung beschreibt neuartige Verbindungen mit antibakterieller Aktivität.

Die vorliegende Erfindung betrifft Verbindungen der allgemeinen Formel (I): 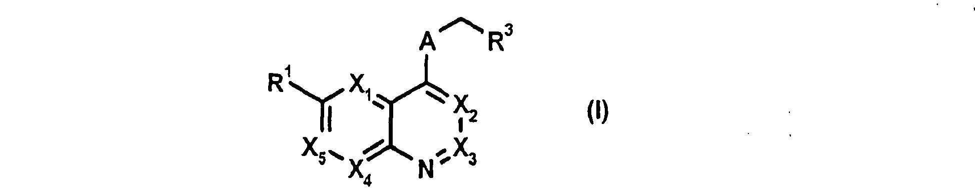 wobei
A ein Sauerstoff-, Schwefel-, Stickstoffatom, eine C₁₋₄ Alkylen-, C₂₋₄ Alkenylen, C₂₋₄ Alkinylen oder eine C₁₋₄ Heteroalkylengruppe ist,
X₁, X₂, X₃, X₄ und X₅ unabhängig voneinander Stickstoffatome oder Gruppen der Formel CR² sind,
R¹ ein Wasserstoffatom, ein Halogenatom, eine Hydroxy-, eine Alkyloxy- oder eine Heteroalkyloxygruppe ist,
R² ein Wasserstoffatom, ein Halogenatom, eine Hydroxy-, Alkyl-, Alkenyl-, Alkinyl- oder eine Heteroalkylgruppe ist,
R³ aus den folgenden Gruppen ausgewählt ist: 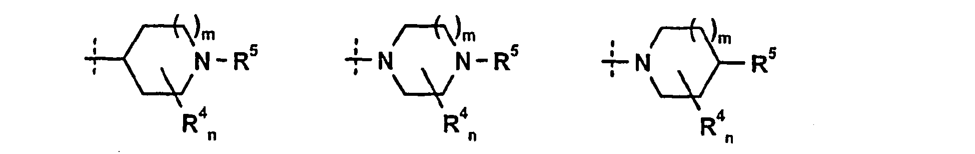 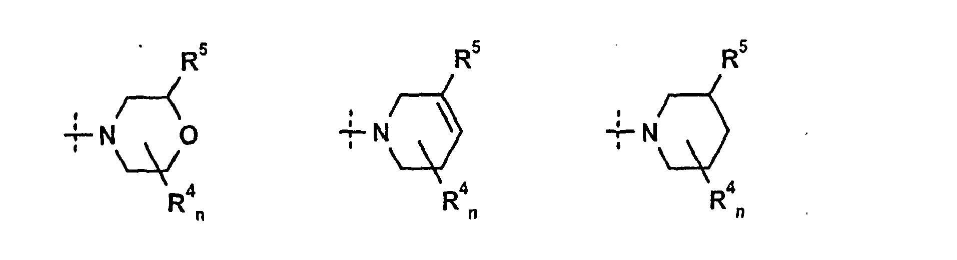 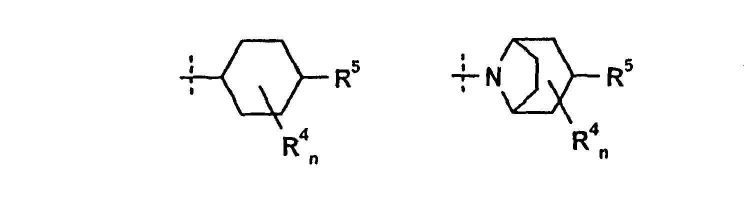 die Reste R⁴ unabhängig voneinander eine Hydroxygruppe, eine C₁₋₆ Alkyl- oder eine C₁₋₈ Heteroalkylgruppe sind, R⁵ ein Alkyl-, Alkenyl-, Alkinyl-, Heteroalkyl-, Aryl-, Heteroaryl-, Cycloalkyl-, Alkylcycloalkyl-, Heteroalkylcycloalkyl-, Heterocycloalkyl-, Aralkyl- oder ein Heteroaralkylrest ist,
- n: gleich 0, 1, 2 oder 3 ist und
- m: gleich 0 oder 2 ist,
oder ein pharmakologisch akzeptables Salz, Solvat, Hydrat oder eine pharmakologisch akzeptable Formulierung derselben.

Der Ausdruck Alkyl bezieht sich auf eine gesättigte, geradkettige oder verzweigte Kohlenwasserstoffgruppe, die 1 bis 20 Kohlenstoffatome, vorzugsweise 1 bis 12 Kohlenstoffatome, besonders bevorzugt 1 bis 8 oder 1 bis 6 oder 1 bis 4 Kohlenstoffatome aufweist, z.B. die Methyl-, Ethyl-, Propyl-, Isopropyl-, Isobutyl-, *tert*-Butyl, n-Hexyl-, 2,2-Dimethylbutyl- oder n-Octylgruppe.

Die Ausdrücke Alkenyl und Alkinyl beziehen sich auf zumindest teilweise ungesättigte, geradkettige oder verzweigte Kohlenwasserstoffgruppen, die 2 bis 20 Kohlenstoffatome, vorzugsweise 2 bis 12 Kohlenstoffatome, besonders bevorzugt 2 bis 6 Kohlenstoffatome aufweisen, z. B. die Ethenyl-, Allyl-, Acetylenyl-, Propargyl-, Isoprenyl- oder Hex-2-enyl-Gruppe. Bevorzugt weisen Alkenylgruppen eine oder zwei (besonders bevorzugt eine) Doppelbindungen bzw. Alkinylgruppen eine oder zwei (besonders bevorzugt eine) Dreifachbindungen auf.

Des weiteren beziehen sich die Begriffe Alkyl, Alkenyl und Alkinyl auf Gruppen, bei der ein oder mehrere Wasserstoffatome durch ein Halogenatom (bevorzugt F oder Cl) ersetzt sind wie z. B. die 2,2,2-Trichlorethyl-, oder die Trifluormethylgruppe.

Der Ausdruck Heteroalkyl bezieht sich auf eine Alkyl-, eine Alkenyl- oder eine Alkinyl-Gruppe, in der ein oder mehrere (bevorzugt 1, 2 oder 3) Kohlenstoffatome durch ein Sauerstoff-, Stickstoff-, Phosphor-, Bor-, Selen-, Silizium- oder Schwefelatom ersetzt sind (bevorzugt Sauerstoff, Schwefel oder Stickstoff). Der Ausdruck Heteroalkyl bezieht sich des weiteren auf eine Carbonsäure oder eine von einer Carbonsäure abgeleitete Gruppe wie z. B. Acyl, Acylalkyl, Alkoxycarbonyl, Acyloxy, Acyloxyalkyl, Carboxyalkylamid oder Alkoxycarbonyloxy.

Beispiele für Heteroalkylgruppen sind Gruppen der Formeln R^{a}-O-y^{a}-, R^{a}-S-Y^{a}-, R^{a}-N(R^{b})-Y^{a}-, R^{a}-CO-Y^{a}-, R^{a}-O-CO-Y^{a}-, R^{a}-CO-O-Y^{a}-, R^{a}-CO-N(R^{b})-Y^{a}-, R^{a}-N(R^{b})-CO-Y^{a}-, R^{a}-O-CO-N(R^{b})-Y^{a}-, R^{a}-N(R^{b})-CO-O-Y^{a}-, R^{a}-N(R^{b})-CO-N(R^{c})-Y^{a}-, R^{a}-O-CO-O-Y^{a}-, R^{a}-N(R^{b})-C(=NR^{d})-N(R^{C})-Y^{a}-, R^{a}-CS-Y^{a}-, R^{a}-O-CS-Y^{a}-, R^{a}-CS-O-Y^{a}-, R^{a}-CS-N(R^{b})-Y^{a}-, R^{a}-N(R^{b})-CS-Y²-, R^{a}-O-CS-N(R^{b})-Y^{a}-, R^{a}-N(R^{b})-CS-O-Y^{a}- R^{a}-N(R^{b})-CS-N(R^{c})-Y^{a}-, R^{a}-O-CS-O-Y^{a}-, R^{a}-S-CO-Y^{a}-, R^{a}-CO-S-Y^{a}-, R^{a}-S-CO-N(R^{b})-Y^{a}-, R^{a}-N(R^{b})-CO-S-Y^{a}, R^{a}-S-CO-O-Y^{a}-, R^{a}-O-CO-S-Y^{a}-, R^{a}-S-CO-S-Y^{a}-, R^{a}-S-CS-Y^{a}-, R^{a}-CS-S-Y^{a}-, R^{a}-S-CS-N(R^{b})-Y^{a}-, R^{a}-N(R^{b})-CS-S-Y^{a}-, R^{a}-S-CS-O-Y^{a}-, R^{a}-O-CS-S-Y^{a}-, wobei R^{a} ein Wasserstoffatom, eine C₁-C₆-Alkyl-, eine C₂-C₆-Alkenyloder eine C₂-C₆-Alkinylgruppe; R^{b} ein Wasserstoffatom, eine C₁-C₆-Alkyl-, eine C₂-C₆-Alkenyl- oder eine C₂-C₆-Alkinylgruppe; R^{c} ein Wasserstoffatom, eine C₁-C₆-Alkyl-, eine, C₂-C₆-Alkenyl- oder eine C₂-C₆-Alkinylgruppe; R^{d} ein Wasserstoffatom, eine C₁-C₆-Alkyl-, eine C₂-C₆-Alkenyloder eine C₂-C₆-Alkinylgruppe und Y^{a} eine direkte Bindung, eine C₁-C₆-Alkylen-, eine C₂-C₆-Alkenylen- oder eine C₂-C₆-Alkinylengruppe ist, wobei jede Heteroalkylgruppe mindestens ein Kohlenstoffatom enthält und ein oder mehrere Wasserstoffatome durch Fluor- oder Chloratome ersetzt sein können. Konkrete Beispiele für Heteroalkylgruppen sind Methoxy, Trifluormethoxy, Ethoxy, n-Propyloxy, iso-Propyloxy, *tert*-Butyloxy, Methoxyethyl, Ethoxymethyl, Methoxyethyl, Methylamino, Ethylamino, Dimethylamino, Diethylamino, iso-Propylethylamino, Methylaminomethyl, Ethylaminomethyl, Di-iso-Propylaminoethyl, Enolether, Dimethylaminomethyl, Dimethylaminoethyl, Acetyl, Propionyl, Butyryloxy, Acetyloxy, Methoxycarbonyl, Ethoxycarbonyl, N-Ethyl-N-Methylcarbamoyl oder N-Methylcarbamoyl. Weitere Beispiele für Heteroalkylgruppen sind Nitril-, Isonitril, Cyanat-, Thiocyanat-, Isocyanat-, Isothiocyanat und Alkylnitril-gruppen.

Der Ausdruck Cycloalkyl bezieht sich auf eine gesättigte oder teilweise ungesättigte (z. B. Cycloalkenyl) cyclische Gruppe, die einen oder mehrere Ringe (bevorzugt 1 oder 2) mit 3 bis 14 Ring-Kohlenstoffatomen, vorzugsweise 3 bis 10 (insbesondere 3, 4, 5, 6 oder 7) Ring-Kohlenstoffatomen enthält. Der Ausdruck Cycloalkyl bezieht sich weiterhin auf Gruppen, bei denen ein oder mehrere Wasserstoffatome durch Fluor-, Chlor-, Brom- oder Jodatome oder OH, =O, SH, =S, NH₂, =NH oder NO₂-Gruppen ersetzt sind also z. B. cyclische Ketone wie z. B. Cyclohexanon, 2-Cyclohexenon oder Cyclopentanon. Weitere konkrete Beispiele für Cycloalkylgruppen sind die Cyclopropyl-, Cyclobutyl-, Cyclopentyl-, Spiro[4,5]decanyl-, Norbornyl-, Cyclohexyl-, Cyclopentenyl-, Cyclohexadienyl-, Decalinyl-, Cubanyl-, Bicyclo[4.3.0]nonyl-, Tetralin-, Cyclopentylcyclohexyl-, Fluorcyclohexyl- oder die Cyclohex-2-enyl-Gruppe.

Der Ausdruck Heterocycloalkyl bezieht sich auf eine Cycloalkylgruppe wie oben definiert, in der ein oder mehrere (bevorzugt 1, 2 oder 3) Ring-Kohlenstoffatome durch ein Sauerstoff-, Stickstoff-, Silizium-, Selen-, Phosphor- oder Schwefelatom (bevorzugt Sauerstoff, Schwefel oder Stickstoff) ersetzt sind. Bevorzugt besitzt eine Heterocycloalkylgruppe 1 oder 2 Ringe mit 3 bis 10 (insbesondere 3, 4, 5, 6 oder 7) Ringatomen. Der Ausdruck Heterocycloalkyl bezieht sich weiterhin auf Gruppen, bei denen ein oder mehrere Wasserstoffatome durch Fluor-, Chlor-, Brom- oder Jodatome oder OH, =O, SH, =S, NH₂, =NH oder NO₂-Gruppen ersetzt sind. Beispiele sind die Piperidyl-, Morpholinyl-, Urotropinyl-, Pyrrolidinyl-, Tetrahydrothiophenyl-, Tetrahydropyranyl-, Tetrahydrofuryl-, Oxacyclopropyl-, Azacyclopropyl- oder 2-Pyrazolinylgruppe sowie Lactame, Lactone, cyclische Imide und cyclische Anhydride.

Der Ausdruck Alkylcycloalkyl bezieht sich auf Gruppen, die entsprechend den obigen Definitionen sowohl Cycloalkylwie auch Alkyl-, Alkenyl- oder Alkinylgruppen enthalten, z. B. Alkylcycloalkyl-, Alkylcycloalkenyl-, Alkenylcycloalkyl- und Alkinylcycloalkylgruppen. Bevorzugt enthält eine Alkylcycloalkylgruppe eine Cycloalkylgruppe, die einen oder zwei Ringsysteme mit 3 bis 10 (insbesondere 3, 4, 5, 6 oder 7) Ring-Kohlenstoffatomen enthält und eine oder zwei Alkyl-, Alkenyl- oder Alkinylgruppen mit 1 oder 2 bis 6 Kohlenstoffatomen.

Der Ausdruck Heteroalkylcycloalkyl bezieht sich auf Alkylcycloalkylgruppen, wie oben definiert, in der ein oder mehrere (bevorzugt 1, 2 oder 3) Kohlenstoffatome durch ein Sauerstoff-, Stickstoff-, Silizium-, Selen-, Phosphor- oder Schwefelatom (bevorzugt Sauerstoff, Schwefel oder Stickstoff) ersetzt sind. Bevorzugt besitzt eine Heteroalkylcycloalkylgruppe 1 oder 2 Ringsysteme mit 3 bis 10 (insbesondere 3, 4, 5, 6 oder 7) Ringatomen und eine oder zwei Alkyl, Alkenyl, Alkinyl oder Heteroalkylgruppen mit 1 oder 2 bis 6 Kohlenstoffatomen. Beispiele derartiger Gruppen sind Alkylheterocycloalkyl, Alkylheterocycloalkenyl, Alkenylheterocycloalkyl, Alkinylheterocycloalkyl, Heteroalkylcycloalkyl, Heteroalkylheterocycloalkyl und Heteroalkylheterocylcloalkenyl, wobei die cyclischen Gruppen gesättigt oder einfach, zweifach oder dreifach ungesättigt sind.

Der Ausdruck Aryl bzw. Ar bezieht sich auf eine aromatische Gruppe, die einen oder mehrere Ringe mit 6 bis 14 Ring-Kohlenstoffatomen, vorzugsweise 6 bis 10 (insbesondere 6) Kohlenstoffatomen enthält. Der Ausdruck Aryl (bzw. Ar) bezieht sich weiterhin auf Gruppen, bei denen ein oder mehrere Wasserstoffatome durch Fluor-, Chlor-, Brom- oder Jodatome oder OH, SH, NH₂, oder NO₂-Gruppen ersetzt sind. Beispiele sind die Phenyl-, Naphthyl-, Biphenyl-, 2-Fluorphenyl, Anilinyl-, 3-Nitrophenyl oder 4-Hydroxy-phenyl-Gruppe.

Der Ausdruck Heteroaryl bezieht sich auf eine aromatische Gruppe, die einen oder mehrere Ringe hat, und durch ein Gerüst gebildet wird, das 5 bis 14 Ringatome, vorzugsweise 5 bis 10 (insbesondere 5 oder 6) Ringatome enthält und ein oder mehrere (bevorzugt 1, 2, 3 oder 4) Sauerstoff-, Stickstoff-, Phosphor- oder Schwefel-Ringatome (bevorzugt O, S oder N) enthält. Der Ausdruck Heteroaryl bezieht sich weiterhin auf Gruppen, bei denen ein oder mehrere Wasserstoffatome durch Fluor-, Chlor-, Brom- oder Jodatome oder OH, SH, NH₂, oder NO₂-Gruppen ersetzt sind. Beispiele sind 4-Pyridyl-, 2-Imidazolyl-, 3-Phenylpyrrolyl-, Thiazolyl-, Oxazolyl-, Triazolyl-, Tetrazolyl-, Isoxazolyl-, Indazolyl-, Indolyl-, Benzimidazolyl-, Pyridazinyl-, Chinolinyl-, Purinyl-, Carbazolyl=, Acridinyl-, Pyrimidyl-, 2,3'-Bifuryl-, 3-Pyrazolyl- und Isochinolinyl-Gruppen.

Der Ausdruck Aralkyl bezieht sich auf Gruppen, die entsprechend den obigen Definitionen sowohl Aryl- wie auch Alkyl-, Alkenyl-, Alkinyl- und/oder Cycloalkylgruppen enthalten, wie z. B. Arylalkyl-, Arylalkenyl-, Arylalkinyl-, Arylcycloalkyl-, Arylcycloalkenyl-, Alkylarylcycloalkyl- und Alkylarylcycloalkenylgruppen. Konkrete Beispiele für Aralkyle sind Toluol, Xylol, Mesitylen, Styrol, Benzylchlorid, o-Fluortoluol, 1H-Inden, Tetralin, Dihydronaphthaline, Indanon, Phenylcyclopentyl, Cumol, Cyclo-hexylphenyl, Fluoren und Indan. Bevorzugt enthält eine Aralkylgruppe ein oder zwei aromatische Ringsysteme (1 oder 2 Ringe) mit 6 bis 10 Kohlenstoffatomen und ein oder zwei Alkyl-, Alkenyl- und/oder Alkinylgruppen mit 1 oder 2 bis 6 Kohlenstoffatomen und/oder eine Cycloalkylgruppe mit 5 oder 6 Ringkohlenstoffatomen.

Der Ausdruck Heteroaralkyl bezieht sich auf eine Aralkylgruppe wie oben definiert, in der ein oder mehrere (bevorzugt 1, 2, 3 oder 4) Kohlenstoffatome durch ein Sauerstoff-, Stickstoff-, Silizium-, Selen-, Phosphor-, Bor- oder Schwefelatom (bevorzugt Sauerstoff, Schwefel oder Stickstoff) ersetzt sind, d. h. auf Gruppen, die entsprechend den obigen Definitionen sowohl Aryl- bzw. Heteroaryl- wie auch Alkyl-, Alkenyl-, Alkinyl- und/oder Heteroalkyl- und/oder Cycloalkyl- und/oder Heterocycloalkylgruppen enthalten. Bevorzugt enthält eine Heteroaralkylgruppe ein oder zwei aromatische Ringsysteme (1 oder 2 Ringe) mit 5 oder 6 bis 10 Kohlenstoffatomen und ein oder zwei Alkyl-, Alkenyl- und/oder Alkinyl-gruppen mit 1 oder 2 bis 6 Kohlenstoffatomen und/oder eine Cycloalkylgruppe mit 5 oder 6 Ringkohlenstoffatomen, wobei 1, 2, 3 oder 4 dieser Kohlenstoffatome durch Sauerstoff-, Schwefel- oder Stickstoffatome ersetzt sind.

Beispiele sind Arylheteroalkyl-, Arylheterocycloalkyl-, Arylheterocycloalkenyl-, Arylalkylheterocycloalkyl-, Arylalkenylheterocycloalkyl-, Arylalkinylheterocyclo-alkyl-, Arylalkylheterocycloalkenyl-, Heteroarylalkyl-, Heteroarylalkenyl-, Heteroarylalkinyl-, Heteroarylheteroalkyl-, Heteroarylcycloalkyl-, Heteroarylcycloalkenyl-, Heteroarylheterocycloalkyl-, Heteroarylheterocycloalkenyl-, Heteroarylalkylcycloalkyl-, Heteroarylalkylheterocycloalkenyl-, Heteroarylheteroalkylcycloalkyl-, Heteroarylheteroalkylcycloalkenyl- und Heteroarylheteroalkylheterocycloalkyl-Gruppen, wobei die cyclischen Gruppen gesättigt oder einfach, zweifach oder dreifach ungesättigt sind. Konkrete Beispiele sind die Tetrahydroisochinolinyl-, Benzoyl-, 2- oder 3-Ethylindolyl-, 4-Methylpyridino-, 2-, 3- oder 4-Methoxyphenyl-, 4-Ethoxyphenyl-, 2-, 3- oder 4-Carboxyphenylalkylgruppe.

Die Ausdrücke Cycloalkyl, Heterocycloalkyl, Alkylcycloalkyl, Heteroalkylcycloalkyl, Aryl, Heteroaryl, Aralkyl und Heteroaralkyl beziehen sich auch auf Gruppen, in denen ein oder mehrere Wasserstoffatome solcher Gruppen durch Fluor-, Chlor-, Brom- oder Jodatome oder OH, =O, SH, =S, NH₂, =NH oder NO₂-Gruppen ersetzt sind.

Der Ausdruck "gegebenenfalls substituiert" bezieht sich auf Gruppen, in denen ein oder mehrere Wasserstoffatome durch Fluor-, Chlor-, Brom- oder Jodatome oder OH, =O, SH, =S, NH₂, =NH oder NO₂-Gruppen ersetzt sind. Dieser Ausdruck bezieht sich weiterhin auf Gruppen, die mit unsubstituierten C₁-C₆ Alkyl-, C₂-C₆ Alkenyl-, C₂-C₆ Alkinyl-, C₁-C₆ Heteroalkyl-, C₃-C₁₀ Cycloalkyl-, C₂-C₉ Heterocycloalkyl-, C₆-C₁₀ Aryl-, C₁-C₉ Heteroaryl-, C₇-C₁₂ Aralkyl- oder C₂-C₁₁ Heteroaralkyl-Gruppen substituiert sind.

Verbindungen der Formel (I) können aufgrund ihrer Substitution ein oder mehrere Chiralitätszentren enthalten. Die vorliegende Erfindung umfasst daher sowohl alle reinen Enantiomere und alle reinen Diastereomere, als auch deren Gemische in jedem Mischungsverhältnis. Des weiteren sind von der vorliegenden Erfindung auch alle *cis*/*trans*-Isomeren der Verbindungen der allgemeinen Formel (I) sowie Gemische davon umfasst. Des weiteren sind von der vorliegenden Erfindung alle tautomeren Formen der Verbindungen der Formel (I) umfasst.

Bevorzugt sind Verbindungen der Formel (I), wobei A ein Sauerstoffatom oder eine Gruppe der Formel CH₂ oder CH(OH) ist.

Weiter bevorzugt sind Verbindungen der Formel (I), wobei eine der Gruppen X₁, X₂, X₃, X₄ und X₅ (insbesondere X₁ oder X₂) ein Stickstoffatom ist und die anderen CH-Gruppen sind oder alle Gruppen X₁, X₂, X₃, X₄ und X₅ CH-Gruppen sind.

Des weiteren bevorzugt ist R³ eine der folgenden Gruppen:

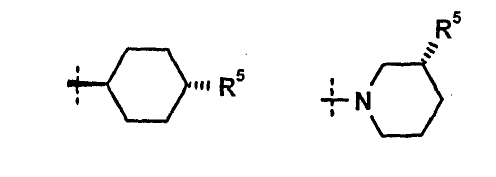

Besonders bevorzugt ist R¹ ein Halogenatom, eine C₁₋₆ Alkyloxygruppe (z. B. Methoxy oder Ethoxy), eine Methyloder eine Ethylgruppe; insbesondere eine Methoxygruppe.

Wiederum bevorzugt ist R⁴ eine C₁₋₆ Heteroalkylgruppe, wie oben definiert, mit ein oder zwei Sauerstoffatomen als einzige Heteroatome.

Besonders bevorzugt ist R⁴ eine Gruppe der Formel -COOH, -CH₂COOH, -CH₂CH₂COOH, -CH₂COOCH₃, -CH₂CH₃, -CH₂OH, -CH₂CH₂OH, -OH, -OCH₃, -CH₂OCONH₂, -CH₂CH₂COOCH₃, -COOCH₃, -CH₃ oder -(CH₂)₃OH.

Des weiteren bevorzugt ist n gleich 0 oder 1.

Weiter bevorzugt ist R⁵ eine Aralkyl- oder eine Heteroaralkylgruppe.

Wiederum bevorzugt ist R⁵ eine Gruppe der Formel' -Y-Cy, wobei Y eine C₁-C₆ Alkylen-, C₂-C₆ Alkenylen- oder C₁-C₆ Heteroalkylengruppe ist, wobei gegebenenfalls ein Wasserstoffatom durch eine Hydroxygruppe oder zwei Wasserstoffatome durch eine =O Gruppe ersetzt sein können und Cy eine gegebenenfalls substituierte Phenyl-, Naphthyl- oder Heteroarylgruppe mit 1 oder 2 Ringen und 5 bis 10 Ringatomen oder eine gegebenenfalls substituierte Arylheterocycloalkyl- oder Heteroarylheterocycloalkylgruppe mit zwei Ringen und 9 oder 10 Ringatomen ist.

Die therapeutische Verwendung der Verbindungen der Formel (I), ihrer pharmakologisch akzeptablen Salze bzw. Solvate und Hydrate sowie Formulierungen und pharmazeutischen Zusammensetzungen liegt ebenfalls im Rahmen der vorliegenden Erfindung.

Die pharmazeutischen Zusammensetzungen gemäß der vorliegenden Erfindung enthalten mindestens eine Verbindung der Formel (I) als Wirkstoff und fakultativ Trägerstoffe und/oder Adjuvantien.

Beispiele für pharmakologisch akzeptable Salze der Verbindungen der Formel (I) sind Salze von physiologisch akzeptablen Mineralsäuren wie Salzsäure, Schwefelsäure und Phosphorsäure oder Salze von organischen Säuren wie Methansulfonsäure, p-Toluolsulfonsäure, Milchsäure, Essigsäure, Trifluoressigsäure, Zitronensäure, Bernsteinsäure, Fumarsäure, Oxalsäure, Maleinsäure und Salicylsäure. Weitere Beispiele für pharmakologisch akzeptable Salze der Verbindungen der Formel (I) sind Alkali- oder Erdalkalisalze wie z. B. Natrium, Kalium, Lithium, Calcium oder Magnesium Salze, Ammoniumsalze oder Salze von organischen Basen wie z. B. Methylamin, Dimethylamin, Triethylamin, Piperidin, Ethylendiamin, Lysin, Cholinhydroxid, Meglumin, Morpholin oder Arginin Salze. Verbindungen der Formel (I) können solvatisiert, insbesondere hydratisiert sein. Die Hydratisierung kann z.B. während des Herstellungsverfahrens oder als Folge der hygroskopischen Natur der anfänglich wasserfreien Verbindungen der Formel (I) auftreten. Wenn die Verbindungen der Formel (I) asymmetrische C-Atome enthalten,' können sie entweder als achirale Verbindungen, Diastereomeren-Gemische, Gemische von Enantiomeren oder als optisch reine Verbindungen vorliegen.

Die Pro-Drugs, die ebenfalls Gegenstand der vorliegenden Erfindung sind, bestehen aus einer Verbindung der Formel (I) und mindestens einer pharmakologisch akzeptablen Schutzgruppe, die unter physiologischen Bedingungen abgespalten wird, z.B. einer Alkoxy-, Aralkyloxy-, Acyloder Acyloxy-Gruppe, wie z.B. einer Ethoxy-, Daloxat-, Benzyloxy-, Acetyl- oder Acetyloxy-Gruppe oder einer Gruppe der Formel COOCH₂OCOC(CH₃) oder COOCH₂OCOO-Cyclohexyl.

Auch die Verwendung dieser Wirkstoffe zur Herstellung von Arzneimitteln ist Gegenstand der vorliegenden Erfindung. Im allgemeinen werden Verbindungen der Formel (I) unter Anwendung der bekannten und akzeptablen Modi, entweder einzeln oder in Kombination mit einem beliebigen anderen therapeutischen Mittel verabreicht. Solche therapeutisch nützlichen Mittel können auf einem der folgenden Wege verabreicht werden: oral, z.B. als Dragees, überzogene Tabletten, Pillen, Halbfeststoffe, weiche oder harte Kapseln, Lösungen, Emulsionen oder Suspensionen; parenteral, z.B. als injizierbare Lösung; rektal als Suppositorien; durch Inhalation, z.B. als Pulverformulierung oder Spray, transdermal oder intranasal. Zur Herstellung solcher Tabletten, Pillen, Halbfeststoffe, überzogenen Tabletten, Dragees und harten Gelatinekapseln kann das therapeutisch verwendbare Produkt mit pharmakologisch inerten, anorganischen oder organischen Arzneimittelträgersubstanzen vermischt werden, z.B. mit Lactose, Sucrose, Glucose, Gelatine, Malz, Silicagel, Stärke oder Derivaten derselben, Talkum, Stearinsäure oder ihren Salzen, Trockenmagermilch und dgl. Zur Herstellung von weichen Kapseln kann man Arzneimittelträgerstoffe wie z.B. pflanzliche Öle, Petroleum, tierische oder synthetische Öle, Wachs, Fett, Polyole einsetzen. Zur Herstellung von flüssigen Lösungen und Sirups kann man Arzneimittelträgerstoffe wie z.B. Wasser, Alkohole, wäßrige Salzlösung, wäßrige Dextrose, Polyole, Glycerin, pflanzliche Öle, Petroleum, tierische oder synthetische Öle verwenden. Für Suppositorien kann man Arzneimittelträgerstoffe wie z.B. pflanzliche Öle, Petroleum, tierische oder synthetische Öle, Wachs, Fett und Polyole verwenden. Für Aerosol-Formulierungen kann man komprimierte Gase, die für diesen Zweck geeignet sind, wie z.B. Sauerstoff, Stickstoff und Kohlendioxid einsetzen. Die pharmazeutisch verwendbaren Mittel können auch Zusatzstoffe zur Konservierung, Stabilisierung, Emulgatoren, Süßstoffe, Aromastoffe, Salze zur Veränderung des osmotischen Drucks, Puffer, Umhüllungszusatzstoffe und Antioxidantien enthalten.

Kombinationen mit anderen therapeutischen Mitteln können andere antimikrobielle und antifungale Wirkstoffe beinhalten.

Zur Vorbeugung und/oder Behandlung von Bakterien-infektionen kann die Dosis der erfindungsgemäßen biologisch aktiven Verbindung innerhalb breiter Grenzen variieren und kann auf den individuellen Bedarf eingestellt werden. Im allgemeinen ist eine Dosis von 10 mg bis 4000 mg pro Tag geeignet, wobei eine bevorzugte Dosis 50 bis 3000 mg pro Tag ist. In geeigneten Fällen kann die Dosis auch unter oder über den oben angegebenen Werten liegen. Die tägliche Dosis kann als einfache Gabe oder in mehrfachen Gaben verabreicht werden. Eine typische Einzeldosis beinhaltet etwa 50 mg, 100 mg, 250 mg, 500 mg, 1 g oder 2 g des Wirkstoffs.

### BEISPIELE

Beispiel 1: 2-((3RS)-{[(2,3-Dihydro-benzo[1,4]dioxin-6-yl-methyl)amino]methyl}piperidin-1-yl)-(1RS)-(6-methoxychinolin-4-yl)ethanol.

### 1.a) (3RS)-Azidomethylpiperidin-1-benzylcarbamat:

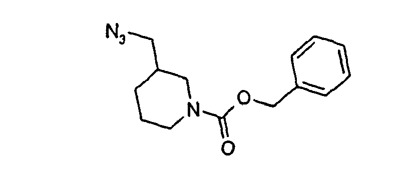

Zu einer Lösung von 3-Hydroxymethylpiperidin-1-benzylcarbamat (5g, 20.05mmol) in Dichlormethan (100mL) wurden bei 0°C Triethylamin (5.6mL, 40.1mmol) und anschliessend Methansulfonylchlorid (2mL, 25.7mmol) gegeben. Nachdem die Lösung 20 min gerührt hatte, wurde die Reaktionsmischung auf -60°C gekühlt und eine Lösung von 4-Oxopiperidin-1-benzylcarbamat (2.33g, 10mmol) in Diethylether (10mL) zugegeben. Die Reaktionsmischung würde innerhalb von 30 Minuten auf Raumtemperatur aufgewärmt und Wasser (40mL) zugegeben. Die beiden Phasen wurden getrennt und die wässrige Phase zweimal mit je 50mL Essigester extrahiert. Die vereinten organischen Phasen wurden mit 20mL gesättigter Kochsalzlösung gewaschen, über MgSO₄ getrocknet, filtriert und zur Trockne einrotiert. Der Rückstand wurde mittels Säulenchromatographie an Kieselgel (EtOAc) gereinigt. Das erstandene Öl wurde in DMF (45 mL) gelöst und mit Natriumazid (2.6g, 40mmol) versetzt. Das Reaktionsgemisch wurde 3 Stunden bei 80°C weitergerührt dann mit Wasser (100mL) und Essigester (200mL) verdünnt. Die beiden Phasen wurden getrennt und die wässrige Phase zweimal mit je 50mL Essigester extrahiert. Die vereinten organischen Phasen wurden mit 20mL gesättigter Kochsalzlösung gewaschen, über MgSO₄ getrocknet, filtriert und zur Trockne einrotiert. Der Rückstand wurde mittels Säulenchromatographie an Kieselgel (Hexan/EtOAc 1/1) gereinigt.
Ausbeute: 6.1 g (18.6 mmol)
¹H-NMR (CDC13, 300MHz: 1.28 (m, 1H); 1.51 (m, 1H); 1.60-1.87 (m, 3H); 2.74 br s, 1H); 2.91 (m, 1H); 3.23 (br d, J= 4.5Hz, 2H); 3.98 (td, J= 4.1, 13.2Hz, 1H);
4.06 (br s, 1H) ; 5.15 (s, 2H); 7.28-7.38 (m, 5H).

3-Hydroxymethyl-piperidin-1-benzylcarbamat wurde bereits in Arch. Pharm. (Weinheim, Germany) 1990 p. 9-12 beschrieben.

### 1.b) (3RS)-Aminomethyl-piperidin-1-benzylcarbamat:

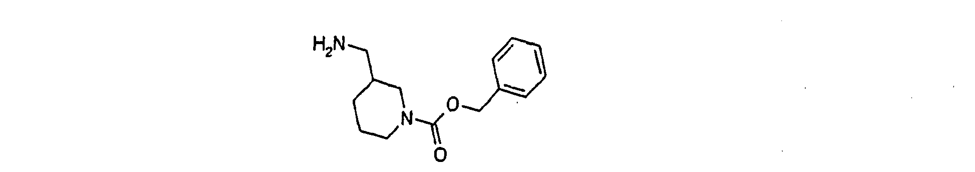

Zu einer Lösung von 3-Azidomethylpiperidin-1-benzylcarbamat (6.1g, 18.6mmol) in THF (37mL) und Wasser (5mL) wurde Triphenylphosphin (8g, 30mmol) zugegeben. Nachdem die Lösung 3 Stunden bei 60°C gerührt hatte, wurde die Reaktionsmischung eingeengt und der Rückstand in 3N HCl (200mL) and Ether (200mL) aufgenommen. Die beiden Phasen wurden getrennt und die wässrige Phase zweimal mit je 100mL Essigester extrahiert. Festes Natriumhydroxid (16g, 640mmol) wurde sorgfältig zugegeben bis sich ein Öl abscheidet. Das Gemisch wurde mit Essigester verdünnt, die organische Phase über MgSO₄ getrocknet, filtriert und das Filtrat zur Trockne einrotiert.
Ausbeute: 2.81 g, 11.3mmol
MS (EI) m/z 249 [M+H]⁺

### 1.c) (3RS)-{[(2,3-Dihydrobenzo[1,4]dioxin-6-ylmethyl) amino]-methyl}-piperidin-1-benzylcarbamat:

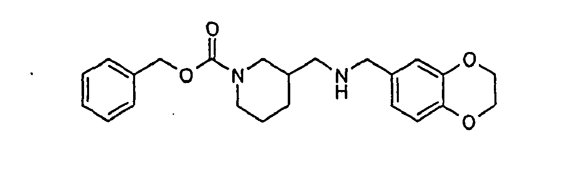

Zu einer Lösung von 3-Aminomethylpiperidin-1-benzylcarbamat (1.5g, 6mmol) in Dichloroethan (37mL) und THF (4mL) wurden 1,4-Benzodioxan-6-carbaldehyd (0.984g, 6mmol) und Natriumtriacetoxyborhydrid (1.7g, 8mmol) zugegeben. Nachdem die Lösung 3 Stunden bei Raumtemperatur gerührt hatte, wurde gesättigte NaHCO₃-Lösung (20mL) zugegeben. Die beiden Phasen wurden getrennt und die wässrige Phase zweimal mit je 50mL Dichlormethan extrahiert. Die vereinten organischen Phasen wurden mit 20mL gesättigter Kochsalzlösung gewaschen, über MgSO₄ getrocknet, filtriert und zur Trockne einrotiert. Der Rückstand wurde mittels Säulenchromatographie an Kieselgel (EtOAc dann MeOH/EtOAc 1/9) gereinigt.
Ausbeute: 1.7g, 4.28mmol (Öl)

MS (EI) m/z 397 [M+H]⁺

1.d (2,3-Dihydro-benzo[1,4]dioxin-6-ylmethyl)-piperidin-(3RS)-yl-methylamin: 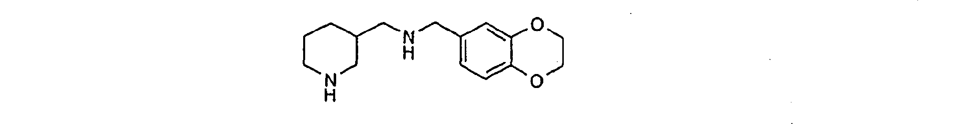 Zu einer Lösung 3-{[(2,3-Dihydrobenzo[1,4]dioxin-6-yl-methyl)amino]methyl}piperidin-1-benzylcarbamat (0.98g, 2.47mmol) in EtOH (10mL) und EtOAc (10 mL) wurde 20% Pd(OH)₂ auf Kohle (0.23g) zugegeben und unter Wasserstoffatmosphäre (1bar) hydriert. Die Reaktions-mischung wurde abfiltriert und das Filtrat eingeengt.
Ausbeute: 0.64g, 2.43mmol
MS (EI) m/z 308 [M+H]⁺

1.e) (RS)-6-Methoxy-4-oxiranylchinolin: 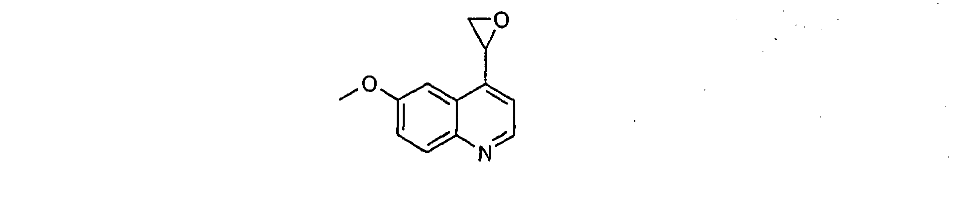 Zu einer Lösung von 6-Methoxychinolin-4-carbaldehyd (0.85g, 4.54mmol) in Acetonitril (13.5mL) und Wasser (6 Tropfen) wurden Trimethylsulfoniumjodid (0.954g, 4.67mmol) und Kaliumhydroxid Pulver (1.8 g, 32mmol) gegeben. Die Reaktionsmischung wurde während einer Stunde auf 60°C erhitzt. Anschliessend wurde die Mischung auf Raumtemperatur abgekühlt und Benzol (40mL) zugegeben. Der Niederschlage wurde abfiltriert und das Filtrat zur Trockne einrotiert. Der Rückstand wurde mit Wasser (100mL) und Essigester (100 mL) verdünnt, die Phasen getrennt und die wässrige Phase zweimal mit je 50mL Essigester extrahiert. Die vereinten organischen Phasen wurden über MgSO₄ getrocknet, filtriert und zur Trockne einrotiert. Der Rückstand wurde mittels Säulen-chromatographie an Kieselgel (EtOAc) gereinigt.
Ausbeute: 0.904g, 4.5mmol
MS (EI) m/z 202 [M+H]⁺

6-Methoxychinolin-4-carbaldehyd wurde nach Eur. J. Med: Chem. Chim. Ther. 2000 (35) p-707-714 hergestellt.

1.f) 2-((3RS)-{[(2,3-Dihydrobenzo[1,4]dioxin-6-ylmethyl)-amino]-methyl}-piperidin-1-yl)-(1RS) (6-methoxychinolin-4-yl)ethanol: 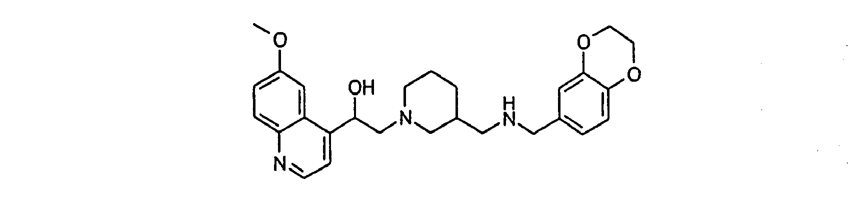 Eine Lösung von (RS)-6-Methoxy-4-oxiranylchinolin (0.161g, 0.8mmol) und (2,3-Dihydrobenzo[1,4]dioxin-6-ylmethyl)-piperidin-(3RS)-ylmethylamin (0.210g, 0.8mmol) in Ethanol (4mL) wurde während 16 Stunden auf 80C erwärmt. Die Reaktionsmischung wurde auf Raumtemperatur abgekühlt und zur Trockne einrotiert. Der Rückstand wurde mittels Säulenchromatographie an Kieselgel (EtOAc dann EtOAc-MeOH 4:1) gereinigt.
Ausbeute: 0.240g. 0.51mmol (Oel)
MS (EI) m/z 464 [M+H]⁺

### Beispiel 2: (1RS)-(6-Methoxy-chinolin-4-yl)-2-((3RS)-{[(naphthalin-2-ylmethyl)-amino]-methyl}-piperidin-1-yl)-ethanol

2.a) (3RS)-(*tert*-Butoxycarbonylamino-methyl)-piperidin-1-benzylcarbamat 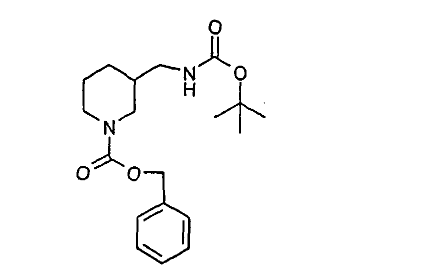 Zu einer Lösung von 3-Aminomethylpiperidin-1-benzylcarbamat (5g, 20.1mmol) in Dichlormethan (100mL) wurden Triethylamin (5.6mL) und Di-*text*.-butyl-dicarbonat (4.9g, 22.4mmol) gegeben. Nachdem die Lösung 4 Stunden bei Raumtemperatur gerührt hatte, wurde die Lösung zur Trockne einrotiert. Der Rückstand wurde mittels Säulenchromatographie an Kieselgel (EtOAc-Hexan 1-4) gereinigt.
Ausbeute: 5.0g, 14.3mmol (Oel)
MS (EI) m/z 349 [M+H]⁺

2.b) (RS)-Piperidin-3-ylmethyl-*tert*-butylcarbamat 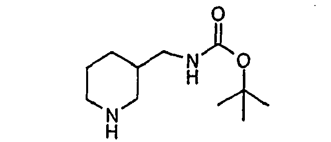 Zu einer Lösung 3-(*tert*-Butoxycarbonylamino-methyl)-piperidin-1-benzylcarbamat (5 g, 14.3 mmol) in EtOH (50mL) und EtOAc (50 mL) wurde 20% Pd(OH)2 auf Kohle (1g) zugegeben und unter Wasserstoffatmosphäre (1bar) hydriert.
Die Reaktions-mischung wurde abfiltriert und das Filtrat eingeengt.
Ausbeute: 3.0g, 14 mmol (Oel)
MS (EI) m/z 215 [M+H]⁺

2.c) {1-[(2RS)-Hydroxy-2-(6-methoxy-chinolin-4-yl)-ethyl]-piperidin-(3RS)-ylmethyl}-*tert*-butylcarbamat 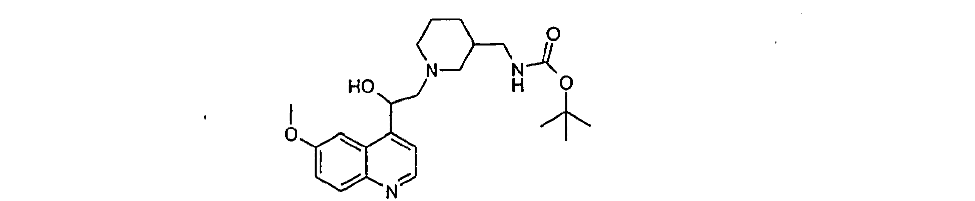 Eine Lösung von 6-Methoxy-4-oxiranylchinolin (2.68g, 13.3mmol) und Piperidin-3-ylmethyl-*tert*-butylcarbamat (2.85 g, 13.3mmol) in Ethanol (35mL) wurde während 12 Stunden auf 80C erwärmt. Die Reaktionsmischung wurde auf Raumtemperatur abgekühlt und zur Trockne einrotiert. Der Rückstand wurde mittels Säulenchromatographie an Kieselgel (Dichlormethan-MeOH 9:1) gereinigt.
Ausbeute: 2.49g. 6mmol (Schaum)
MS (EI) m/z 416 [M+H]⁺

2.d) 2-((3RS)-Aminomethyl-piperidin-1-yl)-(1RS)-(6-methoxy-chinolin-4-yl)-ethanol 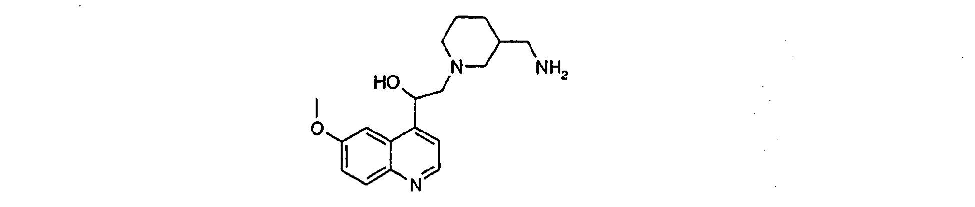 Eine Lösung von {1-[2-Hydroxy-2-(6-methoxy-chinolin-4-yl)-ethyl]-piperidin-3-ylmethyl}-*tert*-butylcarbamat (2.49 g, 6mmol) in TFA (10 ml) wurde 20 Minuten bei Raumtemperatur gerührt. Das Reaktionsgemisch wurde eingeengt und in wässriger 2N NaOH aufgenommen. Die wässrige Phase wurde mit Dichlormethan-MeOH (9-1) extrahiert, die vereinten organischen Phasen über Natriumsulfat getrocknet, filtriert und zur Trockne einrotiert.
Ausbeute: 1.7g. 5.4mmol (Schaum)
MS (EI) m/z 316 [M+H]⁺

2.e) (1RS)-(6-Methoxy-chinolin-4-yl)-2-((3RS)-{[(naphthalin-2-ylmethyl)-amino]-methyl}-piperidin-1-yl)-ethanol 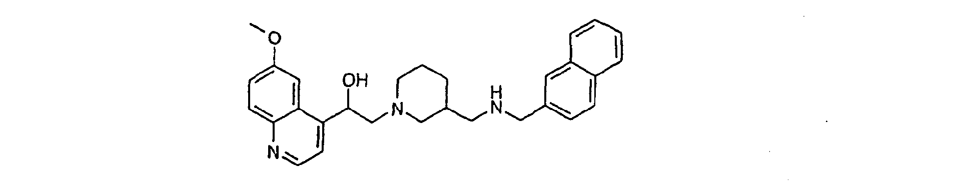 Zu einer Lösung von 2-(3-Aminomethyl-piperidin-1-yl)-1-(6-methoxy-chinolin-4-yl)-ethanol (0.158g, 0.5mmol) in MeOH (1.5mL) and Dichlormethan (3.5mL) wurden Molekularsieb Typ 3A (1g) und 2-Naphtaldehyd (0.078g, 0.5 mmol) gegeben. Das Reaktionsgemisch wurde 16 Stunden bei Raumtemperatur gerührt und Natriumborhydrid (0.05g, 1.35mmol) wurde zugegeben. Das Reaktionsgemisch wurde 2 Stunden bei Raumtemperatur gerührt dann über Hydromatrix (benetzt mit einer NaHCO₃ Lösung, 2mL) filtriert und mit Dichlormethan nachgewaschen. Das Filtrat wurde zur Trockne einrotiert. Der Rückstand wurde mittels Säulenchromatographie an Kieselgel (Dichlor-methan/MeOH 9/1 1% NH₄OH) gereinigt.
Ausbeute:0.158g. (0.34mmol) (Schaum)
MS (EI) m/z 456 [M+H]⁺

### Beipiel 3: (1RS)-(6-Methoxy-chinolin-4-yl)-2-{(3RS)-[(3-phenyl-allylamino)-methyl]-piperidin-1-yl}-ethanol

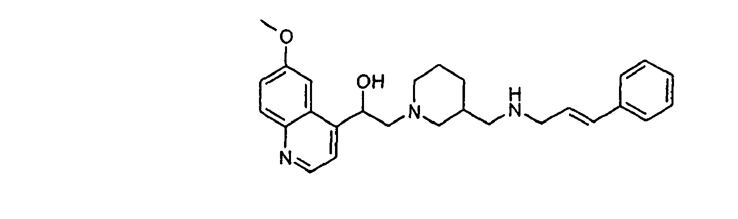

In analoger Weise wurde ausgehend von Beispiel 2.e, (1RS)-(6-Methoxy-chinolin-4-yl)-(2RS)-{3-[(3-phenyl-allylamino)-methyl]-piperidin-1-yl}-ethanol in 74% Ausbeute hergestellt (MS (EI) m/z 432 [M+H]⁺).

### Beispiel 4: 2-{(3RS)-[(3-Furan-2-yl-allylamino)-methyl]-piperidin-1-yl}-(1RS)-(6-methoxy-chinolin-4-yl)-ethanol

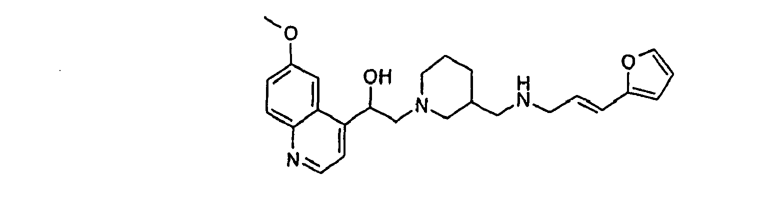

In analoger Weise wurde ausgehend von Beispiel 2.e, 2-{3-[(3-Furan-2-yl-allylamino)-methyl]-piperidin-1-yl}-1-(6-methoxy-chinolin-4-yl)-ethanol in 56% Ausbeute hergestellt (MS (EI) m/z 422 [M+H]⁺).

### Beispiel 5: (3S)-6-[({1-[2-Hydroxy-2-(6-methoxy-chinolin-4-yl)-ethyl]-piperidin-(3RS)-ylmethyl}-amino)-methyl]-4H-benzo[1,4]oxazin-3-on

5 a) (3*S*)-Aminomethyl-piperidin-1-*tert*-butylcarbamat 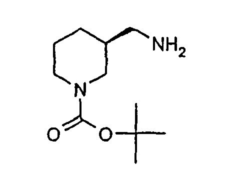 Zu einer Lösung von (3R)-Azidomethyl-piperidin-1-*tert-*butylcarbamat (2.16 g, 8.9 mmol) in THF (60mL) und Wasser (5mL) wurde Triphenylphosphin (3.93g, 15mmol) gegeben. Nachdem die Lösung 3 Stunden bei 60°C gerührt hatte, wurde die Reaktionsmischung eingeengt und der Rückstand in 3N HCl (200mL) and Ether (200mL) aufgenommen. Die beiden Phasen wurden getrennt und die wässrige Phase zweimal mit je 100mL Essigester extrahiert. Festes Natriumhydroxid (6g, 15ommol) wurde sorgfältig zugegeben bis sich ein Öl abscheidet. Das Gemisch wurde mit Essigester verdünnt, die organische Phase über MgSO₄ getrocknet, filtriert und das Filtrat zur Trockne einrotiert.
Ausbeute: 1.90 g, 8.8mmol
MS (EI) m/z 215 [M+H]⁺

3*R*-Azidomethyl-piperidin-1-*tert*-butylcarbamat wurde nach J. Med. Chem. 1994 (37) p-3889-3901 hergestellt

5.b) 3S- (Benzyloxycarbonylamino-methyl)-piperidin-1-*tert-*butylcarbamat

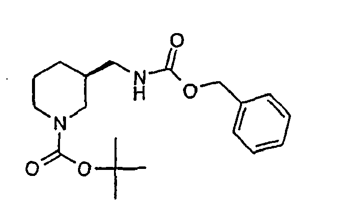

Zu einer Lösung 3S-Aminomethyl-piperidin-1-*tert-*butylcarbamat (1.90g, 8.8mmol) in Aceton-Wasser (1-1, 100mL) wurden Natriumbicarbonat (1.3g, 15.4mmol) und Chlorameisensäure-benzylester (1.3mL, 9mmol) gegeben. Das Reaktionsgemisch wurde während 1 Stunde gerührt dann zur Trockne einrotiert. Der Rückstand wurde mit EtOAc (100mL) verdünnt. Die beiden Phasen wurden getrennt und die wässrige Phase zweimal mit je 50mL EtOAc extrahiert. Die vereinten organischen Phasen wurden mit 20mL gesättigter Kochsalzlösung gewaschen, über MgSO₄ getrocknet, filtriert und zur Trockne einrotiert. Der Rückstand wurde mittels Säulenchromatographie an Kieselgel (EtOAc-Hexan 1-2) gereinigt.
Ausbeute: 2.96g , 8.5mmol (Oel)
MS (EI) m/z 349 [M+H]⁺

5.c) Piperidin-(3R)-ylmethyl-benzylcarbamat 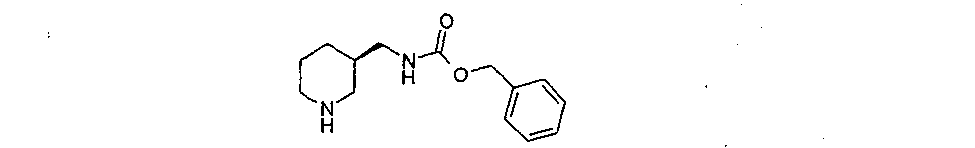 Eine Lösung von 3S-(Benzyloxycarbonylamino-methyl)-piperidin-1-*tert*-butylcarbamat(2.96 g, 6mmol) in TFA (10 ml) wurde 20 Minuten bei Raumtemperatur gerührt. Das Reaktionsgemisch wurde eingeengt und in wässriger 2N NaOH aufgenommen. Die wässrige Phase wurde mit Dichlormethan-MeOH (9-1) extrahiert, die vereinten organischen Phasen über Natriumsulfat getrocknet, filtriert und zur Trockne einrotiert.
Ausbeute: 1.96g. 7.9mmol (Schaum)
MS (EI) m/z 249 [M+H]⁺

5.d) {1-[(2RS)-Hydroxy-2-(6-methoxy-chinolin-4-yl)-ethyl]-piperidin-(3R)-ylmethyl}-benzylcarbamat 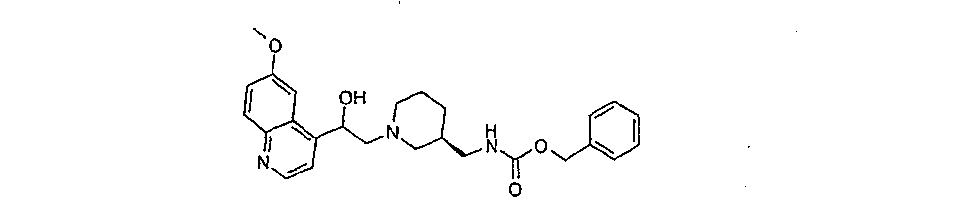 Zu einer Lösung von 6-Methoxy-4-oxiranylchinolin (1.127 g, 5.60mmol) und Piperidin-3-ylmethyl-benzylcarbamat (1.46 g, 5.88mmol) in DMF (17mL) wurden Kaliumcarbonat (1.12 g, 8.10mmol) und Lithiumperchlorat (0.627 g, 5.89mmol) gegeben. Die Reaktionsmischung wurde während 16 Stunden auf 60°C erhitzt dann auf Raumtemperatur abgekühlt und zur Trockne einrotiert. Der Rückstand wurde mittels Säulenchromatographie an Kieselgel (Dichlormethan-MeOH 9:1 1% Ammoniumhydroxid) gereinigt.
Ausbeute: 2.51g. 5.58mmol (Oel)
MS (EI) m/z 450 [M+H]⁺

5.e) (3S)-2-(3-Aminomethyl-piperidin-1-yl)-(1RS)-(6-methoxy-chinolin-4-yl)-ethanol 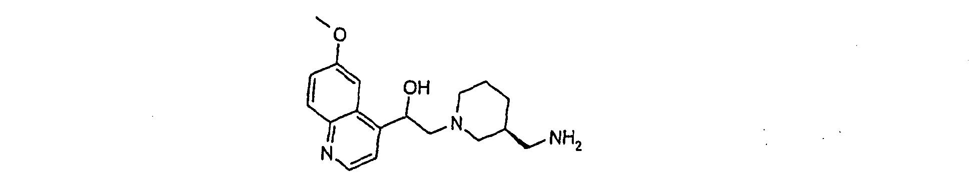 Zu einer Lösung von {1-[2-Hydroxy-2-(6-methoxy-chinolin-4-yl)-ethyl]-piperidin-3-ylmethyl}-benzylcarbamat (2.51 g, 5.58mmol) in THF (35mL) und MeOH (7 mL) wurde 20% Pd(OH)₂ auf Kohle (0.7g) zugegeben und unter Wasserstoffatmosphäre (1bar) hydriert. Die Reaktions-mischung wurde abfiltriert und das Filtrat eingeengt.
Ausbeute: 1.76g. 5.58mmol (Oel)
MS (EI) m/z 316 [M+H]⁺

5.f) (3S)-6-[({1-[(2RS)-Hydroxy-2-(6-methoxy-chinolin-4-yl)-ethyl]-piperidin-3-ylmethyl}-amino)-methyl]-4H-benzo[1,4]oxazin-3-on 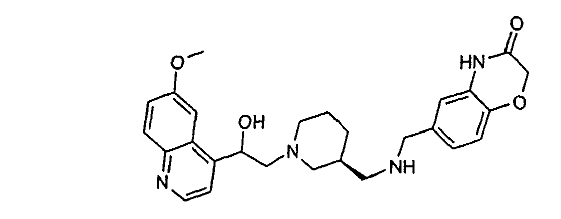 Zu einer Lösung von (3S)-2-(3-Aminomethyl-piperidin-1-yl)-1-(6-methoxy-chinolin-4-yl)-ethanol (0.171g, 0.54mmol) in MeOH (1.6mL) and Dichlormethan (3.8mL) wurden Molekularsieb Typ 3A (1.082g) und 3-Oxo-3,4-dihydro-2H-benzo[1,4]oxazin-6-carbaldehyd (0.095g, 0.54 mmol) gegeben. Das Reaktionsgemisch wurde 16 Stunden bei Raumtemperatur gerührt und Natriumborhydrid (0.054g, 1.43mmol) wurde zugegeben. Das Reaktionsgemisch wurde 2 Stunden bei Raumtemperatur gerührt dann über Hydromatrix (benetzt mit einer NaHCO₃ Lösung, 2mL) filtriert und mit Dichlormethan nachgewaschen. Das Filtrat wurde zur Trockne einrotiert. Der Rückstand wurde mittels Säulenchromatographie an Kieselgel (Dichlor-methan/MeOH 9/1 1% NH₄OH) gereinigt.
Ausbeute:0.112g. (0.24mmol) (Schaum)
MS (EI) m/z 477 [M+H]⁺

3-Oxo-3,4-dihydro-2H-benzo[1,4]oxazin-6-carbaldehyd wurde nach WO 02/34754 hergestellt.

### Beispiel 6: (3S)-2-(3-{[(Benzo[1,2,5]thiadiazol-5-ylmethyl)-amino]-methyl}-piperidin-1-yl)-(1RS)-(6-methoxychinolin-4-yl)-ethanol

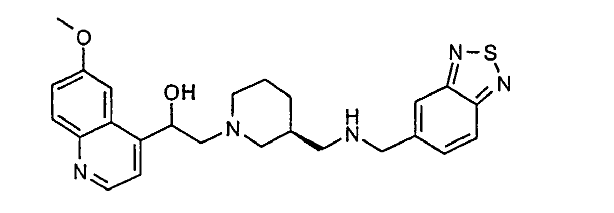

In analoger Weise wurde ausgehend von (3S)-2-(3-Aminomethyl-piperidin-1-yl)-1-(6-methoxy-chinolin-4-yl)-ethanol (Beispiel 5.e), (3S)-2-(3-{[(Benzo[1,2,5]thiadiazol-5-ylmethyl)-amino]-methyl}-piperidin-1-yl)-1-(6-methoxy-chinolin-4-yl)-ethanol in 49% Ausbeute hergestellt (MS (EI) m/z 464 [M+H]⁺).

### Beispiel 7: 2-((3S)-{[(2,3-Dihydro-benzo[1,4]dioxepin-6-ylmethyl)-amino]-methyl}-piperidin-1-yl)-(1RS)-(6-methoxy-chinolin-4-yl)-ethanol

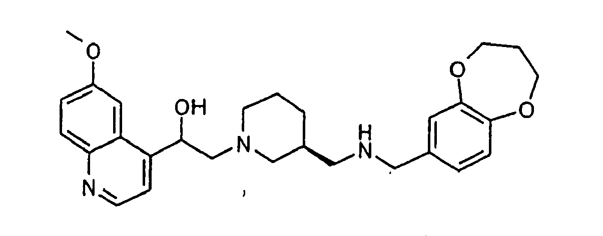

In analoger Weise wurde ausgehend von (3S)-2-(3-Aminomethyl-piperidin-1-yl)-1-(6-methoxy-chinolin-4-yl)-ethanol (Beispiel 5.e), 2-((3S)-{[(2,3-Dihydrobenzo[1,4]dioxepin-6-ylmethyl)-amino]-methyl}-piperidin-1-yl)-(1RS)-(6-methoxy-chinolin-4-yl)-ethanol in 62% Ausbeute hergestellt (MS (EI) m/z 478 [M+H]⁺).
3,4-Dihydro-2H-benzo[b][1,4]dioxepine-7-carbaldehyd wurde nach Chem.Abstr 1958, 3816 hergestellt.

### Beispiel 8: 2-((3S)-{[(2,3-Dihydro-benzo[1,4]dioxin-6-ylmethyl)-amino]-methyl}-piperidin-1-yl)-(1S)-(6-methoxy-chinolin-4-yl)-ethanol

8.a) (3S)-{[(2,3-Dihydro-benzo[1,4]dioxin-6-ylmethyl)-amino]-methyl}-piperidin-1-*tert*-butylcarbamat 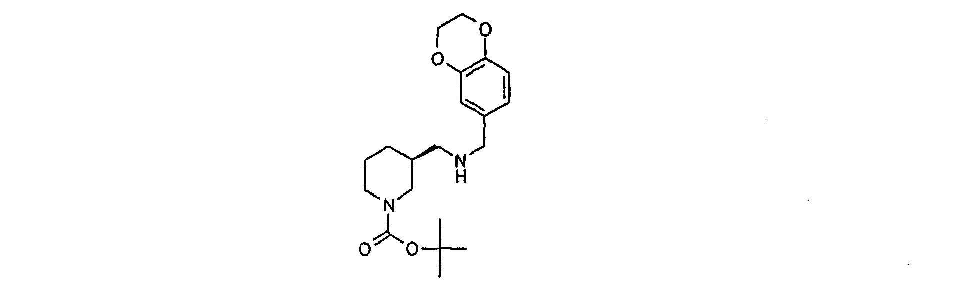 Zu einer Lösung von 3S-Aminomethyl-piperidin-1-*tert-*butylcarbamat (Beispiel 5.a) (0.158g, 0.5mmol) in MeOH (8mL) and Dichlormethan (27mL) wurden Molekularsieb Typ 3A (9.6g) und und 2,3-Dihydro-benzo[1,4]dioxin-6-carbaldehyd (0.078g, 0.5 mmol) gegeben. Das Reaktionsgemisch wurde 16 Stunden bei Raumtemperatur gerührt und Natriumborhydrid (0.05g, 1.35mmol) wurde zugegeben. Das Reaktionsgemisch wurde 2 Stunden bei Raumtemperatur gerührt dann über Hydromatrix (benetzt mit einer NaHCO₃ Lösung, 20mL) filtriert und mit Dichlormethan nachgewaschen. Das Filtrat wurde zur Trockne einrotiert. Der Rückstand wurde mittels Säulenchromatographie an Kieselgel (Dichlormethan/MeOH 19/1) gereinigt.
Ausbeute: 2.2g. 6.0mmol (Schaum)
MS (EI) m/z 363.4 [M-H]⁺

8.b) (R)-(2,3-Dihydro-benzo[1,4]dioxin-6-ylmethyl)-piperidin-3-ylmethyl-amin 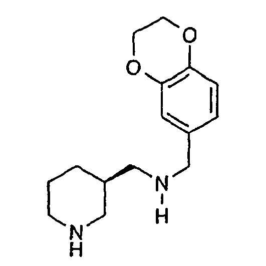 Eine Lösung von 3S-{[(2,3-Dihydro-benzo[1,4]dioxin-6-ylmethyl)-amino]-methyl}-piperidin-1-*tert*-butylcarbamat (2.2 g, 6mmol) in TFA (10 ml) wurde 20 Minuten bei Raumtemperatur gerührt. Das Reaktionsgemisch wurde eingeengt und in wässriger 2N NaOH aufgenommen. Die wässrige Phase wurde mit Dichlormethan-MeOH (9-1) extrahiert, die vereinten organischen Phasen über Natriumsulfat getrocknet, filtriert und zur Trockne einrotiert.
Ausbeute: 1.18 g, 4.53 mmol (Schaum)
MS (EI) m/z 263.4 [M+H]⁺

8.c) 2-((3S)-{[(2,3-Dihydro-benzo[1,4]dioxin-6-ylmethyl)-amino]-methyl}-piperidin-1-yl)-(1S)-(6-methoxy-chinolin-4-yl)-ethanol 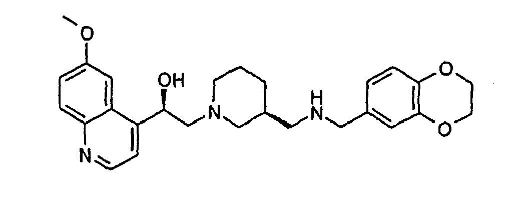 In analoger Weise zu Beispiel 1.f wurde die Titelverbindung in 36% Ausbeute hergestellt (MS (EI) m/z 464.5 [M+H]⁺).
(*R*)-6-Methoxy-4-oxiranylchinolin wurde analog WO 02/50040 hergestellt.

### Beispiel 9: 2-((3S)-{[(2,3-Dihydro-benzo[1,3]dioxo-6-ylmethyl)-amino]-methyl}-piperidin-1-yl)-(1RS)-(6-methoxy-chinolin-4-yl)-ethanol

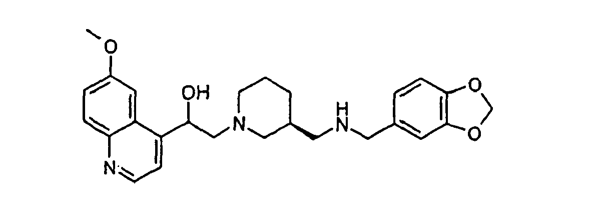

In analoger Weise zu Beispiel 1f wurde die Titelverbindung in 44% Ausbeute hergestellt (MS (EI) m/z 450 [M+H]⁺).

### Beispiel 10 : 4-[(Benzo[1.3]dioxol-5-ylmethyl)-amino]-1-[2-(6-methoxy-chinazolin-4-yl)-ethyl]-cyclohexanol.

10.a) 8-([2-(6-Methoxy-chinazolin-4-yl)-ethyl]-1,4-dioxaspiro-[4.5]decan-8-ol

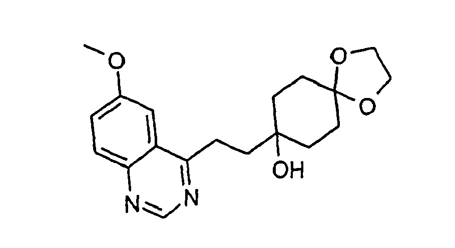

Zu einer Lösung von 8-(6-Methoxy-chinazolin-4-ylethinyl)-1,4-dioxa-spiro[4.5]decan-8-ol (0.96g, 2.88mmol) in EtOH (40mL) und THF (10ml) wurde Platinoxid (0.46g) zugegeben und unter Wasserstoffatmosphäre (1bar) hydriert. Das Reaktionsgemisch wurde in Gegenwart von Aktivkohle (5g) gerührt und abfiltriert. Das Filtrat wurde zur Trockne einrotiert. Der Rückstand wurde mittels Säulenchromatographie an Kieselgel (EtOAc dann EtOAc:MeOH 9/1) gereinigt.
Ausbeute: 0.623g (1.81mmol), Schaum
MS (EI) m/z 344 [M+H]⁺

8-(6-Methoxy-chinazolin-4-ylethinyl)-1,4-dioxa-spiro-[4.5]decan-8-ol wurde nach J. Chem. Soc. Perk. Trans. 1, 2000, 3382 hergestellt.

10.b) 4-Hydroxy-4-[2-(6-methoxychinazolin-4-yl)-ethyl]-cyclo-hexanon 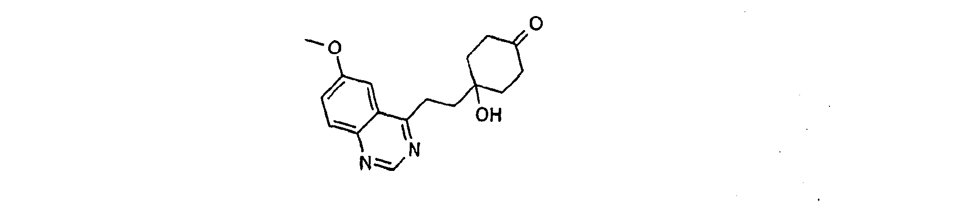 Eine Lösung von 8-([2-(6-Methoxy-chinazolin-4-yl)-ethyl]-1,4-dioxa-spiro[4.5]decan-8-ol (0.623g, 1.81mmol) in AcOH-THF-H₂O (3-2-2, 10mL) wurde während 30 Stunden bei 60°C gerührt. Das Reaktionsgemisch wurde zur Trockne einrotiert. Der Rückstand wurde mit Natriumbicarbonat (100mL) und Essigester (100 mL) verdünnt, die Phasen getrennt und die wässrige Phase zweimal mit je 50mL Essigester extrahiert. Die vereinten organischen Phasen wurden über MgSO₄ getrocknet, filtriert und zur Trockne einrotiert.
Ausbeute: 0.425g (1.41mmol) Schaum.
MS (EI) m/z 301 [M+H]⁺

10.c) 4-[(Benzo[1.3]dioxol-5-ylmethyl)-amino]-1-[2-(6-methoxy-chinazolin-4-yl)-ethyl]-cyclohexanol: 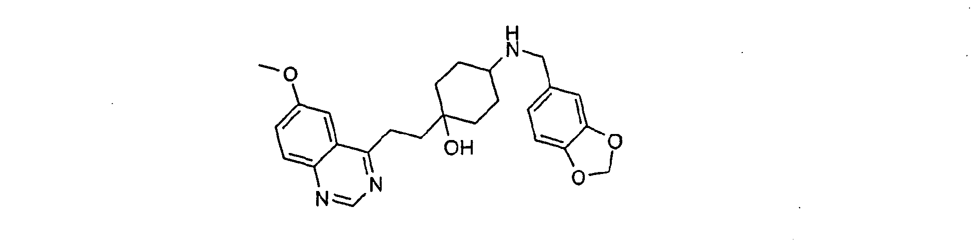 Zu einer Lösung von 4-Hydroxy-4-[2-(6-methoxy-chinazolin-4-yl)-ethyl]-cyclohexanon (0.06g, 0.2mmol) in Dichlormethan (1mL) wurden Piperonylamin (0.030mL, 0.24mmol) und Natriumtriacetoxyborhydrid (0.08g, 0.377mmol) zugegeben. Das Reaktionsgemisch wurde über Nacht gerührt dann über Hydromatrix (benetzt mit einer NaHCO₃ Lösung, 2mL) filtriert und mit Dichlormethan nachgewaschen. Das Filtrat wurde zur Trockne einrotiert. Der Rückstand wurde mittels Säulenchromatographie an Kieselgel (Dichlormethan:MeOH 9/1 dann Dichlormethan:MeOH 9/1 und 2% Triethylamin) gereinigt.
Ausbeute: 0.074g (0.17mmol) *cis*/*trans* Gemisch
MS (EI) m/z 436 [M+H]⁺

### Beispiel 11: 4-[(Benzo[1,2,5]thiadiazol-5-ylmethyl)-amino]-1-[2-(6-methoxy-chinazolin-4-yl)-ethyl]-cyclohexanol

11.a) 4-Amino-1-[2-(6-methoxy-chinazolin-4-yl)-ethyl]-cyclohexanol 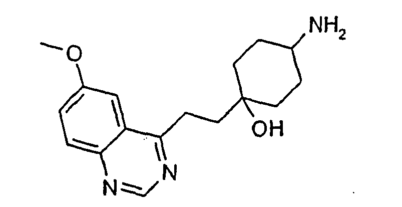 Zu einer Lösung von 4-Hydroxy-4-[2-(6-methoxy-chinazolin-4-yl)-ethyl]-cyclohexanon (1.25 g, 4.2mmol) in Methanol (50mL) wurden Ammoniumacetat (14 g, 44mmol) und Natriumtriacetoxyborhydrid (1.2 g, 5mmol) zugegeben. Das Reaktionsgemisch wurde über Nacht gerührt und zur Trockne einrotiert. Der Rückstand wurde mit Wasser (100mL) und Dichlormethan (100 mL) verdünnt, die Phasen getrennt und die wässrige Phase zweimal mit je 50mL Dichlormethan extrahiert. Die vereinten organischen Phasen wurden über Na₂SO₄ getrocknet, filtriert und zur Trockne einrotiert.
Ausbeute: 0.85g. 2.82mmol (Oel) *cis*/*trans* Gemisch
MS (EI) m/z 302 [M+H]⁺

11.b) 4-[(Benzo[1,2,5]thiadiazol-5-ylmethyl)-amino]-1-[2-(6-methoxy-chinazolin-4-yl)-ethyl]-cyclohexanol 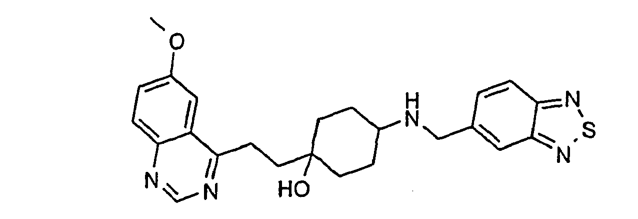 Zu einer Lösung von 4-Amino-1-[2-(6-methoxy-chinazolin-4-yl)-ethyl]-cyclohexanol (0.116 g, 0.38mmol) in Methanol (1mL) und Dichlorethan (3mL) wurden Molekularsieb Typ 3A (1g) und Benzo[1,2,5]thiadiazol-5-carbaldehyd (0.066g, 0.4 mmol) gegeben. Das Reaktionsgemisch wurde 20 Stunden bei Raumtemperatur gerührt und Natriumtriacetoxyborhydrid (0.22g, 1.04mmol) wurde zugegeben. Das Reaktionsgemisch wurde 2 Stunden bei Raumtemperatur gerührt dann über Hydromatrix (benetzt mit einer NaHCO₃ Lösung, 2mL) filtriert und mit Dichlormethan nachgewaschen. Das Filtrat wurde zur Trockne einrotiert. Der Rückstand wurde mittels Säulenchromatographie an Kieselgel (Dichlor-methan/MeOH 9/1 1% NH₄OH).
Ausbeute: 0.112g. 0.24mmol (Oel)cis/txans-Gemisch.
MS (EI) m/z 450 [M+H]⁺

### Beispiel 12: 1-[2-(6-Methoxy-chinazolin-4-yl)-ethyl]-4-(3-phenyl-allylamino)-cyclohexanol

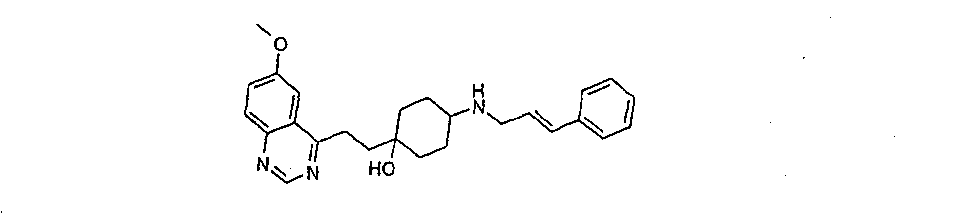

In analoger Weise wurde ausgehend von 4-Amino-1-[2-(6-methoxy-chinazolin-4-yl)-ethyl]-cyclohexanol (Beispiel 11.b), 1-[2-(6-Methoxy-chinazolin-4-yl)-ethyl]-4-(3-phenyl-allylamino)-cyclohexanol als *cis*/*trans*-Gemisch in 54% Ausbeute hergestellt (MS (EI) m/z 418 [M+H]⁺).

### Beispiel 13: 4-(3-Furan-2-yl-allylamino)-1-[2-(6-methoxy-chinazolin-4-yl)-ethyl]-cyclohexanol

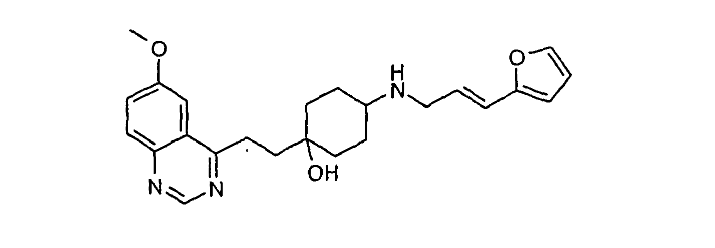

In analoger Weise wurde ausgehend von 4-Amino-1-[2-(6-methoxy-chinazolin-4-yl)-ethyl]-cyclohexanol (Beispiel 11.b), 4-(3-Furan-2-yl-allylamino)-1-[2-(6-methoxy-chinazolin-4-yl)-ethyl]-cyclohexanol als *cis*/*trans*-Gemisch in 62% Ausbeute hergestellt (MS (EI) m/z 408 [M+H]⁺).

### Beispiel 14: 4-[(2,3-Dihydro-benzo[1,4]dioxin-6-ylmethyl)-amino]-1-[2-(6-methoxy-chinazolin-4-yl)-ethyl]-cyclohexanol

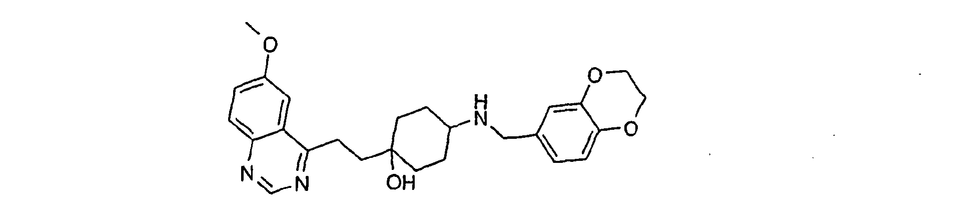

In analoger Weise wurde ausgehend von 4-Amino-1-[2-(6-methoxy-chinazolin-4-yl)-ethyl]-cyclohexanol (Beispiel 11.b), 4-[(2,3-Dihydro-benzo[1,4]dioxin-6-ylmethyl)-amino]-1-[2-(6-methbxy-chinazolin-4-yl)-ethyl]-cyclohexanol als *cis*/*trans*-Gemisch in 68% Ausbeute hergestellt (MS (EI) m/z 450 [M+H]⁺).

### Beispiel 15: 1-[2-(6-Methoxy-chinazolin-4-yl)-ethyl]-4-[(chinoxalin-2-ylmethyl)-amino]-cyclohexanol

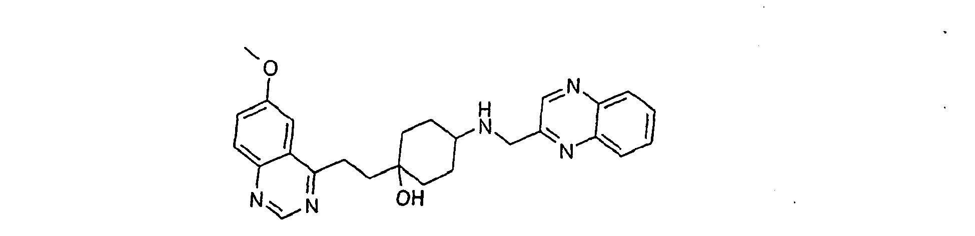

In analoger Weise wurde ausgehend von 4-Amino-1-[2-(6-methoxy-chinazolin-4-yl)-ethyl]-cyclohexanol (Beispiel 11.b), 1-[2-(6-Methoxy-chinazolin-4-yl)-ethyl]-4-[(quinoxalin-2-ylmethyl)-amino]-cyclohexanol als *cis*/ *trans*-Gemisch in 59% Ausbeute hergestellt (MS (EI) m/z 450 [M+H]⁺) .

### Beispiel 16: cis und trans-4-[(Benzo[1,3]dioxol-5-ylmethyl)-amino]-1-[2-(6-methoxy-[1,5]naphthyridin-4-yl)-ethyl]-cyclohexanol

16 a) 8-(6-Methoxy-[1,5]naphthyridin-4-ylethinyl)-1,4-dioxa-spiro[4.5]decan-8-ol 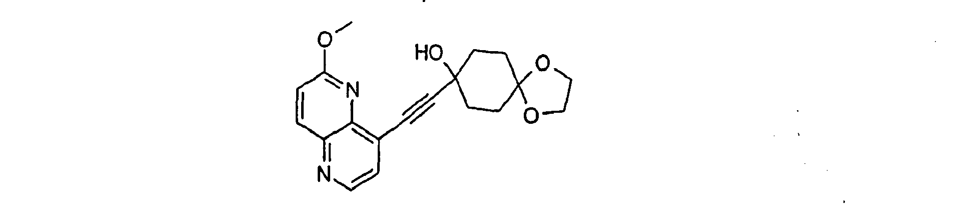 Eine entgaste Lösung von Trifluoromethanesulfonsäure 6-methoxy-[1,5]naphthyridin-4-yl ester (986 mg, 3.2 mmol) (WO 03 010138) und 8-Ethinyl-1,4-dioxa-spiro[4.5]decan-8-ol (638 mg, 3.5 mmol) (hergestellt nach J. Chem. Soc. Perk. Trans. 1, 2000, 3382) in DMF (3 ml) wurde zu einer ebenfalls entgasten Suspension von Kupfer(I)iodid (50 mg) und PdCl₂(PPh₃)₂ (100 mg) in DMF (2 ml) und Triethylamin (3 ml) getropft. Das Reaktionsgemisch wurde während 30
Minuten bei Raumtemperatur gerührt, mit Wasser und Ether verdünnt und die organische Phase mit Wasser, ges. Ammoniumchloridlösung und ges. Kochsalzlösung gewaschen, über MgSO₄ getrocknet und eingeengt. Das Rohprodukt wurde mittles Chromatographie an Kieselgel (Hex/EtOAc 1:1, EtOAc) gereinigt.
Ausbeute: 0.965g. 2.8 mmol (Oel).
MS (EI) m/z 341 [M+H]⁺

16.b) 8-[2-(6-Methoxy-[1,5]naphthyridin-4-yl)-ethyl]-1,4-dioxa-spiro[4.5]decan-8-ol 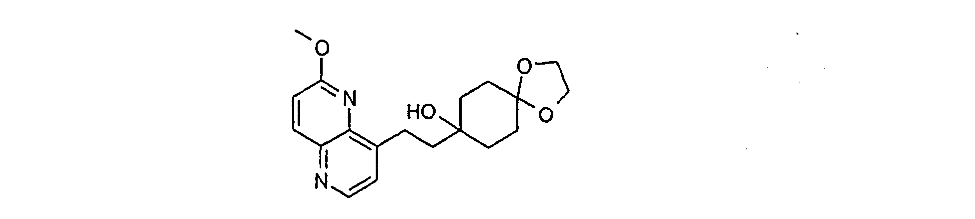 Eine Lösung von 8-(6-Methoxy-[1,5]naphthyridin-4-yl-ethinyl)-1,4-dioxa-spiro[4.5]decan-8-ol (965 mg, 2.8 mmol) in Ethanol (100 ml) wurde während 6h über PtO₂ (200 mg) bei 1 bar Wasserstoff hydriert. Der Katalysator wurde abfiltriert und durch frischen ersetzt und die Hyrdierung während weiteren 3h fortgesetzt. Der Katalysator wurde abfiltriert und das Lösungsmittel eingeengt. Das Rohprodukt wurde mittels Chromatographie an Kieselgel (EtOAc) gereinigt.
Ausbeute: 0.960g, 2.79mol (Oel).
MS (EI) m/z 334.4 [M+H]⁺

16.c) 4-Hydroxy-4-[2-(6-methoxy-[1,5]naphthyridin-4-yl)-ethyl]-cyclohexanon 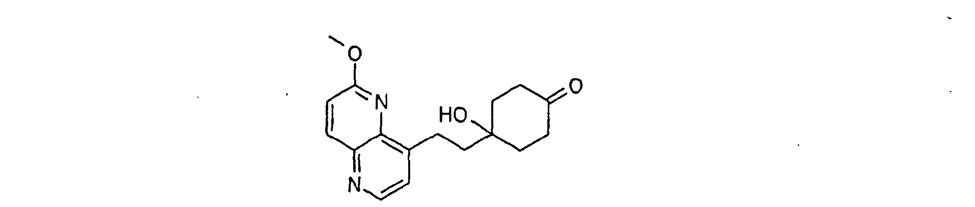 Eine Lösung von 8-[2-(6-Methoxy-[l,5]naphthyridin-4-yl)-ethyl]-1,4-dioxa-spiro[4.5]decan-8-ol (960 mg, 2.79 mmol) in THF/H₂O/AcOH (2:2:3, 22ml) wurde über Nacht bei 65 C gerührt. Das Reaktionsgemisch wurde eingeengt und mittels Chromatographie an Kieselgel (EtOAc) gereinigt.
Ausbeute: 0.700g, 2.33mmol (Oel).
MS (EI) m/z 301 [M+H]⁺

16.d) *cis* und *trans*-4-[(Benzo[1,3]dioxol-5-ylmethyl)-amino]-1-[2-(6-methoxy-[1,5]naphthyridin-4-yl)-ethyl]-cyclohexanol 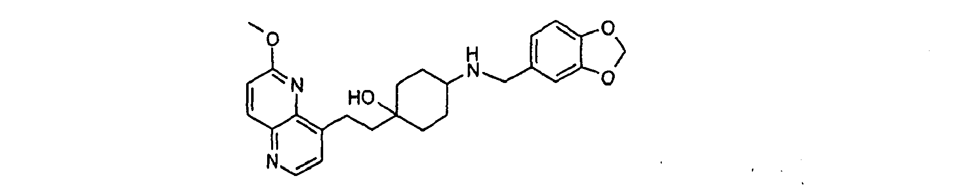 Zu einer Lösung von 4-Hydroxy-4-[2-(6-methoxy-[1,5]-naphthyridin-4-yl)-ethyl]-cyclohexanon (100 mg, 0.3 mmol) und Piperonylamin (100ul) in THF (3 ml) wurde NaBH(OAc)₃ (100 mg) zugegeben. Das Reaktionsgemisch wurde während 4h bei Raumtemperatur gerührt, mit Dichlormethan und Ammoniumhydroxid verdünnt. Die organische Phase wurde über MgSO₄ getrocknet und eingeengt. Der Rückstand wurde mittels Chromatographie an Kieselgel (EtOAc/MeOH 9:1 +1% NH₄OH) gereinigt. Man erhielt *cis*-4-[(Benzo[1,3]dioxol-5-ylmethyl)-amino]-1-[2-(6-methoxy-[1,5]naphthyridin-4-yl)-ethyl]-cyclohexanol (38 mg, MS (EI) m/z 357 [M+H]⁺) und *trans*-4-[(Benzo[1,3]dioxol-5-ylmethyl)-amino]-1-[2-(6-methoxy-[1,5]naphthyridin-4-yl)-ethyl]-cyclohexanol (63 mg, MS (EI) m/z 357 [M+H]⁺)

### Beispiel 17: cis und trans-4-[(Benzo[1,3]dioxo-5-ylmethyl)-amino]-1-[2-(6-methoxy-[1,5]naphthyridin-4-yl)-ethyl]-cyclohexanol

17.a) 4-[2-(6-Ethoxy-chinolin-4-yl)-ethyl]-4-hydroxy-cyclohexanon 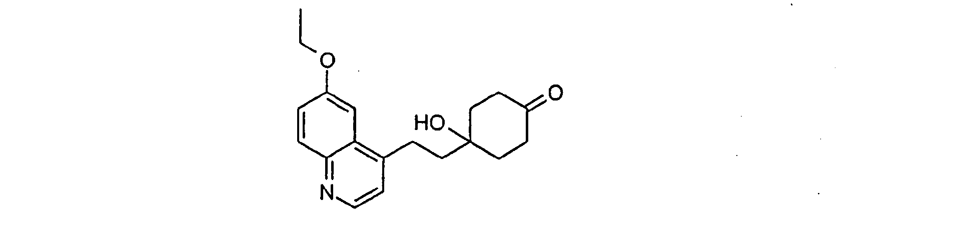 In analoger Weise zu Beispiel 16.c, 4-[2-(6-Ethoxy-chinolin-4-yl)-ethyl]-4-hydroxy-cyclohexanon wurde aus 6-Ethoxy-chinolin-4-ol hergestellt. MS (EI) m/z 314 [M+H]⁺).
6-Ethoxychinolin-4-ol wurde bereits in Synth. Comm. 2002, 32, 3185 beschrieben.

### 17.b) cis und trans-4-[(Benzo[1,3]dioxo-5-ylmethyl)-amino]-1-[2-(6-methoxy-[1,5]naphthyridin-4-yl)-ethyl]-cyclohexanol

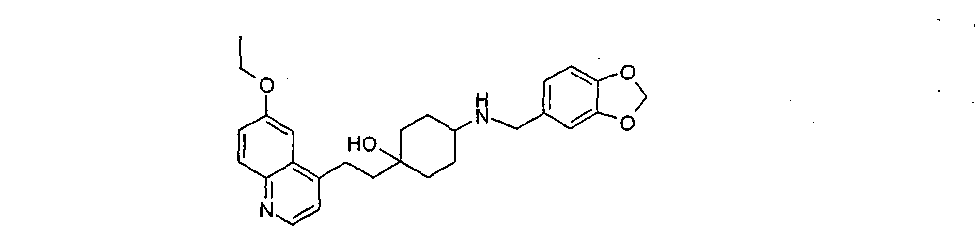

In analoger Weise zu Beispiel 16.d erhielt man cis-4-[(Benzo[1,3]dioxol-5-ylmethyl)-amino]-1-[2-(6-methoxy-[1,5]naphthyridin-4-yl)-ethyl]-cyclohexanol (48% Ausbeute, MS (EI) m/z 449 [M+H]⁺) und *trans*-4-[(Benzo[1,3]dioxol-5-ylmethyl)-amino]-1-[2-(6-methoxy-[1,5]naphthyridin-4-yl)-ethyl]-cyclohexanol (29% Ausbeute, MS (EI) m/z 449 [M+H]⁺).

### Beispiel 18: Synthese von cis- und trans-4-[(2,3-Dihydrobenzo[1,4]dioxin-6-ylmethyl)-amino]-1-[2-(6-methoxy-[1,5]naphthyridin-4-yl)-ethyl]-cyclohexanol

18.a) 4-Amino-1-[2-(6-methoxy-[1,5]naphthyridin-4-yl)-ethyl]-cyclohexanol 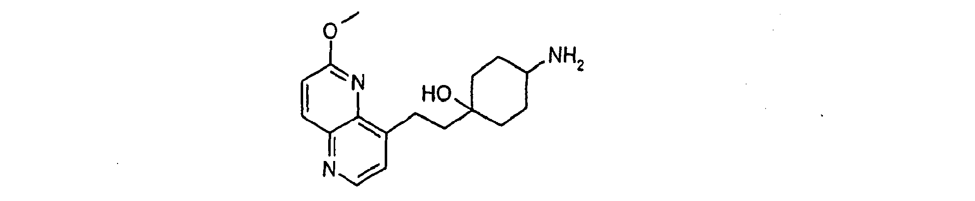 Zu einer Lösung von 4-Hydroxy-4-[2-(6-methoxy-[1,5]naphthyridin-4-yl)-ethyl]-cyclohexanon (3g, 8.5 mmol) und Ammoniumacetat (25.5g) in Methanol (65ml) wurde Natriumtriacetoxyborhydrid (1.89g, 8.75 mmol) zugegeben. Das Reaktionsgemisch wurde während 3h bei Raumtemperatur gerührt, 0.5g Natriumtriacetoxyborhydrid zugegeben und weitere 2h gerührt. Das Reaktionsgemisch wurde mit NH₄OH verdünnt und mit EtOAc und Dichlormethan extrahiert. Organische Phasen wurden über MgSO₄ getrocknet und eingeengt.
Ausbeute: 2.6 g, (Oel), *cis*/*trans* Gemisch
MS (EI) m/z 302 [M+H]⁺

18.b) *cis-* und *trans*-4-[(2,3-Dihydro-benzo[1,4]dioxin-6-ylmethyl)-amino]-1-[2-(6-methoxy-[1,5]naphthyridin-4-yl)-ethyl]-cyclohexanol 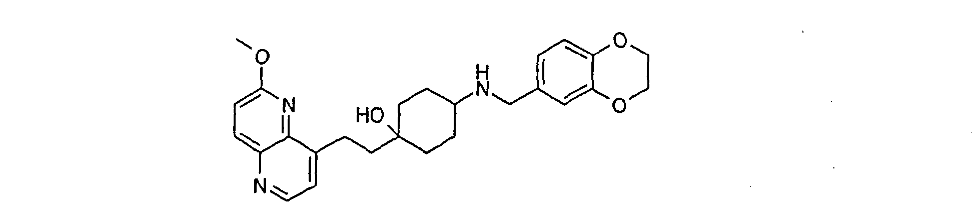 Zu einer Lösung von 4-Amino-1-[2-(6-methoxy-[1,5]naphthyridin-4-yl)-ethyl]-cyclohexanol (1250 mg, 4.14 mmol) und 2,3-Dihydro-benzo[1,4]dioxin-6-carbaldehyd (680 mg, 4.14 mmol) in Methanol (25 mL) und THF (25 mL) wurde Molekularsieb Typ 3A (2.5g) gegeben. Das Reaktionsgemisch wurde 20 Stunden bei Raumtemperatur gerührt und Natriumborhydrid (157 mg, 4.14 mmol) wurde zugegeben. Das Reaktionsgemisch wurde 1 Stunde bei Raumtemperatur gerührt dann über Hydromatrix (benetzt mit NH₄OH) filtriert und mit Dichlormethan nachgewaschen. Das Filtrat wurde zur Trockne einrotiert. Der Rückstand wurde mittels Säulenchromatographie an Kieselgel (EtOAc/MeOH 9/1, 1% NH₄OH)gereinigt.
Man erhielt *cis*-4-[(2,3-Dihydro-benzo[1,4]dioxin-6-yl-methyl)-amino]-1-[2-(6-methoxy-[1,5]naphthyridin-4-yl)-ethyl]-cyclohexanol (640 mg, 34%, MS (EI) m/z 450 [M+H]⁺) und *trans*-4-[(2,3-Dihydro-benzo[1,4]dioxin-6-ylmethyl)-amino]-1-[2-(6-methoxy-[1,5]naphthyridin-4-yl)-ethyl]-cyclohexanol (360 mg, 19%, MS (EI) m/z 450 [M+H]⁺)

### Beispiel 19: cis- und trans-6-({4-Hydroxy-4-[2-(6-methoxy-[1,5]naphthyridin-4-yl)-ethyl]-cyclohexylamino}-methyl)-4H-benzo[1,4]oxazin-3-on

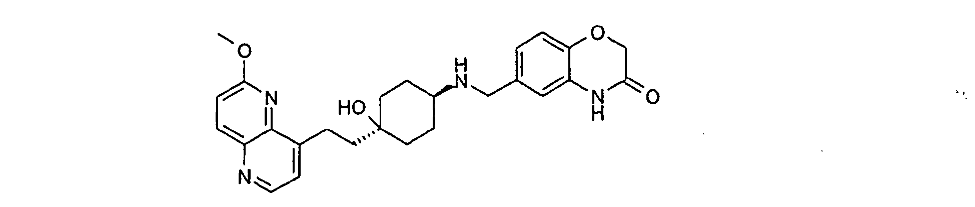

In analoger Weise zu Beipiel 18 wurde ausgehend von 4-Amino-1-[2-(6-methoxy-[1,5]naphthyridin-4-yl)-ethyl]-cyclohexanol und 3-Oxo-3,4-dihydro-2H-benzo[1,4]oxazin-6-carbaldehyd *cis*-6-({4-Hydroxy-4-[2-(6-methoxy-[1,5]naphthyridin-4-yl)-ethyl]-cyclohexylamino}-methyl)-4H-benzo-[1,4]oxazin-3-on (330 mg, 17%, MS (EI) m/z 464 [M+H]⁺) und *trans*-6-({4-Hydroxy-4-[2-(6-methoxy-[1,5]naphthyridin-4-yl)-ethyl]-cyclohexylamino}-methyl)-4H-benzo[1,4]oxazin-3-on (980 mg, 44%, MS (EI) m/z 464 [M+H]⁺) erhalten.

### Beispiel 20: cis- und trans 4-[(2,3-Dihydrobenzo[1,4]dioxin-6-ylmethyl)-amino]-1-[2-(6-methoxy-chinolin-4-yl)-ethyl]-cyclohexanol

20.a) 4-Amino-1-[2-(6-methoxy-chinolin-4-yl)-ethyl]-cyclohexanol 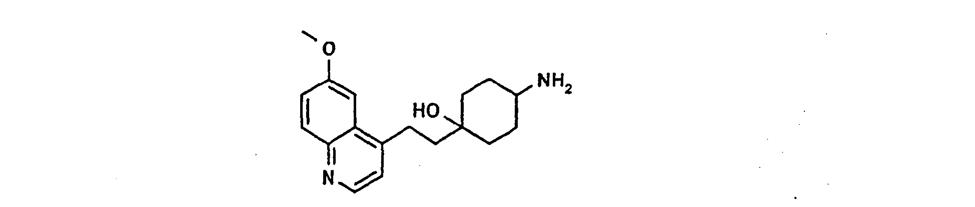 4-Amino-1-[2-(6-methoxy-chinolin-4-yl)-ethyl]-cyclohexanol wurde ausgehend von Trifluoromethanesulfonsäure-6-methoxy-chinolin-4-yl ester und 8-Ethenyl-1,4-dioxa-spiro[4.5]decan-8-ol analog der Sequenz aus Beipiel 14 und 15 erhalten.

20.b) 4-[(2,3-Dihydro-benzo[1,4]dioxin-6-ylmethyl)-amino]-1-[2-(6-methoxy-chinolin-4-yl)-ethyl]-cyclohexanol 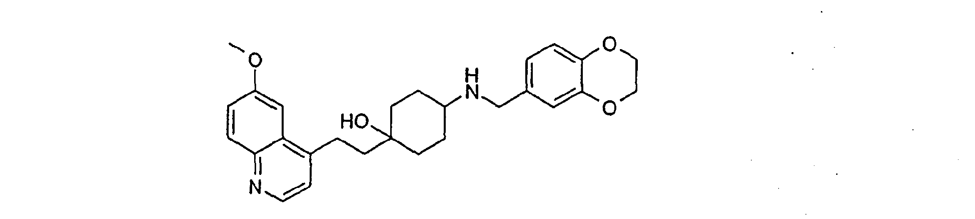 In Analogie zu Beipiel 18 wurde ausgehend von 4-Amino-1-[2-(6-methoxy-chinolin-4-yl)-ethyl]-cyclohexanol und 2,3-Dihydro-benzo[1,4]dioxin-6-carbaldehyd *cis*-4-[(2,3-Dihydrobenzo[1,4]dioxin-6-ylmethyl)-amino]-1-[2-(6-methoxy-chinolin-4-yl)-ethyl]-cyclohexanol (50 mg, MS (EI) m/z 449.5 [M+H]⁺) und *trans*-4-[(2,3-Dihydro-benzo[1,4]dioxin-6-ylmethyl)-amino]-1-[2-(6-methoxy-quinolin-4-yl)-ethyl]-cyclohexanol (56 mg, MS (EI) m/z 449.5 [M+H]⁺) erhalten.

### Beispiel 21: cis- und trans-6-({4-Hydroxy-4-[2-(6-methoxy-chinolin-4-yl)-ethyl]-cyclohexylamino}-methyl)-4H-benzo[1,4]oxazin-3-on

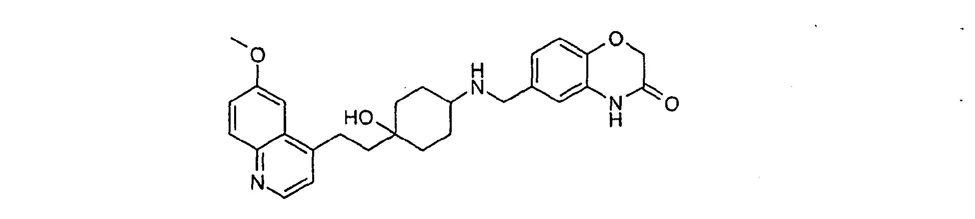

In Analogie zu Beipiel 18 wurde ausgehend von 4-Amino-1-[2-(6-methoxy-chinolin-4-yl)-ethyl]-cyclohexanol und 3-Oxo-3,4-dihydro-2H-benzo[1,4]oxazin-6-carbaldehyd cis-6-({4-Hydroxy-4-[2-(6-methoxy-chinolin-4-yl)-ethyl]-cyclohexylamino}-methyl)-4H-benzo[1,4]oxazin-3-on (32 mg, MS (EI) m/z 462.6 [M+H]⁺) und *trans*-6-({4-Hydroxy-4-[2-(6-methoxy-chinolin-4-yl)-ethyl]-cyclohexylamino}-methyl)-4H-benzo[1,4]oxazin-3-on (40 mg, MS (EI) m/z 462.6 [M+H]⁺) erhalten.

### Beispiel 22: 4-[(2,3-Dihydro-[1,4]dioxino[2,3-c]pyridin-7-ylmethyl)-amino]-1-[2-(6-methoxy-chinolin-4-yl)-ethyl]-cyclohexanol

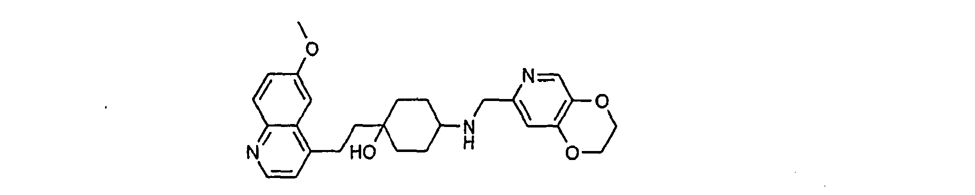

In Analogie zu Beispiel 11.b, wurde 4-[(2,3-Dihydro-[1,4]dioxino[2,3-c]pyridin-7-ylmethyl)-amino]-1-[2-(6-methoxychinolin-4-yl)-ethyl]-cyclohexanol in 72% Ausbeute als *cis*/*trans* Gemisch erhalten (MS (EI) m/z 450 [M+H]⁺).

2,3-Dihydro-[1,4]dioxino[2,3-c]pyridin-7-carbaldehyd wurde nach WO 03/010138 hergestellt. Beispiel 23: 4-[(Benzo[1,2,5]thiadiazol-5-ylmethyl)-amino]-1-[2-(6-methoxy-chinolin-4-yl)-ethyl]-cyclohexanol

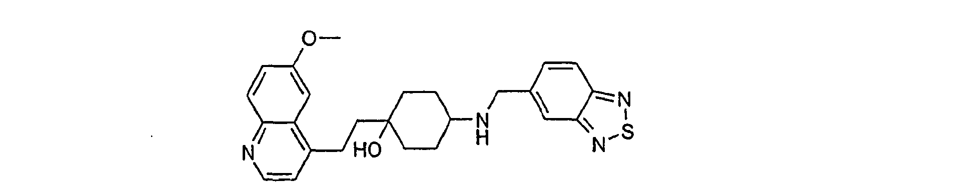

In Analogie zu Beispiel 11.b, wurde 4-[(Benzo[1, 2, 5]-thiadiazol-5-ylmethyl)-amino]-1-[2-(6-methoxy-chinolin-4-yl)-ethyl]-cyclohexanol in 47% Ausbeute als *cis*/*trans* Gemisch erhalten (MS (EI) m/z 449 [M+H]⁺).

### Beispiel 24: 1-[2-(6-Methoxy-chinolin-4-yl)-ethyl]-4-(3-phenyl-allylamino)-cyclohexanol

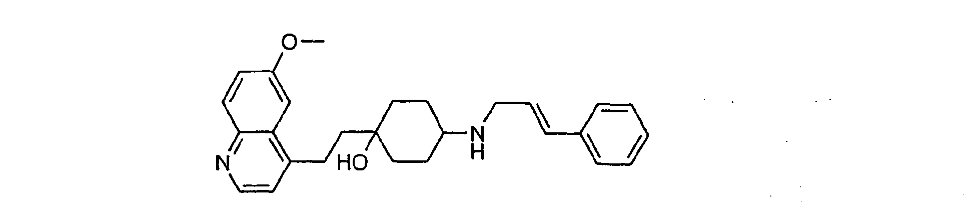

In Analogie zu Beispiel 11.b, wurde 1-[2-(6-Methoxy-chinolin-4-yl)-ethyl]-4-(3-phenyl-allylamino)-cyclohexanol in 46% Ausbeute als *cis*/*trans* Gemisch erhalten (MS (EI) m/z 418 [M+H]⁺).

### Beispiel 25: 1-[2-(6-Methoxy-chinolin-4-yl)-ethyl]-4-[(chinoxalin-2-ylmethyl)-amino]-cyclohexanol

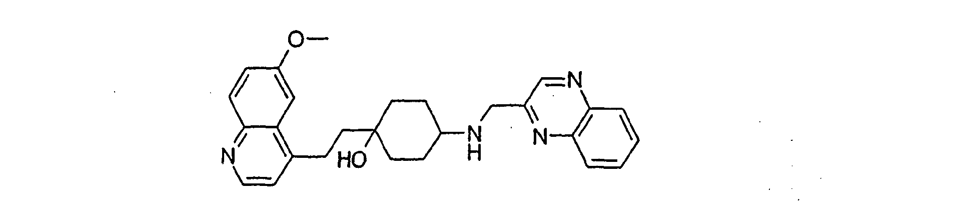

In Analogie zu Beispiel 11.b, wurde 1-[2-(6-Methoxy-chinolin-4-yl)-ethyl]-4-[(chinoxalin-2-ylmethyl)-amino]-cyclohexanol in 61% Ausbeute als *cis*/*trans* Gemisch erhalten (MS (EI) m/z 443[M+H]⁺).

### Beispiel 26: 6-({4-Hydroxy-4-[2-(6-methoxy-chinolin-4-yl)-ethyl]-cyclohexylamino}-methyl)-2,3-dihydrobenzo[1,4]dioxin-5-ol

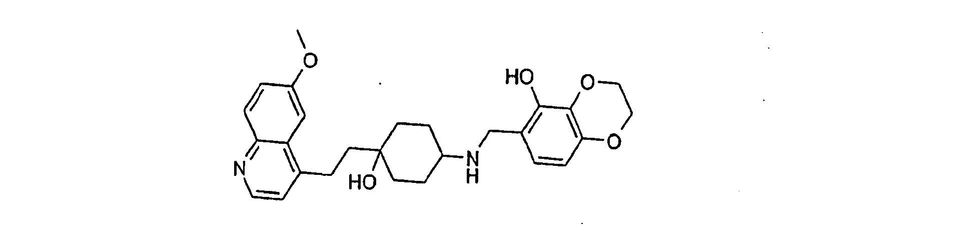

In Analogie zu Beispiel 11.b, wurde 6-({4-Hydroxy-4-[2-(6-methoxy-chinolin-4-yl)-ethyl]-cyclohexylamino}-methyl)-2,3-dihydro-benzo[l,4]dioxin-5-ol in 19% Ausbeute als *cis*/*trans* Gemisch erhalten (MS (EI) m/z 465[M+H]⁺).
Der entsprechende Aldehyd wurde analog J. Heterocycl. Chem 1989, 26, 193-197 hergestellt.

### Beispiel 27: 4-[(2-Chloro-chinolin-3-ylmethyl)-amino]-1-[2-(6-methoxy-chinolin-4-yl)-ethyl]-cyclohexanol

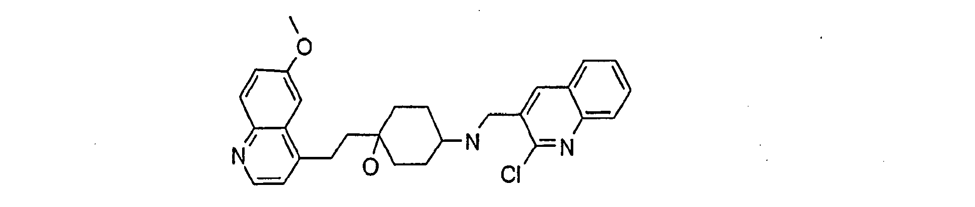

In Analogie zu Beispiel 11.b, wurde 4-[(2-Chloro-chinolin-3-ylmethyl)-amino]-1-[2-(6-methoxy-chinolin-4-yl)-ethyl]-cyclohexanol in 56% Ausbeute als *cis*/*trans* Gemisch erhalten (MS (EI) m/z 477[M+H]⁺).

### Beispiel 28: Synthese von 2-{4-Hydroxy-4-[2-(6-methoxy-chinolin-4-yl)-ethyl]-cyclohexylamino}-N-pyridin-2-yl-acetamid

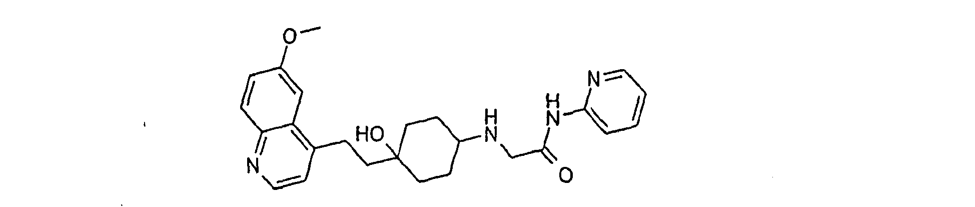

Zu einer Lösung von 4-Amino-1-[2-(6-methoxy-chinolin-4-yl)-ethyl]-cyclohexanol (0.094g, 0.31 mmol) in DMF (3.5mL) wurde 2-Bromo-N-pyridin-2-yl-acetämide (0.068g, 0.31mmol) und Kaliumcarbonat (0.045g, 0.34mmol) gegeben. Das Reaktionsgemisch wurde 2 Tage bei Raumtemperatur gerührt dann zur Trockne einrotiert. Der Rückstand wurde mittels Säulenchromatographie an Kieselgel (Dichlormethan/MeOH 19/1 1% Ammoniumhydroxid) gereinigt.
Ausbeute:0.11g (0.25mmol) als *cis*/*trans*-Gemisch
MS (EI) m/z 435 [M+H]⁺

2-Bromo-N-pyridin-2-yl-acetamide wurde bereits in WO 02/24684 beschrieben.

### Beispiel 29: Benzo[1,3]dioxol-5-ylmethyl-{4-[2-(6-methoxy-chinolin-4-yl)-ethyl]-cyclohexyl}-amin.

29.a) 8-Ethinyl-1,4-dioxa-spiro[4.5]decan: 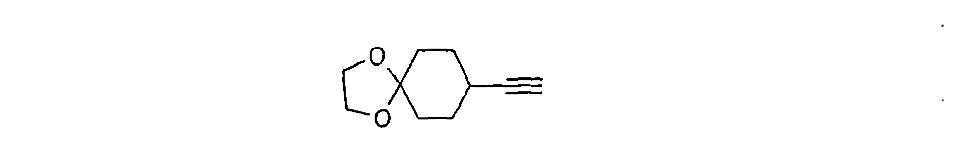 Zu einer Lösung von Triphenylphosphin (19.6g, 74.6mmol) und 1,4-Dioxa-spiro[4.5]dec-8-carbaldehyd (5g, 29.37mmol) in Dichlormethan (100mL) wurde bei -30°C eine Lösung von Tetrabromkohlenstoff (12.4 g, 37.4mmol) in Dichlormethan (40mL) zugegeben. Nachdem die Lösung 2 Stunden bei Raumtemperatur gerührt hatte, wurde sie zur Trockne einrotiert. Der Rückstand wurde mit Essigester und n-Hexan (1:3; 500ml) verdünnt, über Celite filtriert und zur Trockne einrotiert. Der Rückstand wurde mittels Säulenchromatographie an Kieselgel (Hexan/EtOAc 4/1) gereinigt. Ausbeute: 6.08g (18.6mmol). Dieses Material wurde in THF gelöst (90mL) und bei -78°C mit n-BuLi (16.5mL, 38mmol 2.3N in Hexan) tropfenweise versetzt. Nachdem die Lösung 1 Stunde bei -78°C gerührt hatte, wurde eine Lösung von 10% NaHSO₄ (50mL) zugegeben. Die wässerige Phase wurde dreimal mit je 50mL Essigester extrahiert, über MgSO₄ getrocknet, filtriert und zur Trockne einrotiert. Der Rückstand wurde mittels Säulenchromatographie an Kieselgel (Hexan/EtOAc 5/1) gereinigt.
Ausbeute: 2.74g (16.5mmol)
δ H (CDCl₃, 300MHz): 1.61 (m, 2H); 1.70-1.94 (m, 6H);
2.07 (d, J=2.5Hz, 1H); 2.51 (m, 1H); 3.96 (s, 4H).

29.b) 4-(1,4-Dioxaspiro[4.5]dec-8-ylethinyl)-6-methoxy-chinolin: 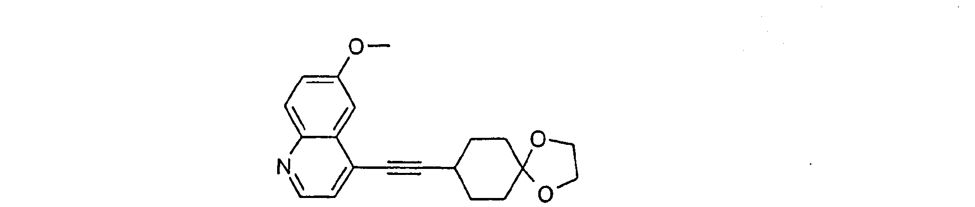 Zu einem Gemisch von PdCl₂(PPh₃)₂ (0.110 g, 0.157mmol) und CuI (0.055 g, 0.288mmol) wurde eine entgaste Lösung, von Trifluormethansulfonsäure-6-methoxychinolin-4-ylester (0.95g, 3.1mmol) und 4-(1,4-Dioxa-spiro[4.5]dec-8-yl-ethinyl)-6-methoxy-chinolin (0.514g, 3.1mmol) in DMF (6mL) und TEA (12mL) zugegeben. Nachdem die Lösung 1 Stunde bei Raumtemperatur gerührt hatte, wurde sie zur Trockne einrotiert. Der Rückstand wurde mittels Säulen-chromatographie an Kieselgel (EtOAc) gereinigt.
Ausbeute: 0.83g (2.56mmol)
MS (EI) m/z 324 [M+H]⁺

29.c) 4-[2-(1,4-Dioxaspiro[4.5]dec-8-yl)ethyl]-6-methoxy-chinolin: 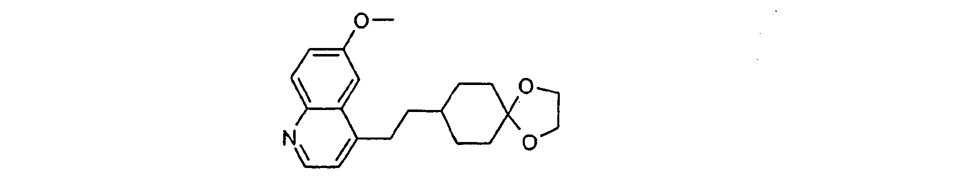 Zu einer Lösung von 4-(1,4-Dioxa-spiro[4.5]dec-8-yl-ethinyl)-6-methoxychinolin (0.83g, 2.53mmol) in EtOH (30mL) und Essigester (10ml) wurde Platinoxid ((0.462 g) zugegeben und unter Wasserstoffatmosphäre (1bar) hydriert. Der Katalysator wurde abfiltriert und das Filtrat zur Trockne einrotiert. Der Rückstand wurde mittels Säulenchromatographie an Kieselgel (EtOAc dann EtOAc-Methanol 9-1) gereinigt.
Ausbeute: 0.77g (2.35mmol)
MS (EI) m/z 328 [M+H]⁺

29.d) 4-[2-(6-Methoxy-chinolin-4-yl)-ethyl]-cyclohexanon: 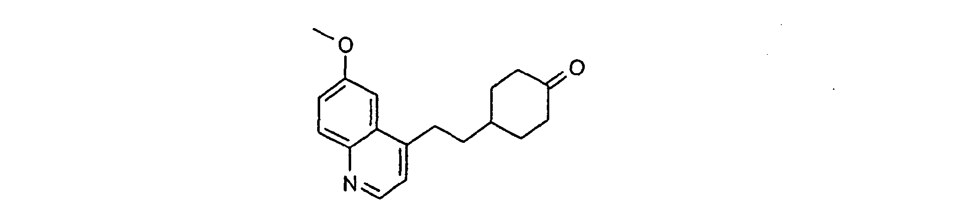 Eine Lösung von 4-[2-(1,4-Dioxa-spiro[4.5]dec-8-yl)-ethyl]-6-methoxy-chinolin (0.77g, 2.35mmol) in AcOH-THF-H₂O (3-2-2, 10mL) wurde während 10 Stunden bei 60°C gerührt. Das Reaktionsgemisch wurde zur Trockne einrotiert. Der Rückstand wurde mit EtOAc (100mL) verdünnt, mit NaHCO₃ (100mL) gewaschen, über MgSO₄ getrocknet, filtriert und zur Trockne einrotiert.
Ausbeute: 0.631g (2.23mmol)
MS (EI) m/z 284 [M+H]⁺

29.e) Benzo[1,3]dioxol-5-ylmethyl-{4-[2-(6-methoxy-chinolin-4-yl)-ethyl]-cyclohexyl}-amin: 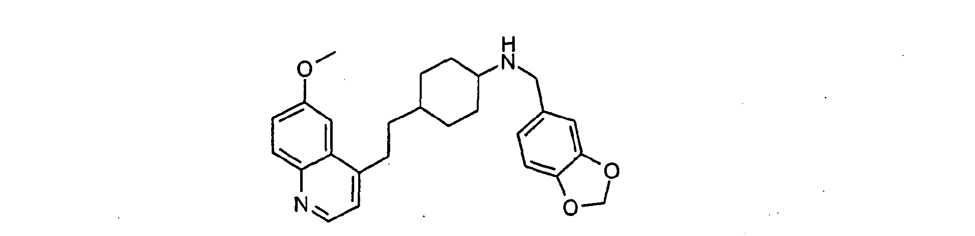 Zu einer Lösung von 4-[2-(6-Methoxy-chinolin-4-yl)-ethyl]-cyclohexanon (0.05g, 0.176mmol) in Dichlormethan (0.5mL) wurden Piperonylamin (0.038mL, 0.3mmol) und Natriumtriacetoxyborhydrid (0.05g) zugegeben. Das Reaktionsgemisch wurde über Nacht gerührt dann über Hydromatrix (benetzt mit einer NaHCO₃ Lösung, 2mL) filtriert und mit Dichlormethan nachgewaschen. Das Filtrat wurde zur Trockne einrotiert. Der Rückstand wurde mittels Säulenchromatographie an Kieselgel (EtOAc dann EtOAc:MeOH 9/1)gereinigt.
Ausbeute: 0.069g (0.165mmol) als *cis*/*trans* Gemisch MS (EI) m/z 419 [M+H]⁺

### Beispiel 30: 6-({4-[2-(6-Methoxy-chinolin-4-yl)-ethyl]-cyclohexylamino}-methyl)-4H-benzo[1,4]oxazin-3-on

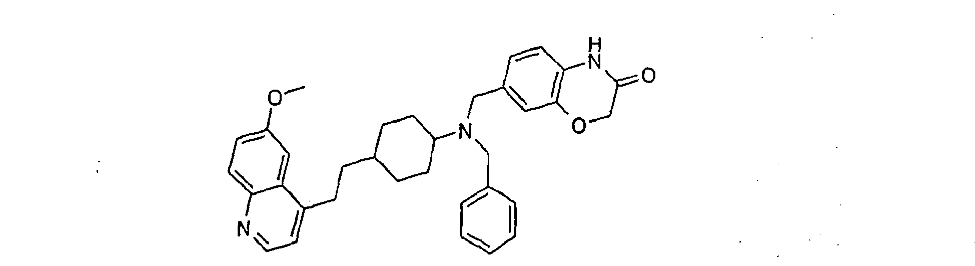

30.a) 7-[(Benzyl-{4-[2-(6-methoxy-chinolin-4-yl)-ethyl]-cyclohexyl}-amino)-methyl]-4H-benzo[1,4]oxazin-3-on
Zu einer Lösung von 4-[2-(6-Methoxy-chinolin-4-yl)-ethyl]-cyclohexanon (Beispiel 26.e)(0.35 g, 1.23mmol) in Dichlorethan (8mL) wurden Benzylamin (0.135 mL, 1.24mmol) und Natriumtriacetoxyborhydrid (0.315 g, 1.55mmol) zugegeben. Das Reaktionsgemisch wurde 2 Stunden gerührt. 3-Oxo-3,4-dihydro-2H-benzo[1,4]oxazin-6-carbaldehyd (0.230g, 1.3mmol), THF (4mL und Natriumtriacetoxyborhydrid (0.315 g, 1.55mmol) wurde zugegeben. Das Reaktionsgemisch wurde über Nacht gerührt und dann über Hydromatrix (benetzt mit einer NaHCO₃ Lösung, 10mL) filtriert und mit Dichlormethan nachgewaschen. Das Filtrat wurde zur Trockne einrotiert. Der Rückstand wurde mittels Säulenchromatographie an Kieselgel (Dichlormethan/MeOH 19/1)gereinigt.
Ausbeute: 0.540g (1.0mmol)
MS (EI) m/z 536 [M+H]⁺

30.b) 6-({4-[2-(6-Methoxy-chinolin-4-yl)-ethyl]-cyclohexylamino}-methyl)-4H-benzo[1,4]oxazin-3-on 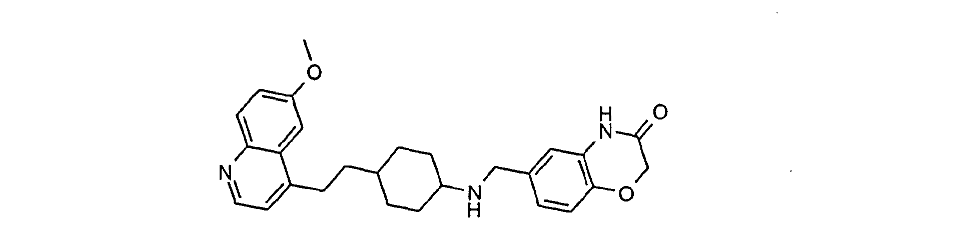 Zu einer Lösung von 7-[(Benzyl-{4-[2-(6-methoxy-chinolin-4-yl)-ethyl]-cyclohexyl}-amino)-methyl]-4H-benzo[1,4]-oxazin-3-on (0.535 g, 1mmol) in THF (15mL) und MeOH (5mL) wurde 20% Pd(OH)₂ auf Kohle (0.5g) zugegeben und unter Wasserstoffatmosphäre (1bar) hydriert. Die Reaktions-mischung wurde abfiltriert und das Filtrat eingeengt. Der Rückstand wurde mittels Säulenchromatographie an Kieselgel (Dichlormethan/MeOH 9/1 1% Ammoniumhydroxid) gereinigt.
Ausbeute: 0.251g (0.56mmol) als *cis*/*trans* Gemisch
MS (EI) m/z 446 [M+H]⁺

### Beispiel 31: 2-{3-[(2,3-Dihydro-benzo[1,4]dioxin-6-yl-methyl)amino]pyrrolidin-1-yl}-1-(6-methoxychinolin-4-yl)-ethanol.

31.a) (3RS)-[(2,3-Dihydro-benzo[1,4]dioxin-6-ylmethyl)-amino]-pyrrolidin-1-benzylcarbamat 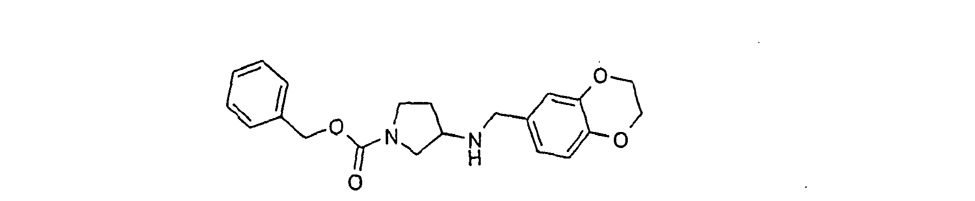 Zu einer Lösung von 3-Aminopyrrolidindihydrochlorid (3.2 g, 20.1 mmol) in Wasser (100mL) und Aceton (150 mL) wurde um 0C Natriumbicarbonat (7g) und Chlorameisensäure-benzylester (2.8 mL) zugegeben. Das Reaktionsgemisch wurde während 10 Stunden gerührt dann zur Trockne einrotiert. Der Rückstand wurde mit EtOAc (100mL) verdünnt. Die beiden Phasen wurden getrennt und die wässrige Phase zweimal mit je 50mL EtOAc extrahiert. Die vereinten organischen Phasen wurden mit 20mL gesättigter Kochsalzlösung gewaschen, über MgSO₄ getrocknet, filtriert und zur Trockne einrotiert. Der Rückstand wurde in Dichlormethan gelöst (30 ml) und 1,4-Benzodioxan-6-carbaldehyd (1.6 g) und nach 20 Minuten Natriumtriacetoxyborhydrid (4g) wurden zugegeben. Das Reaktionsgemisch wurde über Nacht gerührt dann mit Natriumbicarbonat (80 ml) verdünnt. Die beiden Phasen wurden getrennt und die wässrige Phase zweimal mit je 50mL Dichlormethan extrahiert. Die vereinten organischen Phasen wurden mit 20mL gesättigter Kochsalzlösung gewaschen, über MgSO₄ getrocknet, filtriert und zur Trockne einrotiert. Der Rückstand wurde mittels Säulenchromatographie an Kieselgel (EtOAc:MeOH 9/1) gereinigt.
Ausbeute: 3.75g (10.1mmol)
δ H (CDCl₃, 300MHz): 1.57 (br s, 1H), 1.77 (m, 1H), 2.07 (m, 1H); 3.21 (m, 1H); 3.37 (m, 2H); 3.61 (m, 2H), 3.70 (s, 2H); 4.25 (s, 4H); 5.14 (s, 2H); 6.76-6.84 (m, 3H); 7.30-7.38 (m, 5H).

31.b) (2,3-Dihydro-benzo[1,4]dioxin-6-ylmethyl)pyrrolidin-(3RS)-yl-amin: 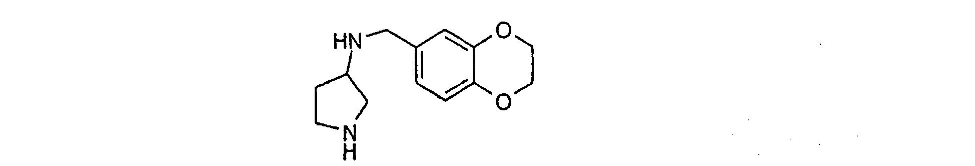 Zu einer Lösung 3-[(2,3-Dihydro-benzo[1,4]dioxin-6-yl-methyl)amino]pyrrolidin-1-benzylcarbamat (3.75g, 10.1 mmol) in EtOH (20mL) und EtOAc (20 mL) wurde 20% Pd(OH)₂ auf Kohle (1g) zugegeben und unter Wasserstoffatmosphäre (1bar) hydriert. Die Reaktions-mischung wurde abfiltriert und das Filtrat eingeengt.
Ausbeute: 2.15g (9.2mmol)
MS (EI) m/z 235 [M+H]⁺

31.c) 2-{(3RS)-[(2,3-Dihydro-benzo[1,4]dioxin-6-ylmethyl)-amino]-pyrrolidin-1-yl}-(1RS)-(6-methoxy-chinolin-4-yl)-ethanol:

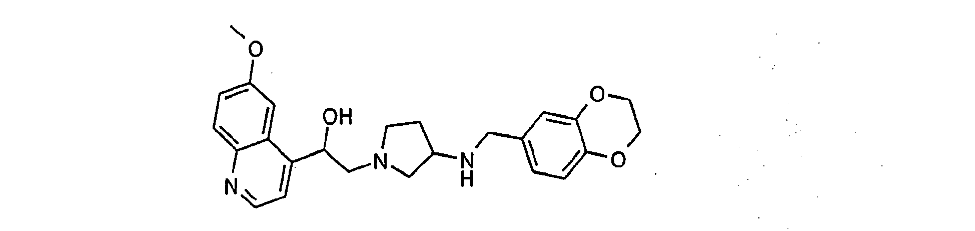

Eine Lösung von 6-Methoxy-4-oxiranylchinolin (0.201g, 1mmol) und (2,3-Dihydrobenzo[1,4]dioxin-6-ylmethyl)-pyrrolidin-3-ylamin (0.234g, 1mmol) in Ethanol (2mL) wurde während 16 Stunden auf 80°C erwärmt. Das Reaktionsgemisch wurde auf Raumtemperatur abgekühlt und zur Trockne einrotiert. Der Rückstand wurde mittels Säulenchromatographie an Kieselgel (EtOAc dann EtOAc-MeOH 5:1) gereinigt.
Ausbeute: 0.208g (0.477mmol)
MS (EI) m/z 436.5 [M+H]⁺

### Beispiel 32: 4-(4-Methoxy-benzylamino)-1-[2-(6-methoxy-chinazolin-4-yl)-ethyl]cyclohexanol.

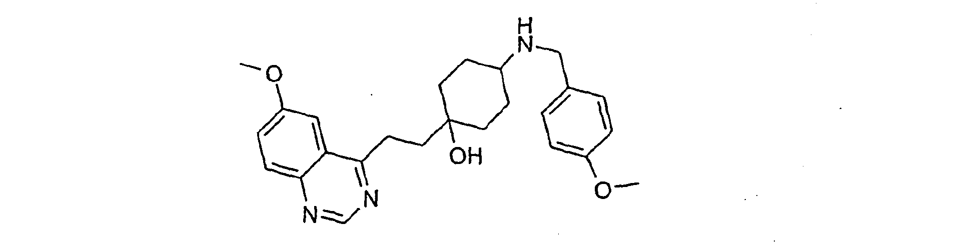

Zu einer Lösung von 4-Hydroxy-4-[2-(6-methoxychinazolin-4-yl)ethyl]cyclohexanon (0.06g, 0.2mmol) in Dichlormethan (1mL) wurde 4-Methoxybenzylamin (0.026mL, 0.24mmol) und dannach Natriumtriacetoxyborhydrid (0.08g, 0.377 mmol) zugegeben. Das Reaktionsgemisch wurde über Nacht gerührt dann über Hydromatrix (benetzt mit einer NaHCO₃ Lösung, 2mL) filtriert und mit Dichlormethan nachgewaschen. Das Filtrat wurde zur Trockne einrotiert. Der Rückstand wurde mittels Säulenchromatographie an Kieselgel (Dichlormethan/MeOH 9/1 dann Dichlormethan/MeOH 9/1 und 2% Triethylamin) gereinigt.
Ausbeute:0.052g (0.124mmol) als *cis*/*trans* Gemisch
MS (EI) m/z 422 [M+H)⁺

### Beispiel 33: 4-(3-Fluorbenzylamino)-1-[2-(6-methoxy-chinazolin-4-yl)-ethyl]cyclohexanol.

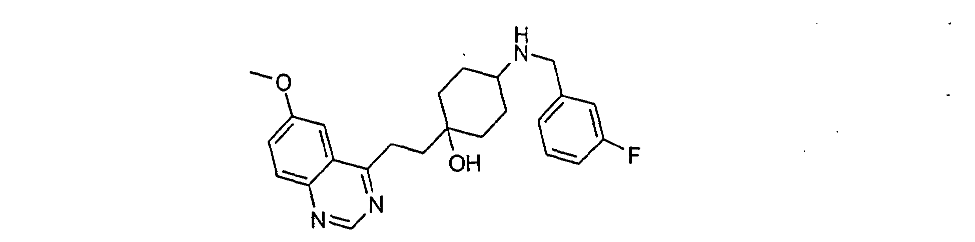

Die Verbindung wurde analog zu Beispiel 10 ausgehend von 3-Fluorbenzylamin (0.026mL, 0.24mmol) hergestellt.
Ausbeute:(0.052g, 0.124mmol) als *cis*/*trans* Gemisch MS (EI) m/z 410 [M+H]⁺

### Beispiel 34: 2-{4-[(2,3-Dihydrobenzo[1,4]dioxin-6-yl-ethyl)-amino]-azepan-1-yl}-1-(6-methoxy-chinolin-4-yl)-ethanol.

34.a) 4-Oxoazepan-1-*tert*-butylcarbamat: 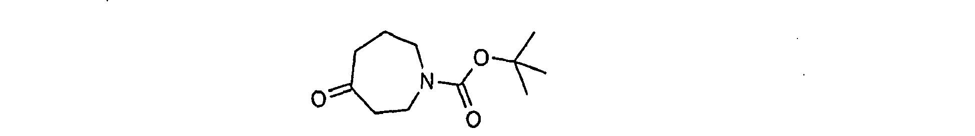 Ein Gemisch aus 5-Oxoazepan-1,4-dicarbonsäure-1-*tert-*butylester-4-ethylester (5.7 g, 20 mmol; hergestellt wie in Synthetic Communications 1992, 22, 1249 beschrieben) wurde in einem Gemisch von 3N NaOH (50 ml) und THF (25 ml) während 3h unter Rückfluss erhitzt. Das Reaktionsgemisch wurde abgekühlt und mit verdünnter HCl neutralisiert. Das Gemisch wurde mit Essigester extrahiert und die organischen Phasen über MgSO₄ getrocknet und eingeengt.
Ausbeute: 4.2 g (100%)
¹H-NMR (CDCl₃, 300MHz): 1.46 (s, 9H); 1.80 (br, 2H); 2.60-2.7 (m, 4H); 3.4-3.6 (m, 4H).

34.b) (4RS)-Aminoazepan-1-*tert*-butylcarbamat: 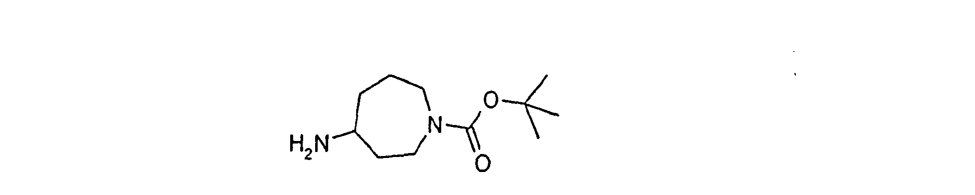 Eine Lösung von 4-Oxoazepan-1-*tert*-butylcarbamat (1g, 4.68 mmol) in Methanol (50 ml) wurde mit Ammoniumacetat (3.5 g) und Natriumcyanoborhydrid (295 mg, 1eq) versetzt. Das Gemisch wurde über Nacht bei Raumtemperatur gerührt. Das Reaktionsgemisch wurde eingeengt und der Rückstand in gesättigter Kaliumcarbonat Lösung und Essigester gelöst. Die wässrige Phase wurde mit Essigester (2x50ml) extrahiert, die vereinten organischen Phasen über Magnesiumsulfat getrocknet und eingeengt. Man erhielt 1g (100%) Produkt, das ohne Reinigung weiterverwendet wurde.
¹H-NMR (CDCl₃, 300MHz): 1.46 (s, 9H); 1.5-1.75 (m, 2H); 1.8-2.05 (m, 4H); 2.4 (br, 2H); 2.95-3.05 (m, 1H); 3.1-3.6 (m, 4H).
MS (EI) m/z 215.6 [M+H]⁺

34.c) (4RS)-[(2,3-Dihydrobenzo[1,4]dioxin-6-ylmethyl)-amino]-azepan-1-*tert*-butylcarbamat: 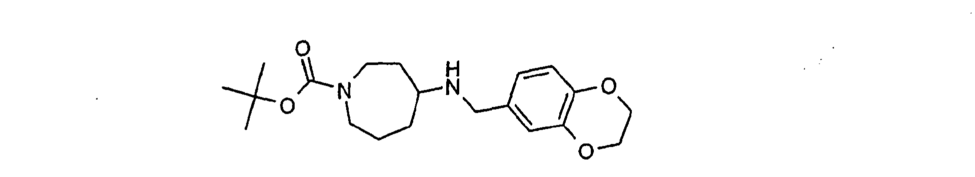 Eine Lösung von 4-Aminoazepan-1-*tert*-butylcarbamat (428 mg, 2 mmol) und 2,3-Dihydrobenzo[1,4]dioxin-6-carbaldehyd (330µl, 2mmol) in Dichlorethan (10 ml) wurde mit Essigsäure (500µl) und Natriumcyanoborhydrid (126 mg) versetzt. Das Gemsich wurde über Nacht bei Raumtemperatur gerührt, mit gesättigter Kaliumcarbonatlösung verdünnt und mit Dichlormethan extrahiert. Die organischen Phasen wurden über Magnesiumsulfat getrocknet und eingeengt. Das Produkt wurde mittels Chromatographie an Kieselgel (Essigester) gereinigt.
Ausbeute: 370 mg (51%)
¹H-NMR (CDCl₃, 300MHz): 1.46 (s, 9H); 1.5-1.75 (m, 2H); 1.8-2.05 (m, 4H); 2.65 (m, 1H); 3.1-3.6 (m, 4H);
3.70 (s, 2H); 4.26 (s, 4H); 6.8-6.9 (m, 3H).
MS (EI) m/z 363.6 [M+H]⁺

34.d) Azepan-(4RS)-yl-(2,3-dihydrobenzo[1,4]dioxin-6-ylmethyl)-amin: 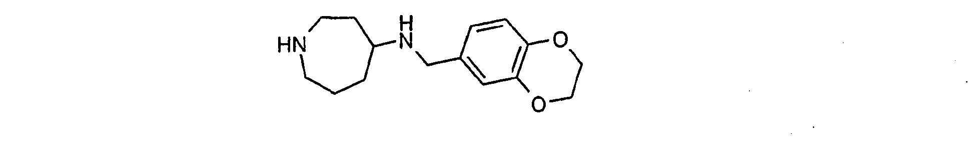 4-[(2,3-Dihydrobenzo[1,4]dioxin-6-ylmethyl)amino]-azepan-1-*tert*-butylcarbamat (370 mg, 1 mmol) wurde in einem Gemisch aus 10 ml Wasser und 2 ml konz. HCl gelöst und 24h bei Raumtemperatur gerührt. Das Reaktionsgemisch wurde mit festem Kaliumcarbonat neutralisiert und mit Essigester extrahiert. Chromatographie an Kieselgel (Dichlormethan/MeOH 9:1) ergab 220 mg reines Produkt (85%).
¹H-NMR (CDCl₃, 300MHz): 1.8-2.0 (m, 1H); 2.0-2.3 (m, 4H); 2.3-2.6 (m, 2H); 3.2-3.4 (m, 4H); 3.6-6.7 (m, 1H); 3.95 (dd, 2H); 4.2-4.3 (m, 5H); 6.8-7.1 (m, 3H). MS (EI) m/z 263.4 [M+H]⁺

34.e) 2-{(4RS)-[(2,3-Dihydrobenzo[1,4]dioxin-6-ylmethyl)-amino]-azepan-1-yl}-(1RS)-(6-methoxychinolin-4-yl)ethanol: 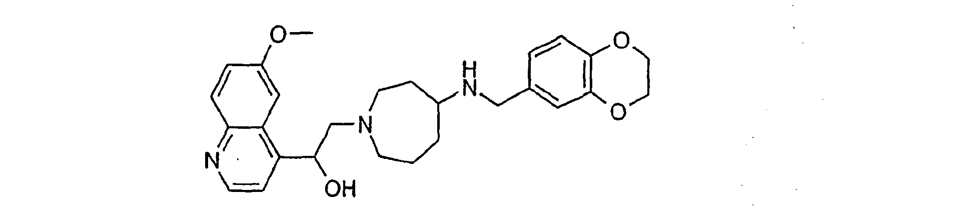 Ein Gemisch aus Azepan-4-yl-(2,3-dihydro-benzo[1,4]-dioxin-6-ylmethyl)amin (60 mg), 6-Methoxy-4-oxiranyl-chinolin (50 mg), Lithiumperchlorat (25mg) und Kaliumcarbonat (35 mg) wurde in DMF (1 ml) über Nacht auf 80°C erhitzt. Das Reaktionsgemisch wurde mittels Chromatographie an Kieselgel (Dichlormethan/MeOH 9:1 (+2% NEt₃) gereinigt.
¹H-NMR (CDCl₃, 300MHz): 1.5-2.0 (m, 7H); 2.45 (dd, 1H); 2.55-3.05 (m, 8H); 3.5-3.8 (m, 2H); 3.85 (s, 3H); 4.15 (s, 4H); 5.25-5.35 (m, 1H); 6.6-6.8 (m, 2H); 7.11 (dd, 1H); 7.30 (dd, 1H); 7.55 (dd, 1H);
7.97 (d, 1H); 8.7 (d, 1H).
MS (EI) m/z 464.6 [M+H]⁺

### Beispiel 35: [1-(2,3-Dihydro-benzo[1,4]dioxin-6-yl-methyl)azepan-4-yl](6-methoxychinolin-4-ylmethyl)amin:

35.a) (4RS)-[(6-Methoxychinolin-4-ylmethyl)-amino]-azepan-1-*tert*-butylcarbamat: 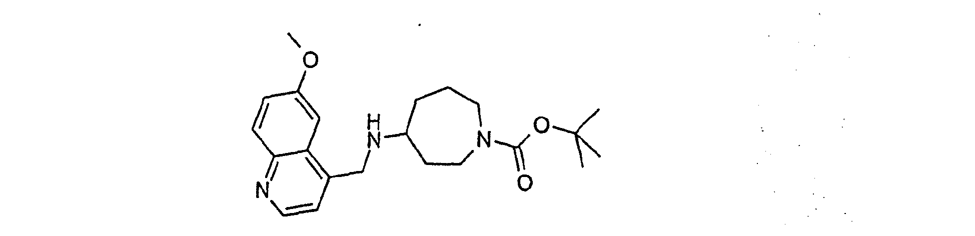 Eine Lösung von 4-Aminoazepan-1-*tert*-butylcarbamat (568 mg, 2.65 mmol) und 6-Methoxychinolin-4-carbaldehyd (497 mg (2.7 mmol) in Dichlorethan (10 ml) und Essigsäure (1 ml) wurde mit Natriumcyanoborhydrid (170 mg, 2.7 mmol) versetzt. Das Reaktionsgemisch wurde über Nacht bei Raumtemperatur gerührt, auf gesättigte Natriumcarbonatlösung gegossen und mit Dichlormethan extrahiert. Die organische Phase wurde über Magnesiumsulfat getrocknet und eingeengt. Das Produkt wurde mittels Chromatographie an Kieselgel (Essigester) gereinigt.
Ausbeute: 515 mg (51%)
¹H-NMR (CDCl₃, 300MHz) : 1.47 (s, 9H); 1.5-2.0 (m, 8H); 2.8-2.9 (m, 1H); 3.2-3.65 (m, 5H); 3.97 (s, 3H);
4.22 (s, 2H); 5.25-5.35 (m, 1H); 6.6-6.8 (m, 2H);
7.11 (dd, 1H); 7.35-7.45 (m, 3H); 8.02 (d, J=9.2Hz, 1H); 8.73 (d, J=1.4Hz, 1H).
MS (EI) m/z 386.5 [M+H]⁺

35.b) Azepan-(4RS)-yl-(6-methoxychinolin-4-ylmethyl)amin: 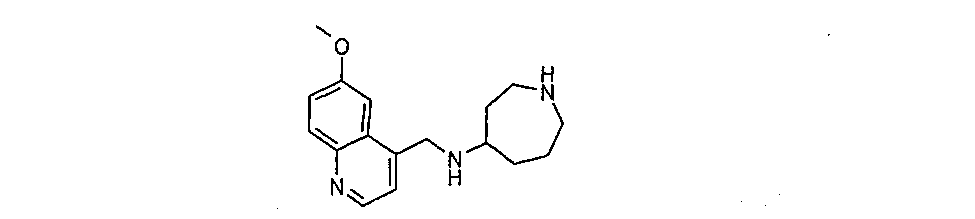 Eine Lösung von 4-[(6-Methoxychinolin-4-ylmethyl)amino]-azepan-1-*tert*-butylcarbamat (700 mg, 1.8 mmol) in Dichlormethan (1 ml) wurde bei Raumtemperatur mit TFA (1 ml) versetzt. Das Reaktionsgemisch wurde während 3h bei Raumtemperatur gerührt, eingeengt und in wässriger NaOH aufgenommen. Die wässrige Phase wurde mit Essigester extrahiert und die vereinten organischen Phasen über Natriumsulfat getrocknet. Das Produkt wurde mittels Chromatographie an Kieselgel (DCM/MeOH 9:1 (1% NH₄OH)) gereinigt.
Ausbeute: 477 mg (92%)
¹H-NMR (CDCl₃, 300MHz): 1.7-2.3 (m, 6H); 3.0-3.3 (m, 4H); 3.35-3.45 (m, 1H); 3.97 (s, 3H); 4.2 (s, 2H);
7.28 (d, 1H); 7.35-7.45 (m, 2H); 8.02 (d, *J*=9.2Hz 1H); 8.73 (d, *J*=1.4Hz, 1H).
MS (EI) m/z 286.3 [M+H]⁺

35.c) [1-(2,3-Dihydrobenzo[1,4]dioxin-6-ylmethyl)-azepan-(4RS)-yl]-(6-methoxychinolin-4-ylmethyl)amin: 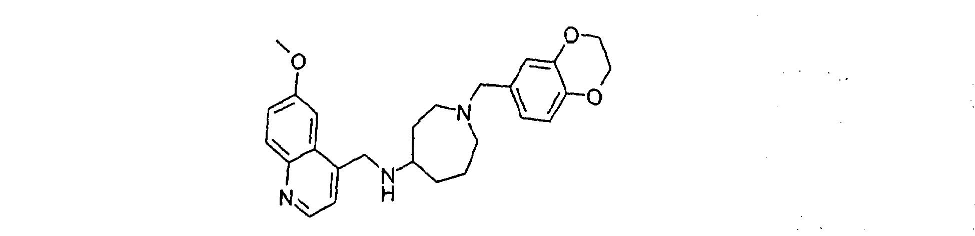 Eine Lösung von Azepan-4-yl-(6-methoxychinolin-4-yl-methyl)amin (90 mg, 0.32 mmol) und 2,3-Dihydro-benzo[1,4]-dioxin-6-carbaldehyd (51.8 mg, 0.32 mmol) in Dichlorethan/THF (1:1, 0.7 ml) wurde mit Natriumtriacetoxyborhydrid (100 mg, 0.47 mmol) versetzt. Das Reaktionsgemisch wurde 3 Stunden bei Raumtemperatur gerührt, eingeengt und über Kieselgel (Essigester, Methanol) chromatographiert.
Ausbeute: 56 mg (41%)
¹H-NMR (CDCl₃, 300MHz): 1.40-2.05 (m, 6H); 2.35-2.80 (m, 3H); 2.8-2.9 (m, 1H); 3.41 (2, 2H); 3.99 (s, 3H);
4.15 (s, 2H); 4.20 (s, 4H); 6.70-6.80 (m, 3H); 7.37 (dd, *J*=2.76, *J*=9.1, 1H); 7.45 (d, *J*=2.76, 1H); 7.51 (d, *J*=4.4, 1H); 7.92 (d, *J*=9.12, 1H); 8.65 (d, *J*=4.4, 1H).
MS (EI) m/z 434.7 [M+H] ⁺

### Beispiel 36: (6-Methoxychinolin-4-ylmethyl)-(1-phenethyl-azepan-(4RS)-yl)-amin:

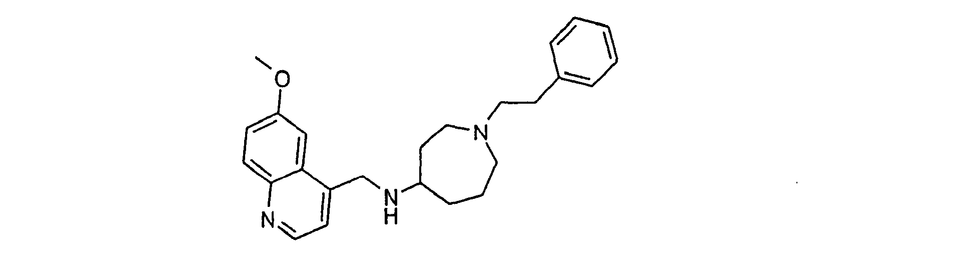

In analoger Weise zu Beispiel 35.c wurden auch: (6-Methoxychinolin-4-ylmethyl)-(1-phenethylazepan-(4RS)-yl)-amin (25% Ausbeute, MS (EI) m/z 390.5 [M+H]⁺),

### Beispiel 37::(6-Methoxychinolin-4-ylmethyl)-[1-(3-phenylpropyl)-azepan-4-yl]amin

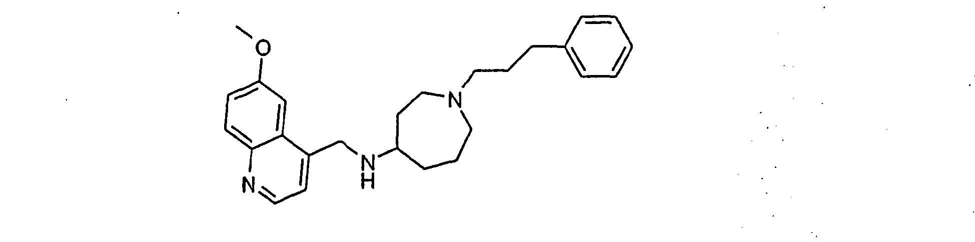

In analoger Weise zu Beispiel 35.c wurden auch: (6-Methoxychinolin-4-ylmethyl)-[1-(3-phenylpropyl)-azepan-4-yl]amin (25% Ausbeute, MS (EI) m/z 404.9 [M+H]⁺)

### Beispiel 38: (1-Heptylazepan-(4RS)-yl)-(6-methoxychinolin-4-ylmethyl)amin:

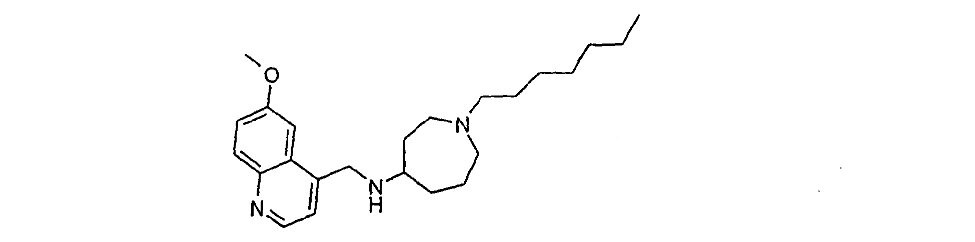

In analoger Weise zu Beispiel 35.c wurden auch: (1-Heptylazepan-(4RS)-yl)-(6-methoxychinolin-4-ylmethyl)amin (43% Ausbeute, MS (EI) m/z 384.4 [M+H]⁺) hergestellt.

### Beispiel 39: 1-{(4RS)-[(6-Methoxychinolin-4-ylmethyl)-amino]-azepan-1-yl}-3-phenylpropenon.

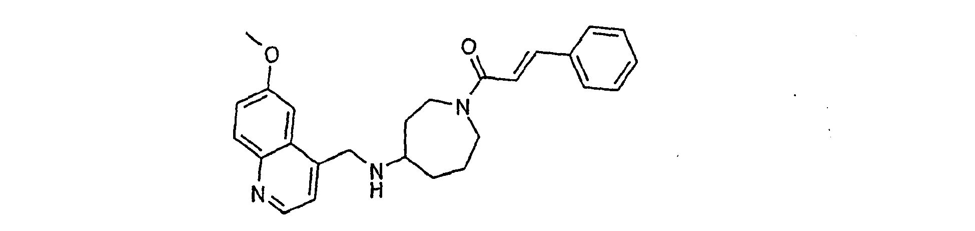

Eine Lösung von Azepan-4-yl-(6-methoxychinolin-4-yl-methyl)amin (90 mg, 0.32 mmol) in THF/DCE wurde mit Zimtsäurechlorid (52.5 mg, 1eq) versetzt. Das Reaktionsgemisch wurde 3h bei Raumtemperatur gerührt, eingeengt und mittels Chromatographie an Kieselgel (DCM/MeOH 9:1 (+1% NH₄OH)) gereinigt.
Ausbeute: 37 mg (38%)
MS (EI) m/z 416.6 [M+H]⁺

### Beispiel 40: 1-[(2RS)-Hydroxy-2-(6-methoxychinolin-4-yl)-ethyl]-4-phenethyl-[1,4]diazepan-(5RS)-carbonsäure-tert-butylester

40.a) 1-[2-Hydroxy-2-(6-methoxychinolin-4-yl)ethyl]-[1,4]-diazepan-5-carbonsäure-*tert*-butylester 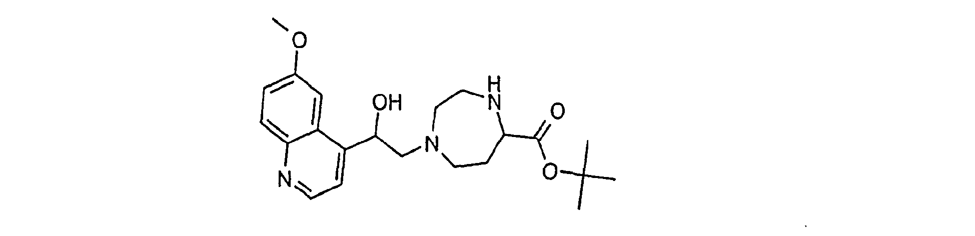 Ein Gemisch aus 6-Methoxy-4-oxiranylchinolin (400 mg, 2 mmol), [1,4]-Diazepan-5-carbonsäure-*tert*-butylester (400 mg, 2 mmol, hergestellt wie in J. Chem. Research (S), 1991, 306, 2876 beschrieben), Lithiumperchlorat (211 mg, 2 mmol) und Kaliumcarbonat (275 mg, 2 mmol) in DMF (5 ml) wurde während 4 Stunden auf 100°C erhitzt. Das Reaktionsgemisch wurde mit Wasser und Essigester verdünnt, die wässige Phase mit Essigester extrahiert und die vereinten organischen Phasen über Natriumsulfat getrocknet und eingeengt. Das Produkt wurde mittels Chromatographie an Kieselgel (DCM/MeOH 9:1) gereinigt.
Ausbeute: 330 mg (41%)
MS (EI) m/z 402.5 [M+H]⁺

40.b) 1-[(2RS)-Hydroxy-2-(6-methoxychinolin-4-yl)ethyl]-4-phen-ethyl-[1,4]diazepan-(5RS)-carbonsäure-*tert-*butylester: 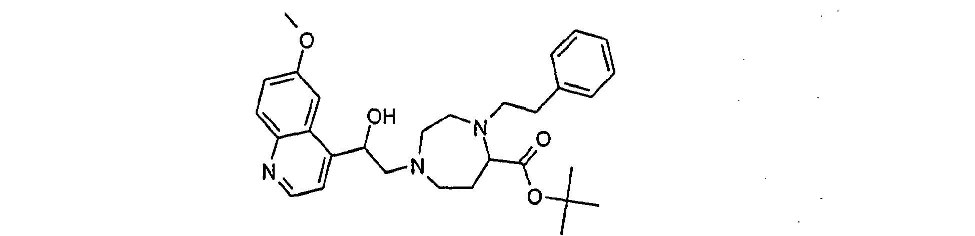 Zu einer Lösung von 1-[2-Hydroxy-2-(6-methoxy-chinolin-4-yl)-ethyl]-[1,4]diazepan-5-carbonsäure-*tert*-butylester (100 mg, 0.25 mmol) und Phenylacetaldehyd (29.2 ul, 1eq) in THF (700 ul) wurde Natriumtriacetoxyborhydrid (79 mg (1.5 eq) zugegeben. Nach 2h wurde ein weiteres Equivalent Phenylacetaldehyd zugegeben und das Reaktionsgemisch auf 40°C erwärmt. Nach weiteren 2h wurde das Reaktionsgemisch zur Trockne eingeengt uns mittels Chromatographie an Kieselgel gereinigt (Essigester, Methanol)
Ausbeute: 67 mg (54%)
MS (EI) m/z 506 [M+H]⁺

40.c) 1-[(2RS)-Hydroxy-2-(6-methoxychinolin-4-yl)-ethyl]-4-phen-ethyl-[1,4]-diazepan-(5RS)-carbonsäure: 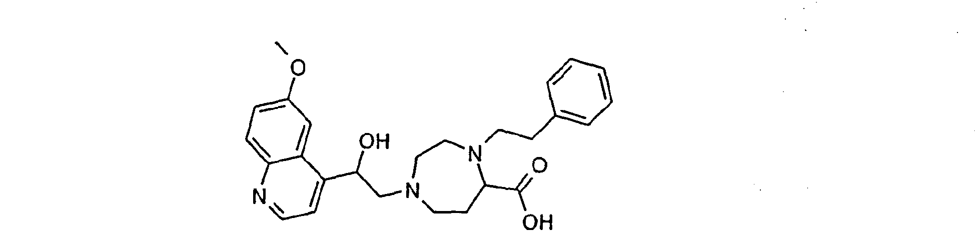 1-[2-H_{y}droxy-2-(6-methox-chinolin-4-yl)-ethyl]-4-phenethyl-[1,4]diazepan-5-carbonsäure-*tert*-butylester (55 mg) wurden in 4M HCl in Dioxan suspendiert und 3.5h bei Raumtemperatur gerührt. Das Reaktionsgemisch wurde eingeengt und mittels präparativer HPLC gereinigt.
Ausbeute: 3.9 mg
MS (EI) m/z 450 [M+H]⁺

In analoger Weise wurden auch:
40.d) 1-[2-Hydroxy-2-(6-methoxy-chinolin-4-yl)-ethyl]-4-(3-phenylpropyl)-[1,4]diazepan-5-carbonsäure-*tert-*butylester und
40.e) 1-[2-Hydroxy-2-(6-methoxychinolin-4-yl)ethyl]-4-(3-phenylpropyl)-[1,4]diazepan-5-carbonsäure ausgehend von Dihydrozimtaldehyd synthetisiert. Ebenso wurden ausgehend von Heptanal
40.f) 4-Heptyl-1-[2-hydroxy-2-(6-methoxychinolin-4-yl)-ethyl]-[1,4]diazepan-5-carbonsäure-*tert*-butylester und 40.g) 4-Heptyl-1-[2-hydroxy-2-(6-methoxychinolin-4-yl)-ethyl]-[1,4]diazepan-5-carbonsäure hergestellt.

### Beispiel 41: 2-[(RS)5-Hydroxymethyl-4-(3-phenylpropyl)-[1,4]diazepan-1-yl]-(1RS)-(6-methoxychinolin-4-yl)-ethanol.

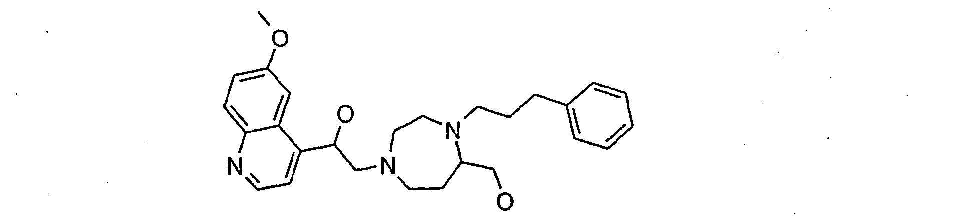

Eine Lösung von 1-[2-Hydroxy-2-(6-methoxychinolin-4-yl)-ethyl]-4-(3-phenylpropyl)-[1,4]-diazepan-5-carbonsäure-*tert*-butylester (80mg) in THF wurde mit Lithiumaluminiumhydrid (28 mg) versetzt und 1 Stunde bei Raumtemperatur gerührt. Das Reaktionsgemisch wurde mit einigen Tropfen gesättigter Rochellesalzlösung versetzt, 15 Minuten gerührt und der Niederschlag abfiltriert.
Ausbeute: 39 mg (57%)
MS (EI) m/z 450 [M+H]⁺

In analoger Weise wurde ausgehend von 4-Heptyl-1-[2-hydroxy-2-(6-methoxychinolin-4-yl)-ethyl]-[1,4]diazepan-5-carbonsäure-*tert*-butylester, 2-(4-Heptyl-5-hydroxy-methyl-[1,4]-diazepan-1-yl)-1-(6-methoxychinolin-4-yl)-ethanol in 33% Ausbeute hergestellt (MS (EI) m/z 430 [M+H]⁺).

### Beispiel 42: (2,3-Dihydro-benzo[1,4]dioxin-6-ylmethyl)-[4-(6-methoxy-chinazolin-4-yloxymethyl)-cyclohexyl]-amin

42.a) 4-(6-Methoxy-chinazolin-4-yloxymethyl)-cyclohexanol 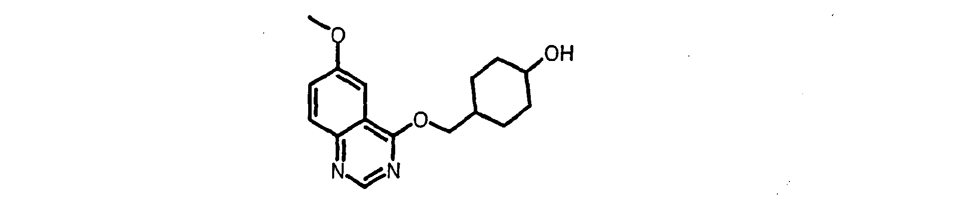 Zu einer Lösung von 4-Hydroxymethyl-cyclohexanol (3g, 12 mmol, synthetisiert analog J.Org.Chem. 1994 59 p. 2748-2761) in DMF (30mL) wurde bei 0C Natriumhydrid (79 mg (1.5 eq) gegeben. Nach 20 minuten, wurde eine Lösung von 4-Chloro-6-methoxy-chinazolin (1.94 g, 10mmol) in DMF (10mL) zugegeben und das Reaktionsgemisch auf 40°C erwärmt. Nach weiteren 2h wurde das Reaktionsgemisch zur Trockne eingeengt. Der Rückstand wurde mit Wasser (100mL) und Essigester (100 mL) verdünnt, die Phasen getrennt und die wässrige Phase zweimal mit je 50mL Essigester extrahiert. Die vereinten organischen Phasen wurden über MgSO₄ getrocknet, filtriert und zur Trockne einrotiert. Der Rückstand wurde mittels Säulenchromatographie an Kieselgel (EtOAc-Hexan 1-2) gereinigt.
Ausbeute: 2.01g, 7 mmol (54%)
MS (EI) m/z 289 [M+H]⁺

42.b) 4-(6-Methoxy-chinazolin-4-yloxymethyl)-cyclohexanon 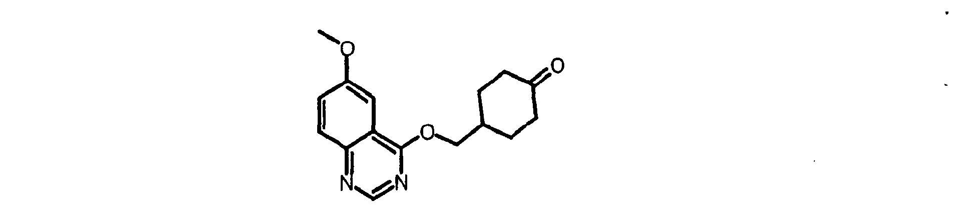 Zu einer Lösung von 4-(6-Methoxy-chinazolin-4-yloxy-methyl)-cyclohexanol (2.88 g, 10mmol) in DMSO (30mL) wurden bei 0C Triethylamin (10mL, 71 mmol) und dann portionenweise Pyr.SO3 (5.8 g, 36mmol) zugegeben. Das Reaktionsgemisch wurde 15 Minuten bei dieser Temperatur gerührt und dann auf Raumtemperatur kommen gelassen. Nach 2h wurde Wasser (300mL) zugegeben. Die wässrige Phase wurde mit Ether (3 x 150mL) extrahiert. Die vereinten organischen Phasen wurden mit ges. Kochsalzlösung gewaschen, über MgSO₄ getrocknet und eingeengt. Das Rohprodukt wurde mittels Chromatographie an Kieselgel (EtOAc-Hex 1-2) gereinigt.
Ausbeute: 2.6g, 9.1 mmol
MS (EI) m/z 287.1 [M+H]⁺

42.c) Benzyl-[4-(6-methoxy-chinazolin-4-yloxymethyl)-cyclohexyl]-amin 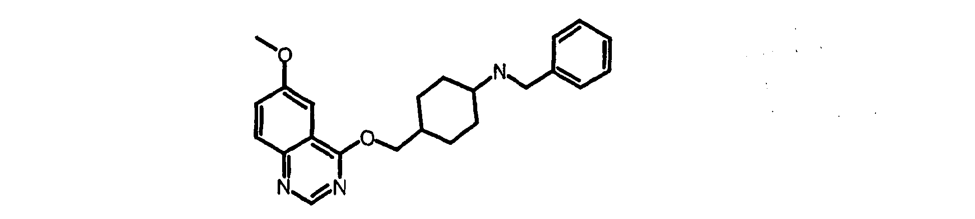 Eine Lösung von 4-(6-Methoxy-chinazolin-4-yloxymethyl)-cyclohexanon (2.87g, 10 mmol) in Methanol (50 ml) wurde mit Benzylamin (1.08mL, 10 mmol) und Natriumcyanoborhydrid (2.2 g, 11 mmol) versetzt. Das Gemisch wurde über Nacht bei Raumtemperatur gerührt. Das Reaktionsgemisch wurde eingeengt und der Rückstand in gesättigter Kaliumcarbonat Lösung und Essigester gelöst. Die wässrige Phase wurde mit Essigester (2x50ml) extrahiert, die vereinten organischen Phasen über Magnesiumsulfat getrocknet und eingeengt. Der Rückstand wurde mittels Säulenchromatographie an Kieselgel (EtOAc-dann EtOAc-MeOH 9/1) gereinigt.
Ausbeute: 2.87g, 10 mmol als *cis*/*trans* Gemisch
MS (EI) m/z 287.1 [M+H]⁺

42.d) 4-(6-Methoxy-chinazolin-4-yloxymethyl)-cyclohexylamin 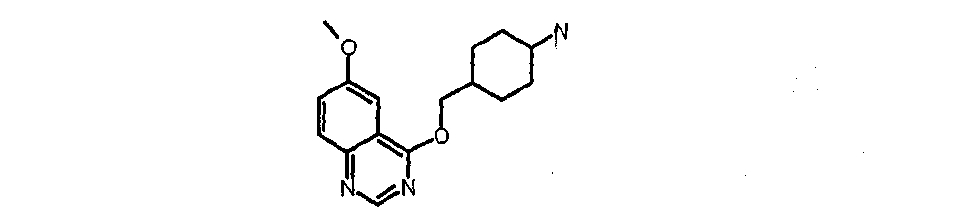 Zu einer Lösung von Benzyl-[4-(6-methoxy-chinazolin-4-yloxymethyl)-cyclohexyl]-amin (2g, 5.3 mmol) in MeOH (30mL) wurde 20% Pd(OH)₂ auf Kohle (1g) zugegeben und unter Wasserstoffatmosphäre (1bar) hydriert bei 65C. Die Reaktionsmischung wurde abfiltriert und das Filtrat eingeengt.
Ausbeute: 1.44g , 5 mmol als *cis*/*trans* Gemisch
MS (EI) m/z 288 [M+H]⁺

42.e) Titelverbindung 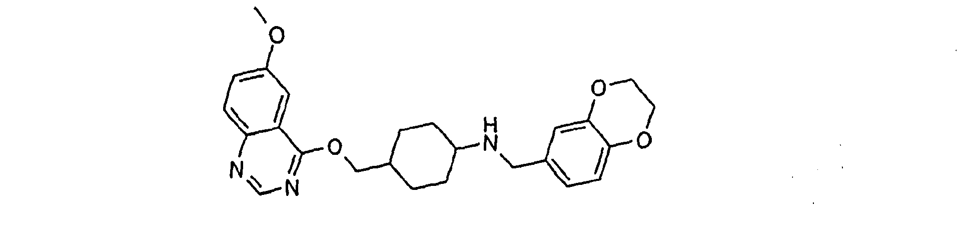 Zu einer Lösung von 4-(6-Methoxy-chinazolin-4-yloxy-methyl)-cyclohexylamin (0.07g, 0.25mmol) in Dichlorethan (1mL) wurde 2,3-Dihydro-benzo[1,4]dioxin-6-carbaldehyd (0.045g, 0.27mmol) und danach Natriumtriacetoxyborhydrid (0.08g, 0.377 mmol) zugegeben. Das Reaktionsgemisch wurde 2 Stunden gerührt dann über Hydromatrix (benetzt mit NaHCO₃ Lösung, 2mL) filtriert und mit Dichlormethan nachgewaschen. Das Filtrat wurde zur Trockne einrotiert. Der Rückstand wurde mittels Säulenchromatographie an Kieselgel (Dichlormethan/MeOH 9/1 dann Dichlormethan/MeOH 6/1) gereinigt.
Ausbeute:0.02g (0.046mmol) als *cis*/*trans*-Gemisch
MS (EI) m/z 436 [M+H]⁺

### Beispiel 43: [4-(6-Methoxy-chinazolin-4-yloxymethyl)-cyclohexyl]-chinoxalin-2-ylmethyl-amin

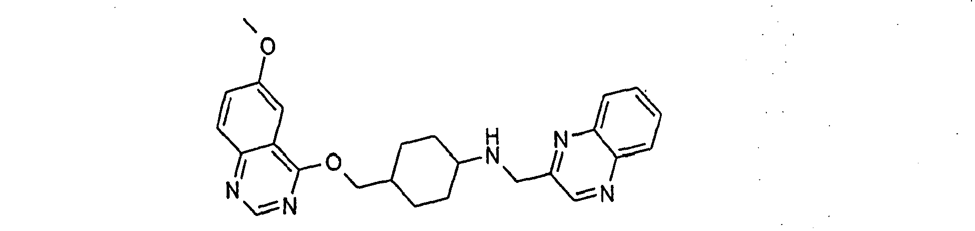

In Analogie zu Beispiel 42.e wurde [4-(6-Methoxy-chinazolin-4-yloxymethyl)-cyclohexyl]-chinoxalin-2-yl-methylamin in 31% Ausbeute hergestellt als *cis*/*trans-*Gemisch (MS (EI) m/z 430 [M+H]⁺).

### Beispiel 44: 6-{[4-(6-Methoxy-chinazolin-4-yloxymethyl)-cyclohexylamino]-methyl}-4H-benzo[1,4]oxazin-3-on

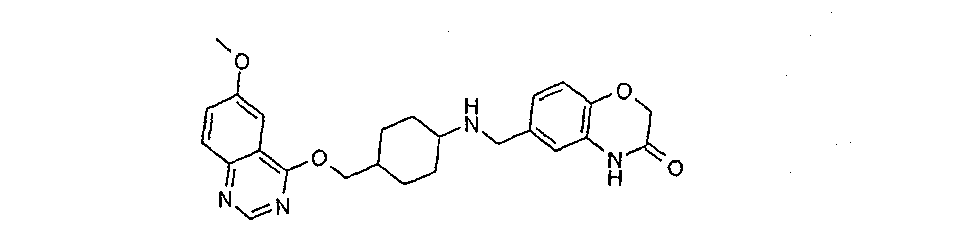

In Analogie zu Beispiel 42.e wurde 6-{[4-(6-Methoxy-chinazolin-4-yloxymethyl)-cyclohexylamino]-methyl}-4H-benzo[1,4]oxazin-3-on in 38% Ausbeute hergestellt als *cis*/*trans*-Gemisch (MS (EI) m/z 448 [M+H]⁺).

### Beispiel 45: [4-(6-Methoxy-chinazolin-4-yloxymethyl)-cyclohexyl]-(3-phenyl-allyl)-amin

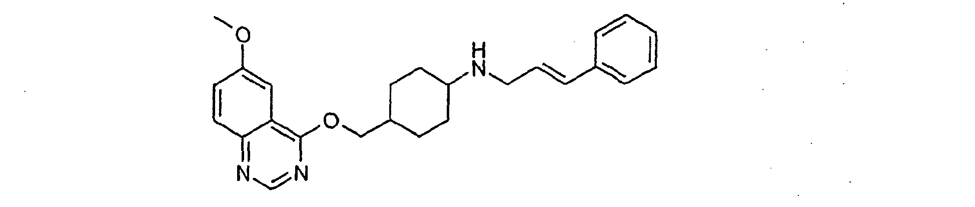

In Analogie zu Beispiel 42.e wurde [4-(6-Methoxy-chinazolin-4-yloxymethyl)-cyclohexyl]-(3-phenyl-allyl)-amin in 25% Ausbeute als *cis*/*trans* Gemisch hergestellt (MS (EI) m/z 405 [M+H]⁺)

### Beispiel 46: 2-[4-(6-Methoxy-chinazolin-4-yloxymethyl)-cyclohexylamino]-N-pyridin-2-yl-acetamid

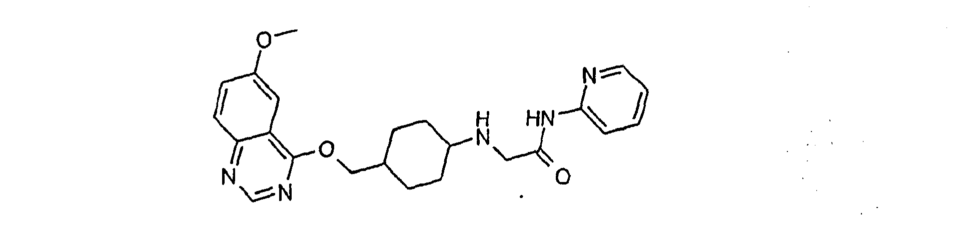

Zu einer Lösung von 4-(6-Methoxy-chinazolin-4-yloxy-methyl)-cyclohexylamin (0.1g, 0.35 mmol) in DMF (3.5mL) wurde 2-Bromo-N-pyridin-2-yl-acetamid (0.075g, 0.35mmol) und Kaliumcarbonat (0.054g, 0.39mmol) gegeben. Das Reaktionsgemisch wurde 2 Tage bei Raumtemperatur gerührt dann zur Trockne einrotiert. Der Rückstand wurde mittels Säulenchromatographie an Kieselgel (Dichlormethan/MeOH 19/1 1% NH₄OH) gereinigt.
Ausbeute:0.086g (0.2mmol) als *cis*/*trans*-Gemisch
MS (EI) m/z 422 [M+H]⁺

### Beispiel 47: (2,3-Dihydro-[1,4]dioxino[2,3-c]pyridin-7-ylmethyl)-[4-(6-methoxy-chinazolin-4-yloxymethyl)-cyclohexyl]-amin

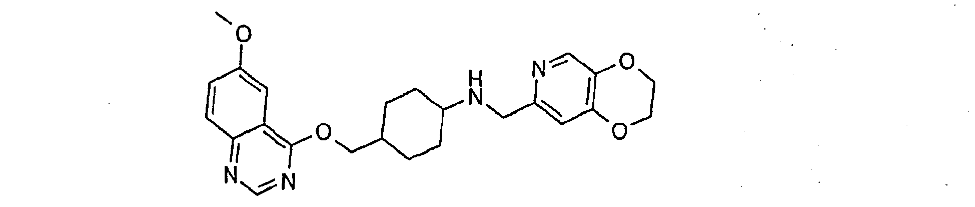

In analoger Weise wurde ausgehend von Beispiel 42.e in 55% Ausbeute als *cis*/*trans* Gemisch hergestellt (MS (EI) m/z 437 [M+H]⁺).

### Beispiel 48: (2,3-Dihydro-benzo[1,4]dioxin-6-ylmethyl)-[4-(6-methoxy-chinolin-4-yloxymethyl)-cyclohexyl]-amin

48.a) *trans*-4-*tert*-Butoxycarbonylamino-cyclohexancarbonsäure 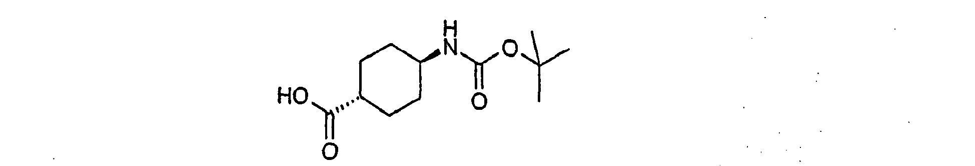 Zu einer Lösung von *trans*-4-aminocyclohexancarbonsäure (Synth. Commun. 2002, 32, 1985) (7.16 g, 50 mmol) in Dioxan (50 ml) und Wasser (50ml) wurde di-tert.-butyl-dicarbonat (12 g, 55 mmol) in Dioxan (50ml) gelöst zugetropft. Nach 30 min Rühren bei Raumtemperatur wurde 1M NaOH (50 ml) zugegeben und das Reaktionsgemisch über Nacht bei Raumtemperatur gerührt. Das Dioxan wurde am Rotationsverdampfer abdestilliert und der wässrige Rückstand mit 1N HCl auf pH 3 gestellt und mit Essigester extrahiert. Organisch Extrakte über Na₂SO₄ getrocknet und eingeengt.
Ausbeute: 13.2 g
MS (EI) m/z 242.4[M-H]⁻).

48.b) *trans*-(4-Hydroxymethyl-cyclohexyl)-*tert*-butylcarbamat 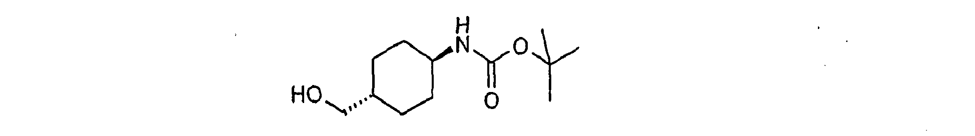 Zu einer Lösung von *trans*-4-*tert*-Butoxycarbonylamino-cyclohexanecarbonsäure (2.43 g, 10 mmol) in THF (100 ml) bei 0°C wurde eine Lösung von Boran Dimethylsulfidkomplex (2.85 ml, 30 mmol) in THF (50 ml) zugetropft. Das Reaktionsgemisch wurde 15 Minuten bei 0C und 3h bei Raumtemperatur gerührt, vorsichtig mit Methanol versetzt und eingeengt. Das Rohprodukt wurde mehrmals mit Methanol versetzt und wieder eingeengt und zum Schluss am Hochvakuum getrocknet. Ohne weitere Reinigung verwendet.
Ausbeute: 2.4 g (quant)

48.c) *trans*-Methanesulfonsäure 4-*tert*-butoxycarbonylaminocyclohexylmethyl ester 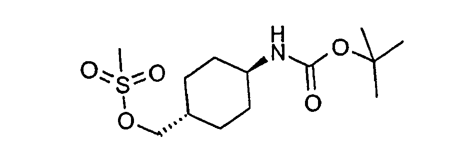 Zu einer Lösung von *trans*-(4-Hydroxymethylcyclohexyl)-*tert*-butylcarbamat (459 mg, 3 mmol) in Dichlormethan (25 ml) bei 0°C wurde Triethylamin (417 uL, 2.2 mmol) zugegeben und Mesthansulfonylchlorid (171 uL, 2.2 mmol) zugetropft. Das Reaktionsgemisch wurde während 5h bei 0°C und während 1h bei Raumtemperatur gerührt, mit Wasser aufgenommen und mit Dichlormethan extrahiert. Die organische Phase wurde über Na₄SO₄ getrocknet und eingeengt. Das Rohprodukt wurde ohne weitere Reinigung umgesetzt.

48.d) [4-(6-Methoxy-chinolin-4-yloxymethyl)-cyclohexyl]-*tert*-butylcarbamat 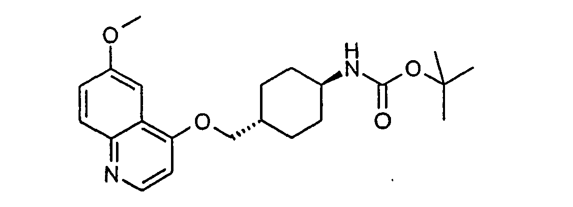 Zu einer Suspension von 6-Methoxy-4-hydroxychinolin (350 mg, 2 mmol) in DMF (4 ml) wurde NaH Dispersion (55% in Mineralöl, 88 mg, 2 mmol) zugegeben und bei Raumtemperatur gerührt bis eine Lösung entstand. *trans*-Methansulfonsäure-4-*tert*-butoxycarbonylaminocyclohexylmethylester (635 mg, 2 mmol) wurde zugegeben und das Reaktiongemisch über Nacht bei 80°C gerührt. Das Gemisch wurde mit Wasser versetzt und mit Essigester extrahiert. Organische Extrakte mit Wasser gewaschen, über Na₂SO₄ getrocknet und eingeengt. Das Rohprodukt wurde mittels Chromatographie an Kieselgel (DCM/MeOH 9:1) gereinigt.
Ausbeute: 438 mg, 56.6% (Oel)
(MS (EI) m/z 387 [M+H]⁺).

48.e) *trans*-4-(6-Methoxy-chinolin-4-yloxymethyl)-cyclohexylamin 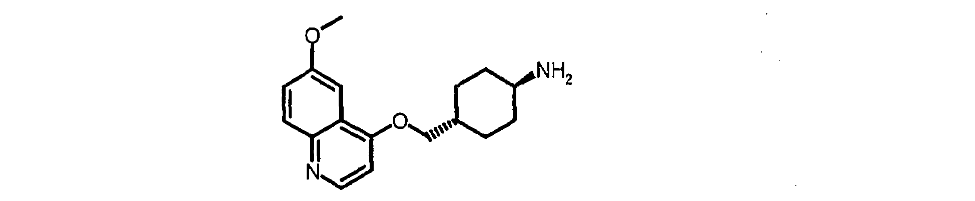 Eine Lösung von [4-(6-Methoxy-chinolin-4-yloxymethyl)-cyclohexyl]-*tert*-butylcarbamat (438 mg, 1.13 mmol) und TFA (1.5 ml) in Dichlormethan (4 ml) wurde während 2h bei Raumtemperatur gerührt. Das Reaktionsgemisch wurde auf Eis/Ammoniumhydroxid gegossen und mit Dichlormethan extrahiert. Die organischen Extrakte wurden über Na₂SO₄ getrocknet und eingeengt.
Ausbeute: 313 mg, 97%
(MS (EI) m/z 287 [M+H]⁺).

48.f) Titelverbindung 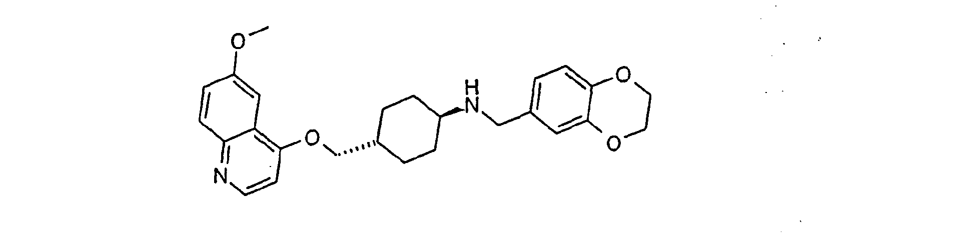 In Analogie zu Beispiel 42.e wurde *trans*-2,3-Dihydrobenzo[1,4]dioxin-6-ylmethyl)-[4-(6-methoxy-chinolin-4-yloxymethyl)-cyclohexyl]-amin in 73% Ausbeute hergestellt (MS (EI) m/z 435 [M+H]⁺).

### Beispiel 49: 6-{[4-(6-Methoxy-chinolin-4-yloxymethyl)-cyclohexylamino]-methyl}-4H-benzo[1,4]oxazin-3-on

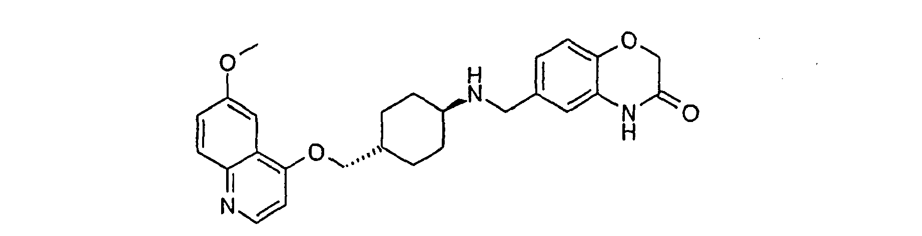

In Analogie zu Beispiel 42.e wurde *trans*-6-{[4-(6-Methoxy-chinolin-4-yloxymethyl)-cyclohexylamino]-methyl}-4H-benzo[1,4]oxazin-3-on in 81% Ausbeute hergestellt (MS (EI) m/z 448 [M+H]⁺).

### Beispiel 50: (2,3-Dihydro-benzo[1,4]dioxin-6-ylmethyl)-[4-(6-methoxy-[1,5]riaphthyridin-4-yloxymethyl)-cyclohexyl]amin

50.a) 4-(6-Methoxy-[1,5]naphthyridin-4-yloxymethyl)-cyclohexylamin 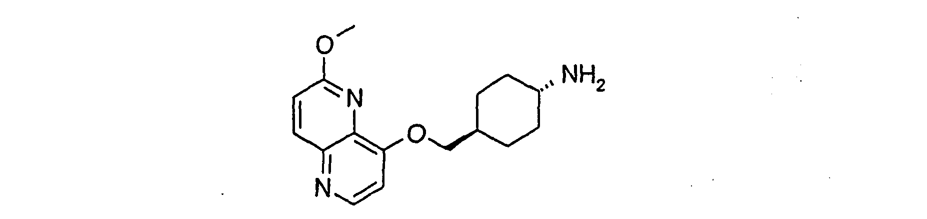 4-(6-Methoxy-[1,5]naphthyridin-4-yloxymethyl)cyclohexylamin wurde analog Beipiel 48 ausgehend von 6-Methoxy-[1,5]naphthyridin-4-ol hergestellt.

50.b) Titelverbindung 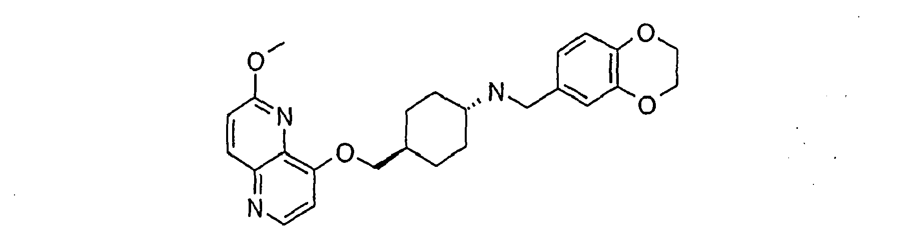 In Analogie zu Beispiel 42.e wurde (2,3-Dihydro-benzo-[1,4]dioxin-6-ylmethyl)-[4-(6-methoxy-[1,5]naphthyridin-4-yloxymethyl)-cyclohexyl]-amin in 55% Ausbeute hergestellt (MS (EI) m/z 456.6 [M+H]⁺).

### Beispiel 51: 6-{[4-(6-Methoxy-[1,5]naphthyridin-4-yloxy-methyl)-cyclohexylamino]-methyl}-4H-benzo[1,4]oxazin-3-on

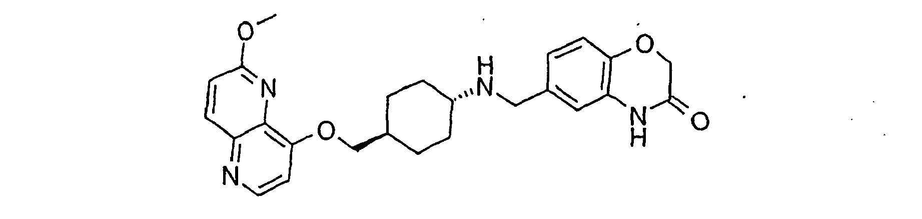

In Analogie zu Beispiel 42.e wurde *trans*-6-{[4-(6-Methoxy-[1,5]naphthyridin-4-yloxymethyl)-cyclohexylamino]-methyl}-4H-benzo[1,4]oxazin-3-on in 79% Ausbeute hergestellt (MS (EI) m/z 456.6 [M+H]⁺).

### Beispiel 52 : 2-{3-[(2,3-Dihydro-benzo[1,4]dioxin-6-yl-methyl)-amino]-8-aza-bicyclo[3.2.1]oct-8-yl}-(1RS)-(6-methoxy-chinolin-4-yl)-ethanol

52.a) {8-[(2RS)-Hydroxy-2-(6-methoxy-quinolin-4-yl)-ethyl]-8-aza-bicyclo[3.2.1]oct-3-yl}-*tert*-butylcarbamat 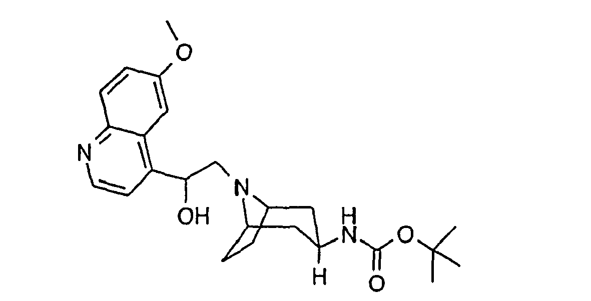 In Analogie zu Beispiel 2.c wurde die Titelverbindung in 82% Ausbeute hergestellt (MS (EI) m/z 428 [M+H]⁺).

(8-Aza-bicyclo[3.2.1]oct-3-yl)-*tert*-butylcarbamat wurde nach Eur. J. Med. Chem. 1991 (34) p-646-653 hergestellt.

52.b) 2-{3-[(2,3-Dihydro-benzo[1,4]dioxin-6-ylmethyl)-amino]-8-aza-bicyclo[3.2.1]oct-8-yl}-(1RS)-(6-methoxy-chinolin-4-yl)-ethanol

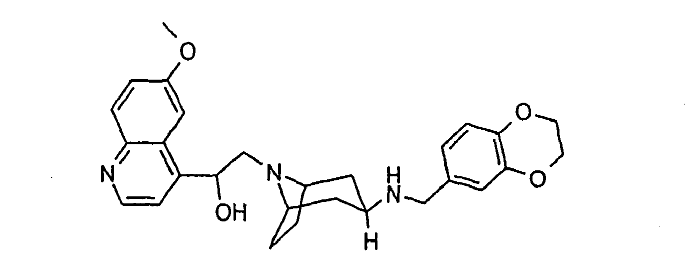

In Analogie zu Beispiel 2.c wurde die Titelverbindung in 86% Ausbeute hergestellt (MS (EI) m/z 328 [M+H]⁺).

### Beispiel 53: 2-{3-[(Benzo[1,3]dioxol-5-ylmethyl)-amino]-8-aza-bicyclo[3.2.1]oct-8-yl}-(1RS)-(6-methoxy-chinolin-4-yl)-ethanol

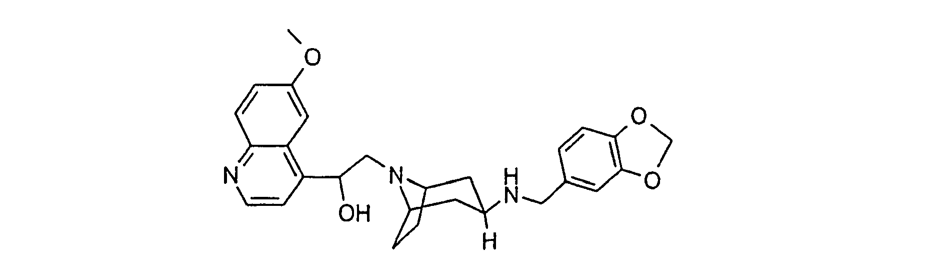

In Analogie zu Beispiel 2.e wurde 2-{3-[(Benzo[1,3]dioxol-5-ylmethyl)-amino]-8-aza-bicyclo[3.2.1]oct-8-yl}-1-(6-methoxy-chinolin-4-yl)-ethanol in 62% Ausbeute hergestellt (MS (EI) m/z 462 [M+H]⁺).

### Beispiel 54: 6-({8-[(2RS)-Hydroxy-2-(6-methoxy-chinolin-4-yl)-ethyl]-8-aza-bicyclo[3.2.1]oct-3-ylamino}-methyl)-4H-benzo[1,4]oxazin-3-on

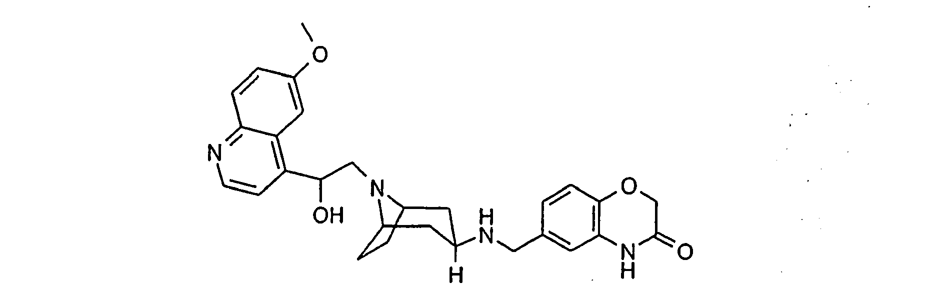

In Analogie zu Beispiel 2.e wurde 6-({8-[2-Hydroxy-2-(6-methoxy-chinolin-4-yl)-ethyl]-8-aza-bicyclo[3.2.1]oct-3-ylamino}-methyl)-4H-benzo[1,4]oxazin-3-on in 7% Ausbeute hergestellt (MS (EI) m/z 489 [M+H]⁺).

### Beispiel 55: 2-{3-[(Benzo[1,2,5]thiadiazol-5-ylmethyl)-amino]-8-aza-bicyclo[3.2.1]oct-8-yl}-(1RS)-(6-methoxy-chinolin-4-yl)-ethanol

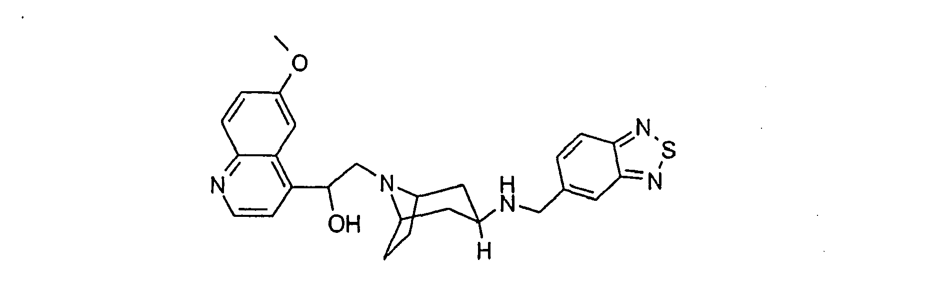

In Analogie zu Beispiel 2.e wurde 2-{3-[(Benzo[1,2,5]-thiadiazol-5-ylmethyl)-amino]-8-aza-bicyclo[3.2.1]oct-8-yl}-1-(6-methoxy-chinolin-4-yl)-ethanol in 59% Ausbeute hergestellt (MS (EI) m/z 476 [M+H]⁺).

### Beispiel 56: 1-(6-Methoxy-chinolin-4-yl)-2-[3-(3-phenyl-allylamino)-8-aza-bicyclo[3.2.1]oct-8-yl]-ethanol

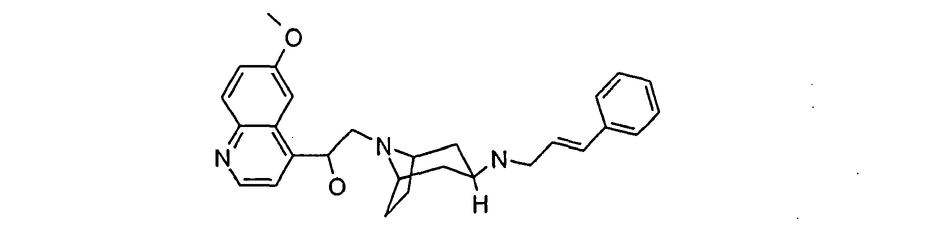

In Analogie zu Beispiel 2.e wurde 1-(6-Methoxy-chinolin-4-yl)-2-[3-(3-phenyl-allylamino)-8-aza-bicyclo[3.2.1]oct-8-yl]-ethanol in 44% Ausbeute hergestellt (MS (EI) m/z 445 [M+H]⁺).

### Beispiel 57: 2-[3-(3-Furan-2-yl-allylamino)-8-aza-bi-cyclo[3.2.1]oct-8-yl]-(1RS)-(6-methoxy-chinolin-4-yl)-ethanol

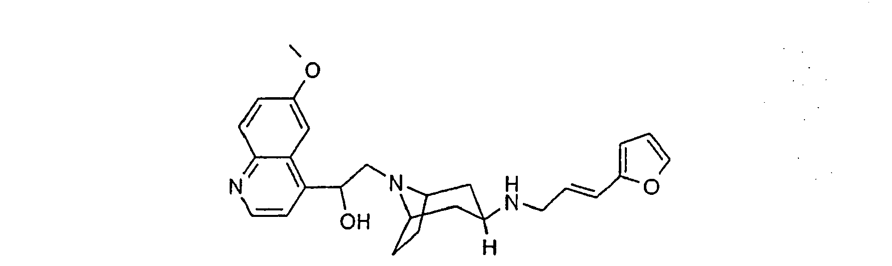

In Analogie zu Beispiel 2.e wurde 2-[3-(3-Furan-2-yl-allylamino)-8-aza-bicyclo[3.2.1]oct-8-yl]-1-(6-methoxy-chinolin-4-yl)-ethanol in 51% Ausbeute hergestellt (MS (EI) m/z 434 [M+H)⁺).

### Beispiel 58: 2-{3-[(Benzofuran-2-ylmethyl)-amino]-8-aza-bicyclo[3.2.1]oct-8-yl}-(1RS)-(6-methoxy-chinolin-4-yl)-ethanol

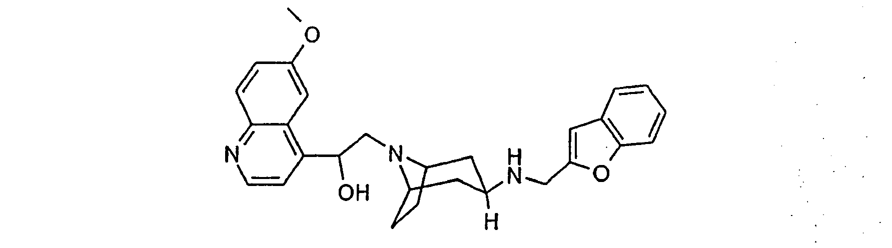

In Analogie zu Beispiel 2.e wurde 2-{3-[(Benzofuran-2-ylmethyl)-amino]-8-aza-bicyclo[3.2.1]oct-8-yl}-1-(6-methoxy-chinolin-4-yl)-ethanol in 62% Ausbeute hergestellt (MS (EI) m/z 458 [M+H]⁺).

### Beispiel 59: 2-(5-{[(2,3-Dihydro-benzo[1,4]dioxin-6-ylmethyl)-amino]-methyl}-3,6-dihydro-2H-pyridin-1-yl)-1-(6-methoxy-quinolin-4-yl)-ethanol

59.a) 5-Azidomethyl-3,6-dihydro-2H-pyridin-1-*tert*-butylcarbamat 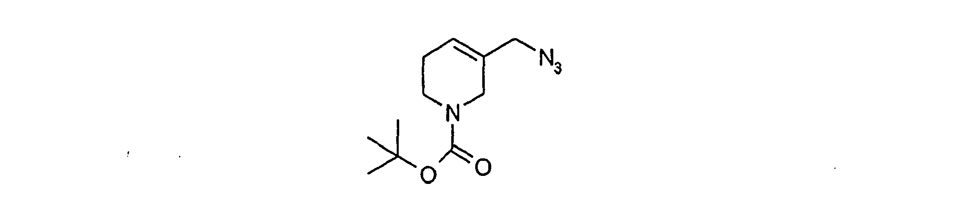 Zu einer Lösung von 5-Hydroxymethyl-3,6-dihydro-2*H-*pyridin-1-*tert*-butylcarbamat (Tetrahedron 1998, 54, 7045-7056, 2.8g, 13.1 mmol) in DCM (50mL) wurden bei 0C Triethylamin (3.7mL, 26.2 mmol) und tropfenweise Methanesulfonylchlorid (1.2mL, 15.44 mmol) zugegeben. Das Reaktionsgemisch wurde während 20 Minuten bei 0C gerührt, bevor eine gesättigte Lösung von NaHCO3 (40mL) zugegeben wurde und die beiden Phasen getrennt wurden. Die wässrige Phase wurde mit Dichlormethan (40mL) extrahiert. Die vereinten organischen Phasen wurden mit ges. Kochsalzlösung gewaschen und über MgSO₄ getrocknet und eingeengt. Das Rohprodukt wurde in DMF (50 mL) gelöst und mit Natriumazid (1.7g, 26.1 mmol) versetzt. Das Gemisch wurde auf 80C erhitzt. Reaktionskontrolle mit LCMS. Nachdem die Reaktion zuende war, wurde das Gemisch abgekühlt und eingeengt. Der Rückstand wurde in EtOAc und Wasser aufgenommen und die wässrige Phase mit EtOAc (2 x 100mL) extrahiert. Die vereinten organischen Phasen wurden mit ges. Kochsalzlösung gewaschen, über MgSO4 getrocknet und eingeengt. Der Rückstand wurde mittels Chromatographie an Kieselgel (EtOAc-HEx 1-6) gereinigt.
Ausbeute: (2.2g, 9.23 mmol) Oel.
MS (EI) m/z 239.4 [M+H]⁺.

59.b) 5-Aminomethyl-3,6-dihydro-2H-pyridin-1-*tert*-butylcarbamat 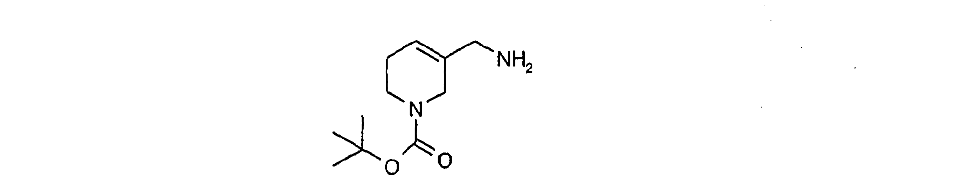 Zu einer Lösung von 5-Azidomethyl-3,6-dihydro-2H-pyridin-1-*tert*-butylcarbamat (1g, 4.2 mmol) in THF (25mL) und Wasser (0.250mL) wurde Triphenylphosphin auf Polystyrol (5.24g) zugegeben. Das Reaktionsgemisch wurde während 36 Stunden bei Raumtemperatur geschüttelt. Das Polymer wurde abfiltriert und mit THF nachgewaschen. Das Filtrat wurde eingeengt.
Ausbeute: (0.893 g, 4.2 mmol)
MS (EI) m/z 213.4 [M+H]⁺.

59.c) 5-{[(2,3-Dihydro-benzo[1,4]dioxin-6-ylmethyl)-amino]-methyl}-3,6-dihydro-2H-pyridin-1-*tert*-butylcarbamat 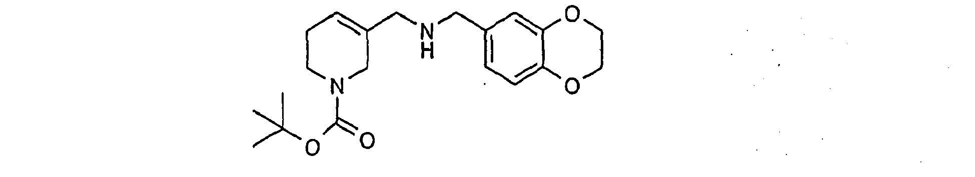 Eine Lösung von 5-Aminomethyl-3,6-dihydro-2H-pyridin-1-*tert*-butylcarbamat (0.7g, 3.3 mmol) und 1,4-Benzodioxan-6-carbaldehyd (0.54 g, 3.3 mmol) in THF (2 mL) und DCE (4 mL) wurde während 1h bei Raumtemperatur gerührt. Natriumtriacetoxyborhydrid (0.770 g, 3.81mmol) wurde dann dazugegeben. Das Reaktionsgemisch wurde während 5h bei Raumtemperatur gerührt, und durch Hydromatrix (benetzt mit ges. NaHCO₃ Lösung) und das Filtrat eingeengt. Der Rückstand wurde mittels Chromatographie an Kieselgel (EtOAc-Hex 2-1 dann EtOAc-MeOH 9-1) gereinigt.
Ausbeute: 0.16g, 0.44 mmol (Oel).
MS (EI) m/z 361 [M+H]⁺.

59.d) (2,3-Dihydro-benzo[1,4]dioxin-6-ylmethyl)-(1,2,5,6-tetrahydropyridin-3-ylmethyl)-amin 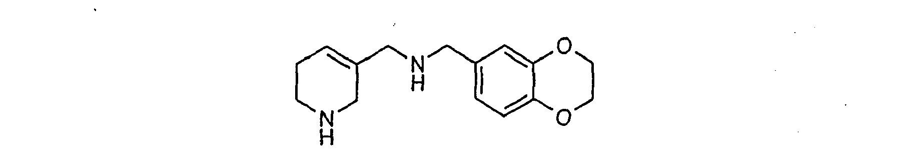 Eine Lösung von 5-{[(2,3-Dihydro-benzo[1,4]dioxin-6-yl-methyl)-amino]-methyl}-3,6-dihydro-2H-pyridin-1-*tert-*butylcarbamat (0.16 g, 0.44 mmol) in TFA (3 mL) wurde während 30 Minuten bei Raumtemperatur gerührt, eingeengt und in ges. Bicarbonatlösung (30 mL) und Dichlormethan (30 mL) aufgenommen. Die wässrige Phase wurde mit Dichlormethan (2 x 30mL) extrahiert und die vereinten organischen Phasen mit ges. Kochsalzlösung gewaschen und über MgSO₄ getrocknet und eingeengt.
Ausbeute: 0.06 g, 0.23 mmol, 52%(Oel)
MS (EI) m/z 261 [M+H]⁺.

59.e) Titelverbindung 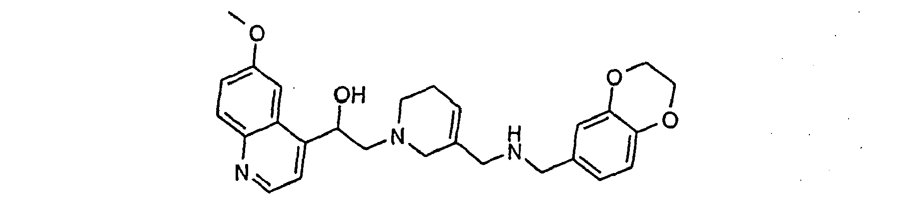 Eine Lösung von (2,3-Dihydro-benzo[1,4]dioxin-6-ylmethyl)-(1,2,5,6-tetrahydro-pyridin-3-ylmethyl)-amin (0.06 g, 0.23mmol) und 6-Methoxy-4-oxiranylchinolin (0.06 g, 0.29mmol) in EtOH (2 mL) wurde während 14h auf 80C erhitzt. Das Reaktionsgemisch wurde eingeengt und der Rückstand mittels Chromatographie an Kieselgel (DCM-MeOH 9-1) gereinigt.
Ausbeute: 0.025 g, 0.054 mmol, 23% (Schaum)
MS (EI) m/z 462 [M+H]⁺.

### Beispiel 60: 2-((2RS)-{[(Benzo[1,3]dioxol-5-ylmethyl)-amino]-methyl}-morpholin-4-yl)-(1RS)-(6-methoxy-chinolin-4-yl)-ethanol

60.a) (2RS)-Azidomethyl-4-benzyl-morpholin 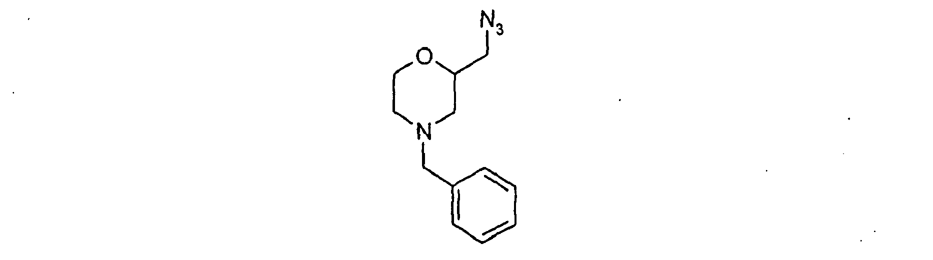 Zu einer Lösung von N-Benzyl-2-hydroxymethylmorpholin (Synthetic Communications, 1980, 10(1), 59-73, 5g, 24.12mmol) in DCM (60mL) bei 0°C wurde Triethylamin (4.88g, 48.25mmol) und tropfenweise Methansulfonylchlorid (2.25mL, 28.95mmol) zugegeben. Nach 30 Minuten wurde die Reaktion durch Zugabe von ges. Bicarbonatlösung gestoppt (50mL). Die organisch Phase wurde mit ges. Kochsalzlösung (50mL) gewaschen, über MgSO4 getrocknet und eingeengt. Das Rohprodukt wurde in DMF (50 mL) gelöst und mit Natriumazid (3.11g, 47.8 mmol) versetzt. Das Gemisch wurde über Nacht auf 80C erhitzt. Das Gemisch wurde abgekühlt und eingeengt. Der Rückstand wurde in Ether (100 mL) und Wasser (50 mL) aufgenommen und die wässrige Phase mit Ether (2 x 100mL) extrahiert. Die vereinten organischen Phasen wurden mit ges. Kochsalzlösung gewaschen, über MgSO₄ getrocknet und eingeengt. Der Rückstand wurde mittels Chromatographie an Kieselgel (EtOAc) gereinigt.
Ausbeute: (4.23g, 18 mmol, 75%) Oel.
MS (EI) m/z 236.2 [M+H]⁺.

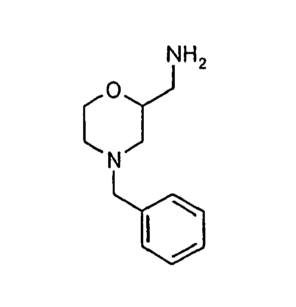

60.b) 2-(4-Benzyl-morpholin-(2RS)-yl)-methylamin
Eine Lösung von 2-Azidomethyl-4-benzyl-morpholin (4.23g, 18mmol) und Triphenylphosphin (9.47g, 36mmol) in THF-Wasser (10-1, 100mL) wurde über Nacht auf 60°C erhitzt. Das Reaktionsgemisch wurde eingeengt und der Rückstand in 3N HCl (200mL) und EtOAc (200mL) aufgenommen. Die wässrige Phase wurde mit EtOAc (4*) extrahiert. Die wässrige Phase wurde mit NaOH auf pH 12 gestellt und mit EtOAc (2 x 200mL) extrahiert, über MgSO₄ getrocknet und eingeengt.
Ausbeute: (3.64g, 16.8 mmol, 93%) Oel.
MS (EI) m/z 207.2 [M+H]⁺.

60.c) (4-Benzyl-morpholin-(2RS)-ylmethyl)-*tert*-butylcarbamat 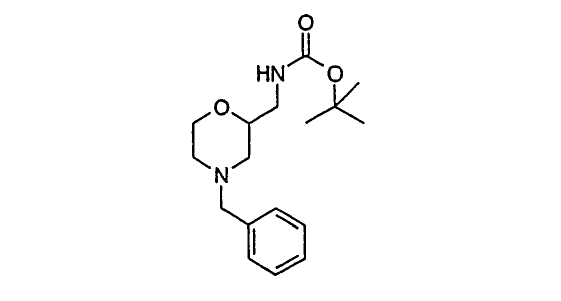 Zu einer Lösung von 2-(4-Benzyl-morpholin-2-yl)-methylamin (1.83g, 8.87mmol) in DCM (45mL) bei 0°C wurde Triethylamin (1.78g, 17.74 mmol) und Di-*tert*-butyl dicarbonat (2.32g, 10.65mmol) zugegeben. Das Reaktionsgemisch wurde während 30 Minuten bei dieser Temperatur gerührt und eingeengt. Der Rückstand wurde mittels Chromatographie an Kieselgel (EtOAc-Hex 1:1) gereinigt.
Ausbeute: (1.51g, 4.93 mmol, 51%) Oel.
MS (EI) m/z 307.3 [M+H]⁺.

60.d) {4-[(2RS)-Hydroxy-2-(6-methoxy-chinolin-4-yl)-ethyl]-morpholin-(2RS)-ylmethyl}-*tert*-butylcarbamat 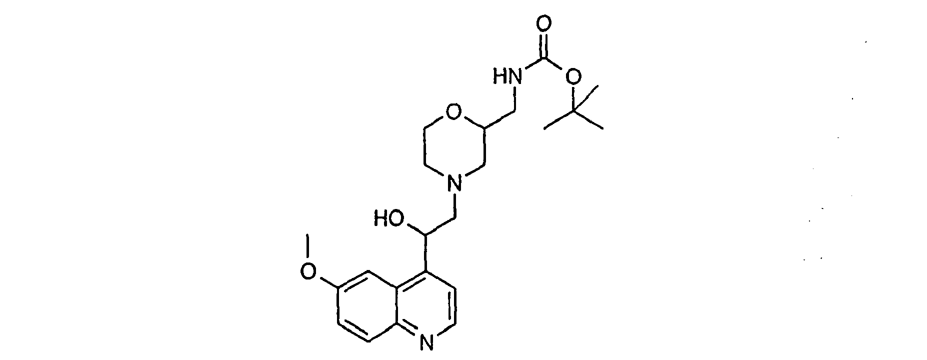 Zu einer Lösung von (4-Benzyl-morpholin-2-ylmethyl)-*tert-*butylcarbamat (1.51g, 4.93mmol) in THF:MeOH (1-1, 28mL) wurde 20% Pd(OH)2 (0.7g) zugegeben. Das Reaktionsgemisch wurde während 2h unter Wasserstoffatmosphäre gerührt. Das Gemisch wurde filtriert und das Filtrat eingeengt. Das Zwischenprodukt wurde in DMF (15mL) gelöst und 6-Methoxy-4-oxiranylchinolin (0.9g, 4.48mmol), Lithium perchlorate (0.477g, 4.48mmol) and Kaliumcarbonat (0.743g, 5.376mmol) zugegeben. Das Gemisch wurde während 23h auf 80C erhitzt. Nach dem Abkühlen wurde filtriert und das Filtrat eingeengt. Der Rückstand wurde in EtOAc gelöst und mit Wasser gewaschen, über MgSO₄ getrocknet und eingeengt. Der Rückstand wurde mittels Chromatographie an Kieselgel (DCM:MeOH 9:1) gereinigt.
Ausbeute: (1g, 2.4 mmol, 48%) Oel.
MS (EI) m/z 418.5 [M+H]⁺.

60.e) 2-((2RS)-Aminomethyl-morpholin-4-yl)-(1RS)-(6-methoxy-chinolin-4-yl)-ethanol 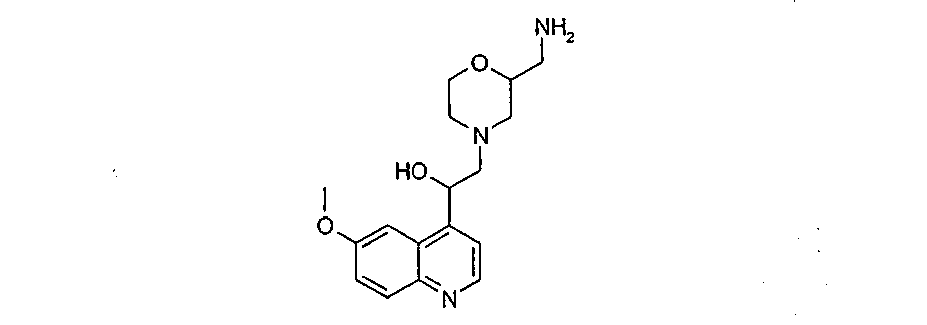 Zu einer Lösung von {4-[2-Hydroxy-2-(6-methoxy-chinolin-4-yl)-ethyl]-morpholin-2-ylmethyl}-*tert*-butylcarbamat (0.95g, 2.27mmol) in DCM (4mL) bei 0C wurde TFA (2.5mL) zugegeben. Das Reaktionsgemisch wurde während 1.5h bei Raumtemperatur gerührt und eingeengt. Der Rückstand wurde in DCM-MeOH (9-1, 30mL) und Ammoniumhydroxid (20mL) aufgenommen. Die wässrige Phase wurde mit DCM/MeOH (2 x 30mL) extrahiert und die vereinten organischen Phasen über Na₂SO₄ getrocknet und eingeengt.
Ausbeute: (0.718g, 2.25 mmol, 99%) Oel.
MS (EI) m/z 318.5 [M+H]⁺.

60.f) 2-(2-{[(Benzo[1,3]dioxol-5-ylmethyl)-amino]-methyl}-morpholin-4-yl)-1-(6-methoxy-chinolin-4-yl)-ethanol 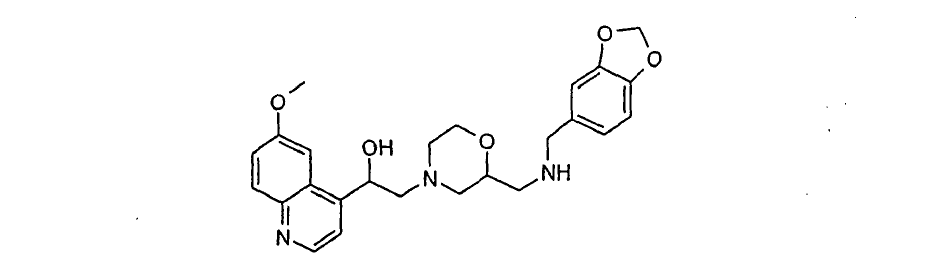 Zu einer Lösung von 2-(2-Aminomethyl-morpholin-4-yl)-1-(6-methoxy-chinolin-4-yl)-ethanol (0.365g, 1.15mmol) in DCM (10.5mL) und methanol (3.5mL) wurde aktiviertes 3A Molekularsieb (3.485g) und Piperonal (0.172g, 1.15mmol) zugegeben. Das Gemisch wurde über Nacht bei Raumtemperatur gerührt, bevor Natriumborhydrid (0.112g, 2.9mmol) zugegeben wurde. Das Reaktionsgemisch wurde noch 2h bei Raumtemperatur gerührt, über Hydromatrix (mit NaHCO₃ benetzt) filtriert und eingeengt. Der Rückstand wurde mittels Chromatographie an Kieselgel (DCM:MeOH 9:1+ 1% NH₄OH) gereinigt.
Ausbeute: (0.261g, 0.57 mmol, 50%) Oel.
MS (EI) m/z 452.5 [M+H]⁺.

### Beispiel 61: 2-((2RS)-{[(2,3-Dihydro-benzo[1,4]dioxin-6-ylmethyl)-amino]-methyl}-morpholin-4-yl)-(1RS)-(6-methoxy-quinolin-4-yl)-ethanol

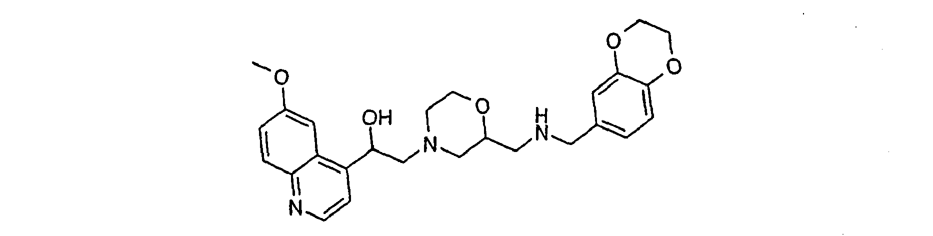

Analog Beispiel 60.f wurde auch 2-(2-{[(2,3-Dihydrobenzo[1,4]dioxin-6-ylmethyl)-amino]-methyl}-morpholin-4-yl)-1-(6-methoxy-chinolin-4-yl)-ethanol in 33% Ausbeute hergestellt (MS (EI) m/z 466 [M+H]⁺.)
Antibakterielle Aktivität:

Die MHK (ug/ml) dieser Verbindungen wurde gegen folgende Bakterienstämme gemessen: S. aureus ATCC 29213, S. aureus 16, E. faecalis ATCC 29212, E. faecium vanA E25-1, H. influenzae 11, E. coli ATCC 25922, M. catarrhalis 117, S. pneumoniae ATCC 49619.

Beispiele 5-6, 8, 11-16, 18-26, 30, 44-45, 47-58 haben eine MHK<=0.125 gegen mindestens einen der aufgelisteten Stämme.

Beispiele 1-4, 7, 9-10, 17, 27-29, 34, 42, 43, 46, 59, 61 haben eine MHK<=0.5 gegen mindestens einen der aufgelisteten Stämme.

Bevorzugte Ausführungsformen
1. Verbindungen der Formel (I): 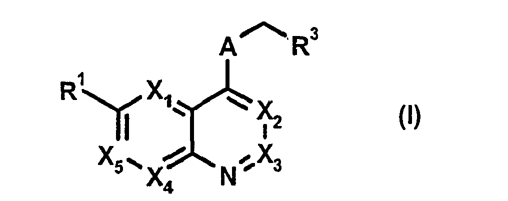 wobei
   A ein Sauerstoff-, Schwefel-, Stickstoffatom, eine C₁₋₄ Alkylen-, C₂₋₄ Alkenylen, C₂₋₄ Alkinylen oder eine C₁₋₄ Heteroalkylengruppe ist,
   X₁, X₂, X₃, X₄ und X₅ unabhängig voneinander Stickstoffatome oder Gruppen der Formel CR² sind,
   R¹ ein Wasserstoffatom, ein Halogenatom, eine Hydroxy-, eine Alkyloxy- oder eine Heteroalkyloxygruppe ist,
   R² ein Wasserstoffatom, ein Halogenatom, eine Hydroxy-, Alkyl-, Alkenyl-, Alkinyl- oder eine Heteroalkylgruppe ist,
   R³ aus den folgenden Gruppen ausgewählt ist: 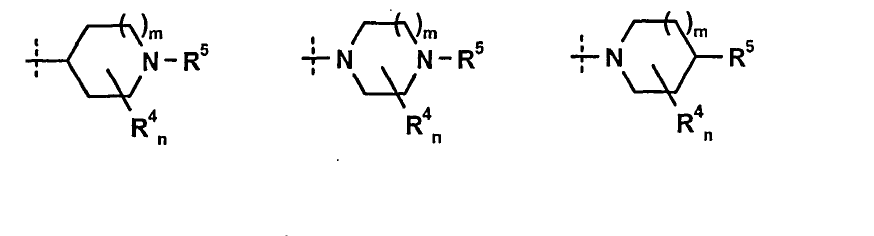 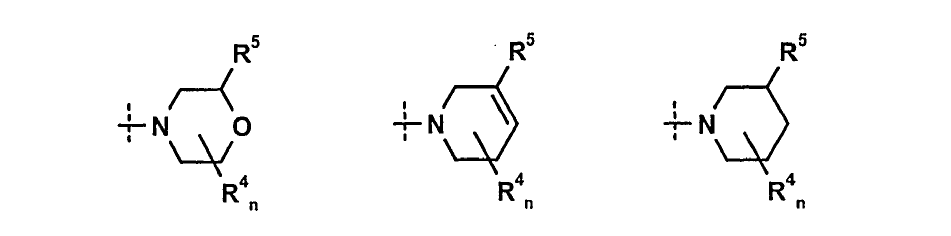 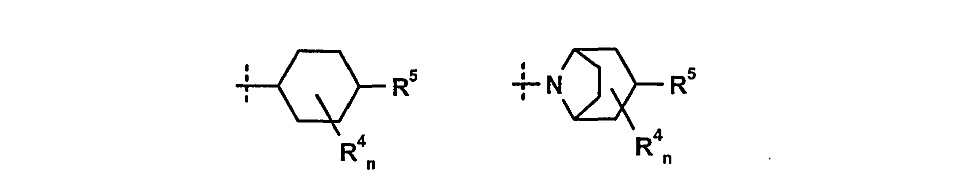 die Reste R⁴ unabhängig voneinander eine Hydroxygruppe, eine C₁₋₆ Alkyl- oder eine C₁₋₈ Heteroalkylgruppe sind,
   R⁵ ein Alkyl-, Alkenyl-, Alkinyl-, Heteroalkyl-, Aryl-, Heteroaryl-, Cycloalkyl-, Alkylcycloalkyl-, Heteroalkyl-cycloalkyl-, Heterocycloalkyl-, Aralkyl- oder ein Heteroaralkylrest ist,
   n gleich 0, 1, 2 oder 3 ist und
   m gleich 0 oder 2 ist,
   oder ein pharmakologisch akzeptables Salz, Solvat, Hydrat oder eine pharmakologisch akzeptable Formulierung derselben.
2.Verbindungen nach Ausführungsform 1, wobei A ein Sauerstoffatom oder eine Gruppe der Formel CH₂ oder CH(OH) ist.
3. Verbindungen nach Ausführungsform 1 oder 2, wobei eine der Gruppen X₁, X₂, X₃, X₄ und X₅ ein Stickstoffatom ist und die anderen CH-Gruppen sind oder alle Gruppen X₁, X₂, X₃, X₄ und X₅ CH-Gruppen sind.
4. Verbindungen nach einer der Ausführungsformen 1 bis 3, wobei R¹ ein Halogenatom, eine C₁₋₆ Alkyloxy, eine Methyl- oder eine Ethylgruppe ist.
5. Verbindungen nach einer der Ausführungsformen 1 bis 4, wobei R¹ eine Methoxygruppe ist.
6. Verbindungen nach einer der Ausführungsformen 1 bis 5, wobei R⁴ eine C₁₋₆ Heteroalkylgruppe mit ein oder zwei Sauerstoffatomen als einzigen Heteroatomen ist.
7. Verbindungen nach einer der Ausführungsformen 1 bis 6, wobei R⁴ eine Gruppe der Formel -COOH, -CH₂COOH, -CH₂CH₂COOH, -CH₂COOCH₃, -CH₂CH₃, -CH₂OH, -CH₂CH₂OH, -OH, -OCH₃, -CH₂OCONH₂, -CH₂CH₂COOCH₃, -COOCH₃, -CH₃ oder -(CH₂)₃OH ist.
8. Verbindungen nach einer der Ausführungsformen 1 bis 7, wobei n gleich 0 oder 1 ist.
9. Verbindungen nach einer der Ausführungsform 1 bis 8, wobei R⁵ eine Aralkyl- oder eine Heteroaralkylgruppe ist.
10. Verbindungen nach einer der Ausführungsform 1 bis 9, wobei R⁵ eine Gruppe der Formel -Y-Cy ist, wobei Y eine C₁-C₆ Alkylen-, C₂-C₆ Alkenylen- oder C₁-C₆ Heteroalkylengruppe ist, wobei gegebenenfalls ein Wasserstoffatom durch eine Hydroxygruppe oder zwei Wasserstoffatome durch eine =O Gruppe ersetzt sein können und Cy eine gegebenenfalls substituierte Phenyl-, Naphthyl- oder Heteroarylgruppe mit 1 oder 2 Ringen und 5 bis 10 Ringatomen oder eine gegebenenfalls substituierte Arylheterocycloalkyl- oder Heteroarylheterocycloalkylgruppe mit zwei Ringen und 9 oder 10 Ringatomen ist
11. Verbindungen nach einer der Ausführungsformen 1 bis 10, wobei R³ aus folgenden Gruppen ausgewählt ist: 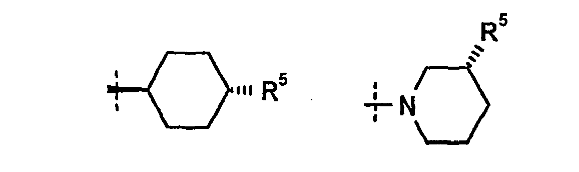
12. Pharmazeutische Zusammensetzungen, die eine Verbindung nach den Ausführungsformen 1 bis 11 und fakultativ Trägerstoffe und/oder Adjuvanzien enthalten.
13. Verwendung einer Verbindung oder einer pharmazeutischen Zusammensetzung nach einer der Ausführungsformen 1 bis 12 zur Behandlung von Bakterieninfektionen.

## Patentansprüche

1. Verbindungen der Formel (I): 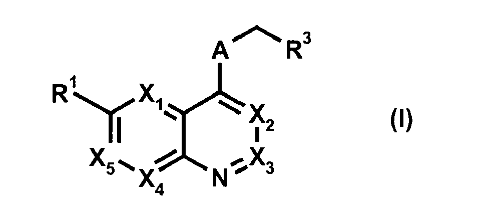 wobei
A eine Gruppe der Formel CH(OH) oder CH₂ oder ein Sauerstoffatom ist,
X₁, X₂, X₃, X₄ und X₅ unabhängig voneinander Stickstoffatome oder Gruppen der Formel CR² sind,
R¹ ein Wasserstoffatom, ein Halogenatom, eine Hydroxy-, eine Alkyloxy- oder eine Heteroalkyloxygruppe ist,
R² ein Wasserstoffatom, ein Halogenatom, eine Hydroxy-, Alkyl-, Alkenyl-, Alkinyl- oder eine Heteroalkylgruppe ist,
R³ aus den folgenden Gruppen ausgewählt ist: 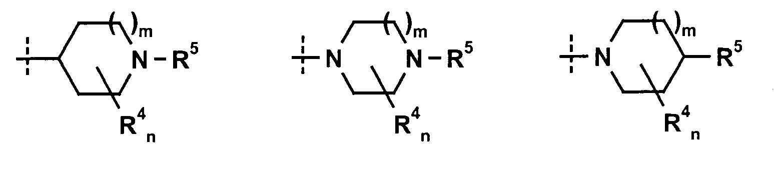 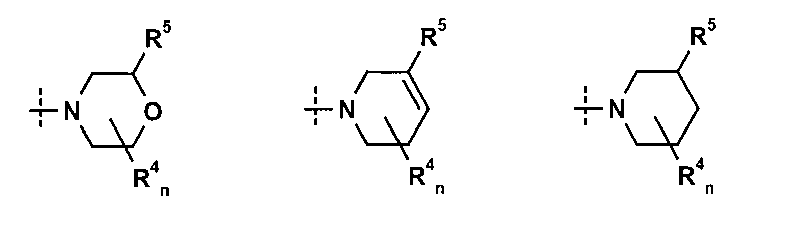 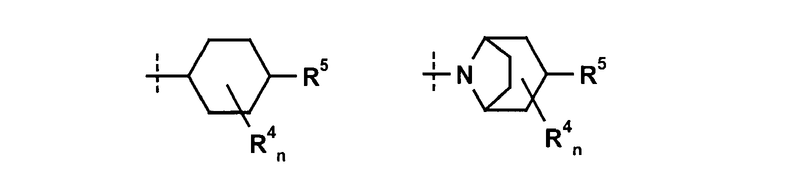 die Reste R⁴ unabhängig voneinander eine Hydroxygruppe, eine C₁₋₆ Alkyl- oder eine C₁₋₈ Heteroalkylgruppe sind,
R⁵ eine Aralkyl- oder eine Heteroaralkylgruppe ist,
n gleich 0, 1, 2 oder 3 ist und
m gleich 0 oder 2 ist,
oder ein pharmakologisch akzeptables Salz, Solvat, Hydrat oder eine pharmakologisch akzeptable Formulierung derselben.

2. Verbindungen nach Anspruch 1, wobei eine der Gruppen X₁, X₂, X₃, X₄ und X₅ ein Stickstoffatom ist und die anderen CH-Gruppen sind oder alle Gruppen X₁, X₂, X₃, X₄ und X₅ CH-Gruppen sind.

3. Verbindungen nach Anspruch 1 oder 2, wobei R¹ ein Halogenatom, eine C₁₋₆ Alkyloxy, eine Methyl- oder eine Ethylgruppe ist.

4. Verbindungen nach einem der Ansprüche 1 bis 3, wobei R¹ eine Methoxygruppe ist.

5. Verbindungen nach einem der Ansprüche 1 bis 4, wobei R⁴ eine C₁₋₆ Heteroalkylgruppe mit ein oder zwei Sauerstoffatomen als einzigen Heteroatomen ist.

6. Verbindungen nach einem der Ansprüche 1 bis 5, wobei R⁴ eine Gruppe der Formel -COOH, -CH₂COOH, -CH₂CH₂COOH, -CH₂COOCH₃, -CH₂CH₃, -CH₂OH, -CH₂CH₂OH, -OH, -OCH₃, -CH₂OCONH₂, -CH₂CH₂COOCH₃, -COOCH₃, -CH₃ oder -(CH₂)₃OH ist.

7. Verbindungen nach einem der Ansprüche 1 bis 6, wobei n gleich 0 oder 1 ist.

8. Verbindungen nach einem der Ansprüche 1 bis 7, wobei R⁵ eine Gruppe der Formel -Y-Cy ist, wobei Y eine C₁-C₆ Alkylen-, C₂-C₆ Alkenylen- oder C₁-C₆ Heteroalkylengruppe ist, wobei gegebenenfalls ein Wasserstoffatom durch eine Hydroxygruppe oder zwei Wasserstoffatome durch eine =O Gruppe ersetzt sein können und Cy eine gegebenenfalls substituierte Phenyl-, Naphthyl- oder Heteroarylgruppe mit 1 oder 2 Ringen und 5 bis 10 Ringatomen oder eine gegebenenfalls substituierte Arylheterocycloalkyl- oder Heteroarylheterocycloalkylgruppe mit zwei Ringen und 9 oder 10 Ringatomen ist.

9. Verbindungen nach einem der Ansprüche 1 bis 8, wobei R³ aus folgenden Gruppen ausgewählt ist:

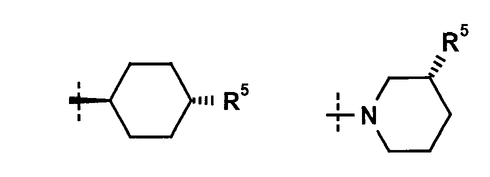

10. Pharmazeutische Zusammensetzungen, die eine Verbindung nach den Ansprüchen 1 bis 9 und fakultativ Trägerstoffe und/oder Adjuvanzien enthalten.

11. Verwendung einer Verbindung oder einer pharmazeutischen Zusammensetzung nach einem der Ansprüche 1 bis 10 zur Behandlung von Bakterieninfektionen.
